# EUROPEAN PATENT SPECIFICATION

(11) **EP 3 587 427 B1**
(45) Date of publication and mention of the grant of the patent: **29.04.2026**
(21) Application number: 19177513.9
(22) Date of filing: 30.05.2019
(51) Int. Cl.: H10K 85/30, H10K 101/10, C07F 7/08, C07D 487/22, C09K 11/06, H10K 50/11

(54) **ORGANOMETALLIC COMPOUND, ORGANIC LIGHT-EMITTING DEVICE INCLUDING THE SAME, AND APPARATUS INCLUDING THE ORGANIC LIGHT-EMITTING DEVICE**
ORGANOMETALLISCHE VERBINDUNG, ORGANISCHE LICHTEMITTIERENDE VORRICHTUNG DAMIT UND VORRICHTUNG MIT DER ORGANISCHEN LICHTEMITTIERENDEN VORRICHTUNG
COMPOSÉ ORGANOMÉTALLIQUE, DISPOSITIF ÉLECTROLUMINESCENT ORGANIQUE LE COMPRENANT ET APPAREIL COMPRENANT LE DISPOSITIF ÉLECTROLUMINESCENT ORGANIQUE

(30) Priority: 28.06.2018 KR 20180074983
(43) Date of publication of application: 01.01.2020
(73) Proprietor: Samsung Display Co., Ltd., Gyeonggi-Do (KR)
(72) Inventor: KIM, Myeongsuk, Gyeonggi-do (KR); YE, Jimyoung, Gyeonggi-do (KR); YOO, Byeongwook, Gyeonggi-do (KR); YOON, Jihwan, Gyeonggi-do (KR); HWANG, Jaehoon, Gyeonggi-do (KR); KIM, Sunjae, Seoul (KR); JEONG, Jaeho, Seoul (KR)
(74) Representative: Marks & Clerk LLP

(56) References cited:
- WO-A1-2016/150677
- WO-A1-2017/167633
- WO-A1-2018/073120
- WO-A2-2009/108947
- US-A1- 2011 304 263
- OU, ZHONGPING ET AL: "Gold(III) Porphyrins Containing Two, Three, or Four .beta.,.beta.'-Fused Quinoxalines. Synthesis, Electrochemistry, and Effect of Structure and Acidity on Electroreduction Mechanism", INORGANIC CHEMISTRY, vol. 52, no. 5, 20 February 2013 (2013-02-20), pages 2474 - 2483, XP002794465, ISSN: 0020-1669, DOI: 10.1021/IC302380Z
- HOLLAUF, M. ET AL: "Dye functionalized-ROMP based terpolymers for the use as a light up-converting material via triplet-triplet annihilation", JOURNAL OF MATERIALS CHEMISTRY C: MATERIALS FOR OPTICAL AND ELECTRONIC DEVICES, vol. 5, no. 30, 19 July 2017 (2017-07-19), pages 7535 - 7545, XP002794466, ISSN: 2050-7534, DOI: 10.1039/C7TC01639E
- ISHII, YUTARO ET AL: "Synthesis of meso-substituted tetrabenzoporphyrin via selective meso-bromination of bicycloporphyrin", TETRAHEDRON LETTERS, vol. 57, no. 46, 6 October 2016 (2016-10-06), pages 5079 - 5083, XP002794467, ISSN: 0040-4039, DOI: 10.1016/J.TETLET.2016.10.001
- YE, LINA ET AL: "Cobalt Tetrabutano- and Tetrabenzotetraarylporphyrin Complexes: Effect of Substituents on the Electrochemical Properties and Catalytic Activity of Oxygen Reduction Reactions", INORGANIC CHEMISTRY, vol. 56, no. 21, 24 October 2017 (2017-10-24), pages 13613 - 13626, XP002794468, ISSN: 0020-1669, DOI: 10.1021/ACS.INORGCHEM.7B02405
- KUMAR, SIDDHARTHA ET AL: ".beta.-Functionalized trans-A2B2 push-pull tetrabenzoporphyrins", CHEMICAL COMMUNICATIONS, vol. 54, no. 42, 28 February 2018 (2018-02-28), pages 5303 - 5306, XP002794469, ISSN: 1359-7345, DOI: 10.1039/C7CC09743C
- DOVER, CAMERON B. ET AL: "Endothermic singlet fission is hindered by excimer formation", NATURE CHEMISTRY, vol. 10, no. 3, 22 January 2018 (2018-01-22), pages 305 - 310, XP002794470, ISSN: 1755-4330, DOI: 10.1038/NCHEM.2926
- DOVER, CAMERON B. ET AL: "Supplementary information to Endothermic singlet fission is hindered by excimer formation", NATURE CHEMISTRY, vol. 10, no. 3, 22 January 2018 (2018-01-22), pages 1 - 24, XP002794688, DOI: 10.1038/nchem.2926

## Description

### BACKGROUND

### 1. Field

One or more embodiments of the present disclosure relate to an organometallic compound, an organic light-emitting device including the same, and an apparatus including the organic light-emitting device.

### 2. Description of the Related Art

Organic light-emitting devices are self-emission devices that produce full-color images, and also have wide viewing angles, high contrast ratios, short response times, and excellent characteristics in terms of brightness, driving voltage, and response speed, as compared to other devices in the art.

An example of such organic light-emitting devices may include a first electrode disposed on a substrate, and a hole transport region, an emission layer, an electron transport region, and a second electrode, which are sequentially disposed on the first electrode. Holes provided from the first electrode may move toward the emission layer through the hole transport region, and electrons provided from the second electrode may move toward the emission layer through the electron transport region. Carriers, such as holes and electrons, recombine in the emission layer to produce excitons. These excitons transit (e.g., transition or relax) from an excited state to a ground state, thereby generating light.
US 2011/304263 A1 discloses devices comprising a layer including compounds that are capable of triplet triplet annihilation up conversion (TTA-UC).

### SUMMARY

One or more embodiments provide a novel organometallic compound, an organic light-emitting device including the same, and an apparatus including the organic light-emitting device.

Additional aspects of embodiments will be set forth in part in the description which follows and, in part, will be apparent from the description, or may be learned by practice of the presented embodiments.

An aspect of an embodiment provides an organometallic compound represented by Formula 1 and emitting near-infrared phosphorescent light having a maximum emission wavelength in a range from 720 nm to 2,500 nm:

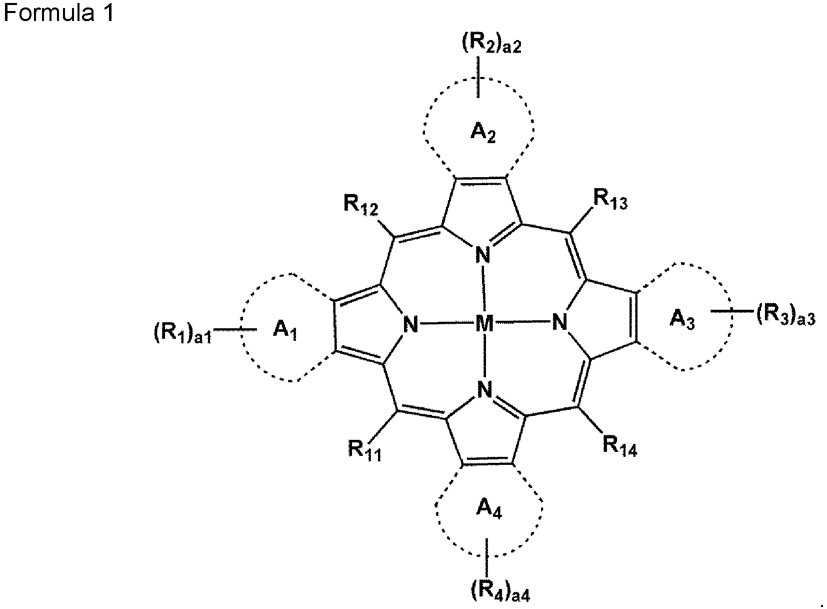

In Formula 1,
M is a transition metal,
ring A₁ to ring A₄ are each independently a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, a pyrrole group, an indole group, an indene group, a benzosilole group, a benzothiophene group, a benzofuran group, a carbazole group, a fluorene group, a dibenzosilole group, a dibenzothiophene group, a dibenzofuran group, a pyridine group, a pyrimidine group, a pyrazine group, a pyridazine group, a triazine group, a quinoline group, an isoquinoline group, a quinazoline group, a phenanthroline group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group,
R₁ to R₄ and R₁₁ to R₁₄ are each independently selected from hydrogen, deuterium, -F, -Cl, -Br, -I, a hydroxyl group, a cyano group, a nitro group, an amidino group, a hydrazine group (a hydrazino group), a hydrazone group (a hydrazono group), a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -O(Q₁), -S(Q₁),-Si(Q₁)(Q₂)(Q₃), -N(Q₁)(Q₂), -B(Q₁)(Q₂), -P(Q₁)(Q₂), -C(=O)(Q₁), -S(=O)₂(Q₁), and-P(=O)(Q₁)(Q₂),
a1 to a4 are each independently an integer from 0 to 10,
a) R₁₁ to R₁₄ are not simultaneously hydrogen, b) R₁₁ to R₁₄ are not simultaneously a phenyl group, c) R₁₁ to R₁₄ are not simultaneously a mesityl group, or d) R₁₁ to R₁₄ are not simultaneously a methyl group,
two selected from R₁₁ to R₁₄ are not linked to each other,
at least one substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₁-C₆₀ heteroaryl group, the substituted monovalent non-aromatic condensed polycyclic group, and the substituted monovalent non-aromatic condensed heteropolycyclic group is selected from:
   deuterium, -F, -Cl, -Br, -I, a hydroxyl group, a cyano group, a nitro group, an amidino group, a hydrazine group (a hydrazino group), a hydrazone group (a hydrazono group), a C₁-C₆₀ alkyl group, a C₂-C₆₀ alkenyl group, and a C₂-C₆₀ alkynyl group;
   a C₁-C₆₀ alkyl group, a C₂-C₆₀ alkenyl group, and a C₂-C₆₀ alkynyl group, each substituted with at least one selected from deuterium, -F, -Cl, -Br, -I, a hydroxyl group, a cyano group, a nitro group, an amidino group, a hydrazine group (a hydrazino group), a hydrazone group (a hydrazono group), a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, a monovalent non-aromatic condensed heteropolycyclic group, -O(Q₁₁), -S(Q₁₁), -Si(Q₁₁)(Q₁₂)(Q₁₃), -N(Q₁₁)(Q₁₂), -B(Q₁₁)(Q₁₂), -P(Q₁₁)(Q₁₂),-C(=O)(Q₁₁), -S(=O)₂(Q₁₁), and -P(=O)(Q₁₁)(Q₁₂);
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazine group (a hydrazino group), a hydrazone group (a hydrazono group), a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, a monovalent non-aromatic condensed heteropolycyclic group, -O(Q₂₁), -S(Q₂₁), -Si(Q₂₁)(Q₂₂)(Q₂₃), -N(Q₂₁)(Q₂₂), -B(Q₂₁)(Q₂₂), -P(Q₂₁)(Q₂₂),-C(=O)(Q₂₁), -S(=O)₂(Q₂₁), and -P(=O)(Q₂₁)(Q₂₂); and
   -O(Q₃₁), -S(Q₃₁), -Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁), -S(=O)₂(Q₃₁), and -P(=O)(Q₃₁)(Q₃₂),
   Q₁ to Q₃, Q₁₁ to Q₁₃, Q₂₁ to Q₂₃, and Q₃₁ to Q₃₃ are each independently selected from hydrogen, deuterium, -F, -Cl, -Br, -I, a hydroxyl group, a cyano group, a nitro group, an amidino group, a hydrazine group (a hydrazino group), a hydrazone group (a hydrazono group), a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, a monovalent non-aromatic condensed heteropolycyclic group, a biphenyl group, a terphenyl group, a C₁-C₆₀ alkyl group substituted with at least one selected from deuterium, -F, a cyano group, a C₁-C₆₀ alkyl group, a phenyl group, and a biphenyl group, and a C₆-C₆₀ aryl group substituted with at least one selected from deuterium, -F, a cyano group, a C₁-C₁₀ alkyl group, a phenyl group, and a biphenyl group,
   and the organometallic compound is not any of the following compounds: 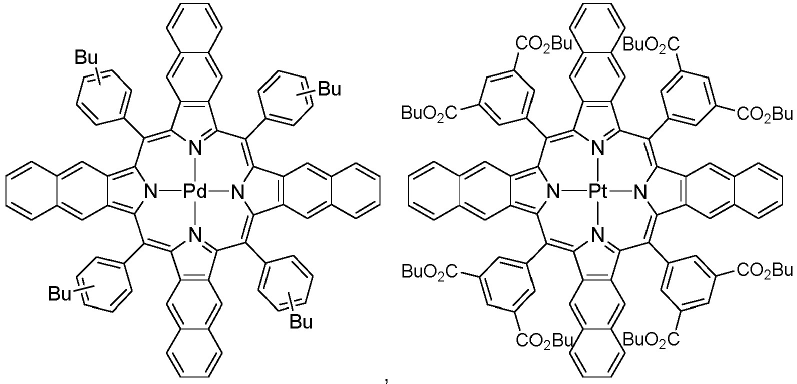 or

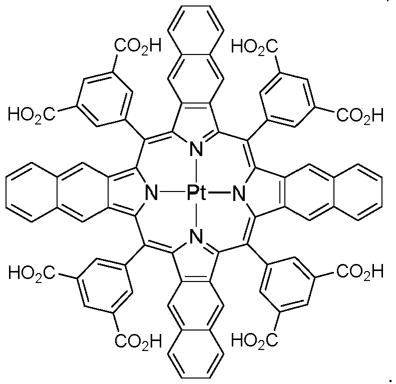

Another aspect of an embodiment provides an organic light-emitting device including: a first electrode, a second electrode facing the first electrode, and an organic layer between the first electrode and the second electrode, the organic layer including an emission layer, and the emission layer comprises at least one of the organometallic compound as described herein and emits near-infrared phosphorescent light having a maximum emission wavelength in a range from 720 nm to 2,500 nm.

Another aspect of an embodiment provides an apparatus including the organic light-emitting device as described herein.

At least some of the above and other features of the invention are set out in the claims.

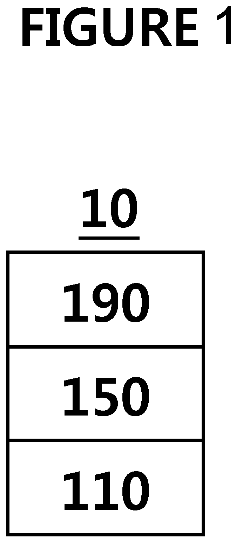

### BRIEF DESCRIPTION OF THE DRAWING

These and/or other aspects of embodiments will become apparent and more readily appreciated from the following description of the embodiments, taken in conjunction with the accompanying drawing which is a schematic view of an organic light-emitting device according to an embodiment.

### DETAILED DESCRIPTION

The subject matter of the present disclosure will now be described more fully with reference to embodiments. The subject matter of the present disclosure may, however, be embodied in many different forms and should not be construed as being limited to the embodiments set forth herein; rather, these embodiments are provided so that this disclosure will be thorough and complete, and will fully convey the concept of the disclosure to those skilled in the art. Features of embodiments of the disclosure, and how to achieve them, will become apparent by reference to the embodiments that will be described herein below in more detail, together with the accompanying drawing. The subject matter of the present disclosure may, however, be embodied in many different forms and should not be limited to the embodiments.

An aspect of an embodiment provides an organometallic compound represented by Formula 1: 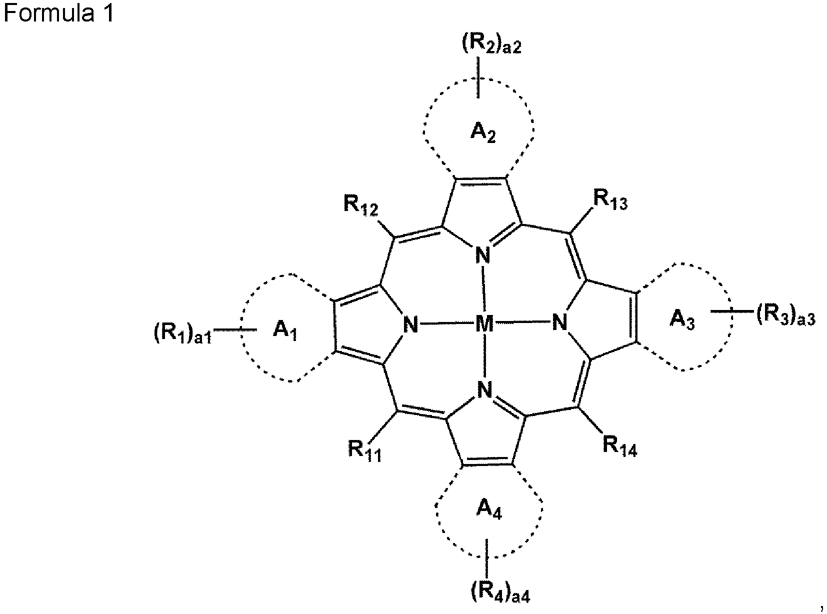 wherein, in Formula 1, M is a transition metal. For example, M may be a first-row transition metal, a second-row transition metal, or a third-row transition metal, of the Periodic Table of Elements.

For example, M may be iridium (Ir), platinum (Pt), osmium (Os), titanium (Ti), zirconium (Zr), hafnium (Hf), europium (Eu), terbium (Tb), thulium (Tm), rhodium (Rh), manganese (Mn), cobalt (Co), copper (Cu), ruthenium (Ru), palladium (Pd), silver (Ag), rhenium (Re), or gold (Au).

In one embodiment, M may be Pt, Pd, or Au, but embodiments of the present disclosure are not limited thereto. For example, M may be Pt or Pd. For example, M may be Pt.

In Formula 1, ring A₁ to ring A₄ are each independently a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, a pyrrole group, an indole group, an indene group, a benzosilole group, a benzothiophene group, a benzofuran group, a carbazole group, a fluorene group, a dibenzosilole group, a dibenzothiophene group, a dibenzofuran group, a pyridine group, a pyrimidine group, a pyrazine group, a pyridazine group, a triazine group, a quinoline group, an isoquinoline group, a quinazoline group, a phenanthroline group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group.

In one embodiment, ring A₁ to ring A₄ may each independently be a naphthalene group, an anthracene group, a phenanthrene group, a pyridine group, a pyrimidine group, a pyrazine group, a quinoline group, or an isoquinoline group, but embodiments of the present disclosure are not limited thereto.

In one or more embodiments, ring A₁ to ring A₄ may be identical to each other (e.g., any two of ring A₁ to ring A₄ may be identical to each other).

In one or more embodiments, ring A₁ and ring A₂ may be different from each other (e.g., ring A₃ and ring A₄ may be identical to each other and, optionally identical to ring A₁ or ring A₂), or ring A₁ and ring A₃ may be different from each other (e.g., ring A₂ and ring A₄ may be identical to each other and, optionally identical to ring A₁ or ring A₃).

In one or more embodiments, ring A₁ to ring A₄ may each independently be selected from groups represented by Formulae 2-2 to 2-13:

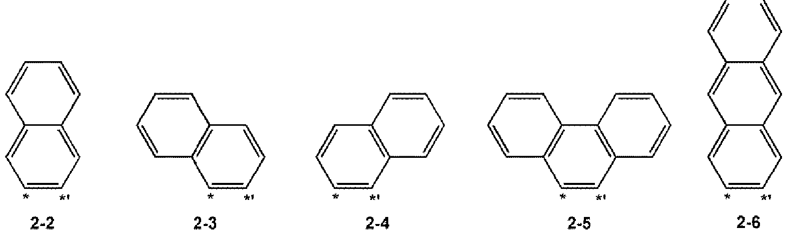

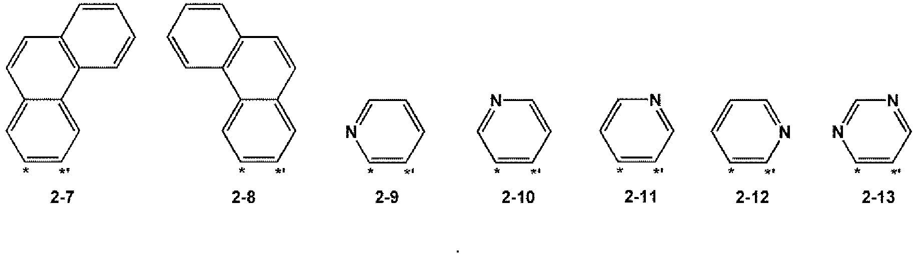

In Formulae 2-2 to 2-13, * and *' each indicate a position of a carbon atom shared with a neighboring 5-membered ring to which each of ring A₁ to ring A₄ is condensed (e.g., combined with), respectively.

In one or more embodiments, ring A₁ to ring A₄ may each be a group represented by Formula 2-2.

In Formula 1, R₁ to R₄ and R₁₁ to R₁₄ are each independently selected from hydrogen, deuterium, -F, -Cl, -Br, -I, a hydroxyl group, a cyano group, a nitro group, an amidino group, a hydrazine group (a hydrazino group), a hydrazone group (a hydrazono group), a substituted or unsubstituted C₁-C₆₀ (*e.g.* C₁-C₂₀) alkyl group, a substituted or unsubstituted C₂-C₆₀ (e.g. C₂-C₂₀) alkenyl group, a substituted or unsubstituted C₂-C₆₀ (*e.g.* C₂-C₂₀) alkynyl group, a substituted or unsubstituted C₁-C₆₀ (*e.g.* C₁-C₂₀) alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e.g.* C₆-C₃₀) aryl group, a substituted or unsubstituted C₁-C₆₀ (*e.g.* C₁-C₂₀) heteroaryl group, a substituted or unsubstituted monovalent non-aromatic condensed polycyclic group, a substituted or unsubstituted monovalent non-aromatic condensed heteropolycyclic group, -O(Q₁), -S(Q₁), -Si(Q₁)(Q₂)(Q₃), -N(Q₁)(Q₂), -B(Q₁)(Q₂), -P(Q₁)(Q₂), -C(=O)(Q₁), -S(=O)₂(Q₁), and -P(=O)(Q₁)(Q₂), and Q₁ to Q₃ may each independently be the same as defined above.

For example, R₁ to R₄ and R₁₁ to R₁₄ may each independently be selected from:
hydrogen, deuterium, -F, -Cl, -Br, -I, a hydroxyl group, a cyano group, a nitro group, an amidino group, a hydrazine group (a hydrazino group), and a hydrazone group (a hydrazono group);
a C₁-C₂₀ alkyl group, a C₂-C₂₀ alkenyl group, a C₂-C₂₀ alkynyl group, and a C₁-C₂₀ alkoxy group, each unsubstituted or substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group (a hydrazino group), a hydrazone group (a hydrazono group), a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₂₀ aryl group, a C₆-C₂₀ aryl group substituted with at least one C₁-C₁₀ alkyl group, a C₁-C₂₀ heteroaryl group, a monovalent non-aromatic condensed polycyclic group, a monovalent non-aromatic condensed heteropolycyclic group,-O(Q₃₁), -S(Q₃₁), -Si(Q₃₁)(Q₃₂)(Q₃₃), -N(Q₃₁)(Q₃₂), -B(Q₃₁)(Q₃₂), -P(Q₃₁)(Q₃₂),-C(=O)(Q₃₁), -S(=O)₂(Q₃₁), and -P(=O)(Q₃₁)(Q₃₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₁-C₆₀ (*e.g.* C₁-C₂₀) heteroaryl group, a monovalent non-aromatic condensed polycyclic group, and a monovalent non-aromatic condensed heteropolycyclic group, each unsubstituted or substituted with at least one selected from deuterium, -F, -Cl,-Br, -I, -CD₃, -CD₂H, -CDH₂, -CF₃, -CF₂H, -CFH₂, a hydroxyl group, a cyano group, a nitro group, an amidino group, a hydrazine group (a hydrazino group), a hydrazone group (a hydrazono group), a C₁-C₂₀ alkyl group, a C₂-C₂₀ alkenyl group, a C₂-C₂₀ alkynyl group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₂₀ aryl group, a C₆-C₂₀ aryl group substituted with at least one C₁-C₁₀ alkyl group, a C₁-C₆₀ (e.g. C₁-C₂₀) heteroaryl group, a monovalent non-aromatic condensed polycyclic group, a monovalent non-aromatic condensed heteropolycyclic group, -O(Q₃₁), -S(Q₃₁),-Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁), -S(=O)₂(Q₃₁), and -P(=O)(Q₃₁)(Q₃₂); and
-O(Q₁), -S(Q₁), -Si(Q₁)(Q₂)(Q₃), -N(Q₁)(Q₂), -B(Q₁)(Q₂), -P(Q₁)(Q₂),-C(=O)(Q₁), -S(=O)₂(Q₁), and -P(=O)(Q₁)(Q₂), and
Q₁ to Q₃ and Q₃₁ to Q₃₃ may each independently be selected from hydrogen, deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group (a hydrazino group), a hydrazone group (a hydrazono group), a C₁-C₂₀ alkyl group, a C₂-C₂₀ alkenyl group, a C₂-C₂₀ alkynyl group, a C₁-C₂₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₂₀ aryl group, a C₁-C₂₀ heteroaryl group, a monovalent non-aromatic condensed polycyclic group, a monovalent non-aromatic condensed heteropolycyclic group, a biphenyl group, a terphenyl group, a C₁-C₂₀ alkyl group substituted with at least one selected from deuterium, -F, a cyano group, a C₁-C₂₀ alkyl group, a phenyl group, and a biphenyl group, and a C₆-C₂₀ aryl group substituted with at least one selected from deuterium, -F, a cyano group, a C₁-C₂₀ alkyl group, a phenyl group, and a biphenyl group.

In one embodiment, R₁ to R₄ and R₁₁ to R₁₄ may each independently be selected from:
hydrogen, deuterium, -F, -Cl, -Br, -I, a hydroxyl group, a cyano group, a nitro group, an amidino group, a hydrazine group (a hydrazino group), and a hydrazone group (a hydrazono group);
a C₁-C₁₀ alkyl group, a C₂-C₁₀ alkenyl group, a C₂-C₁₀ alkynyl group, and a C₁-C₁₀ alkoxy group, each unsubstituted or substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group (a hydrazino group), a hydrazone group (a hydrazono group), a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a tetrahydro-2H-pyranyl group, a morpholinyl group, a phenyl group, a (C₁-C₁₀ alkyl)phenyl group, a di(C₁-C₁₀ alkyl)phenyl group, a biphenyl group, a di(phenyl)phenyl group, a terphenyl group, a naphthyl group, a pyridinyl group, a pyrimidinyl group, -O(Q₃₁), -S(Q₃₁), -Si(Q₃₁)(Q₃₂)(Q₃₃), -N(Q₃₁)(Q₃₂), -B(Q₃₁)(Q₃₂),-P(Q₃₁)(Q₃₂), -C(=O)(Q₃₁), -S(=O)₂(Q₃₁), and -P(=O)(Q₃₁)(Q₃₂);
a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a tetrahydro-2H-pyranyl group, a morpholinyl group, a dioxanyl group, a phenyl group, a (C₁-C₁₀ alkyl)phenyl group, a di(C₁-C₁₀ alkyl)phenyl group, a biphenyl group, a di(phenyl)phenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group (a thienyl group), a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group (a benzothienyl group), an benzoisothiazolyl group, a benzoxazolyl group, an benzoisoxazolyl group, a triazolyl group, a tetrazolyl group, an oxadiazolyl group, a triazinyl group, a dibenzofuranyl group, a dibenzothiophenyl group (a dibenzothienyl group), a benzocarbazolyl group, a dibenzocarbazolyl group, an imidazopyridinyl group, an imidazopyrimidinyl group, a 2,3-dihydro-1H-indenyl group, a 1,2,3,4-tetrahydronaphthalenyl group, a 2,3-dihydrobenzodioxinyl group, and a benzodioxanyl group, each unsubstituted or substituted with at least one selected from deuterium, -F, -Cl, -Br, -I, -CD₃, -CD₂H, - CDH₂, -CF₃, -CF₂H, -CFH₂, a hydroxyl group, a cyano group, a nitro group, an amidino group, a hydrazine group (a hydrazino group), a hydrazone group (a hydrazono group), a C₁-C₁₀ alkyl group, a C₂-C₁₀ alkenyl group, a C₂-C₁₀ alkynyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a tetrahydro-2H-pyranyl group, a morpholinyl group, a dioxanyl group, a phenyl group, a (C₁-C₁₀ alkyl)phenyl group, a di(C₁-C₁₀ alkyl)phenyl group, a biphenyl group, a di(phenyl)phenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, a 2,3-dihydro-1H-indenyl group, a 1,2,3,4-tetrahydronaphthalenyl group, a 2,3-dihydrobenzodioxinyl group, a benzodioxanyl group, -O(Q₃₁), -S(Q₃₁), -Si(Q₃₁)(Q₃₂)(Q₃₃), -N(Q₃₁)(Q₃₂),-B(Q₃₁)(Q₃₂), -P(Q₃₁)(Q₃₂), -C(=O)(Q₃₁), -S(=O)₂(Q₃₁), and -P(=O)(Q₃₁)(Q₃₂); and
-O(Q₁), -S(Q₁), -Si(Q₁)(Q₂)(Q₃), -N(Q₁)(Q₂), -B(Q₁)(Q₂), -P(Q₁)(Q₂),-C(=O)(Q₁), -S(=O)₂(Q₁), and -P(=O)(Q₁)(Q₂), and
Q₁ to Q₃ and Q₃₁ to Q₃₃ may each independently be selected from hydrogen, deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group (a hydrazino group), a hydrazone group (a hydrazono group), a C₁-C₁₀ alkyl group, a C₂-C₁₀ alkenyl group, a C₂-C₁₀ alkynyl group, a C₁-C₁₀ alkoxy group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a tetrahydro-2H-pyranyl group, a morpholinyl group, a dioxanyl group, a phenyl group, a (C₁-C₁₀ alkyl)phenyl group, a di(C₁-C₁₀ alkyl)phenyl group, a biphenyl group, a di(phenyl)phenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, a 2,3-dihydro-1H-indenyl group, a 1,2,3,4-tetrahydronaphthalenyl group, a 2,3-dihydrobenzodioxinyl group, and a benzodioxanyl group, but embodiments of the present disclosure are not limited thereto.

In one embodiment, R₁ to R₄ and R₁₁ to R₁₄ may each independently be selected from hydrogen, deuterium, -F, -Cl, -Br, -I, -CH₃, -CD₃, -CD₂H, -CDH₂, -CF₃,-CF₂H, -CFH₂, a hydroxyl group, a cyano group, a nitro group, an amidino group, a hydrazine group (a hydrazino group), a hydrazone group (a hydrazono group), and groups represented by Formulae 3-1(1) to 3-1(29), 3-2(1) to 3-2(21), 3-3(1) to 3-3(19), 3-4(1) to 3-4(4), 3-5(1) to 3-5(107), 3-6(1) to 3-6(10), and 3-7(1) to 3-7(27):

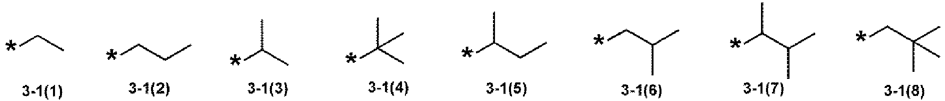

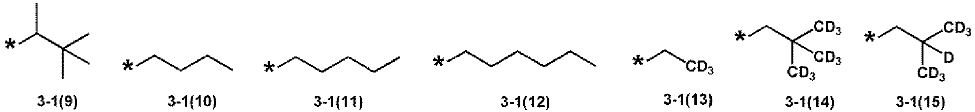

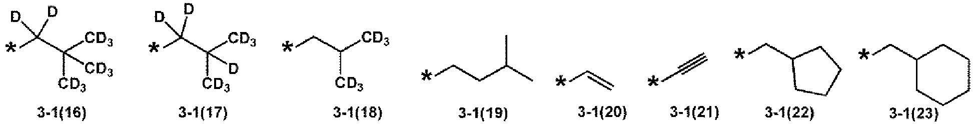

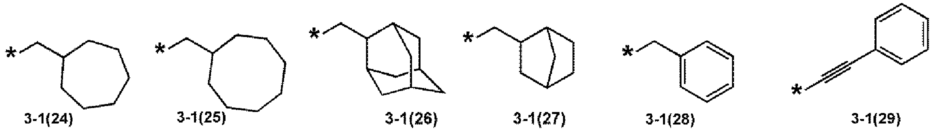

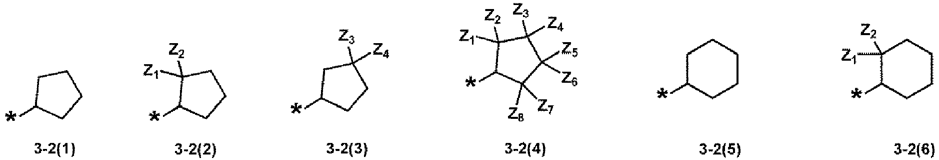

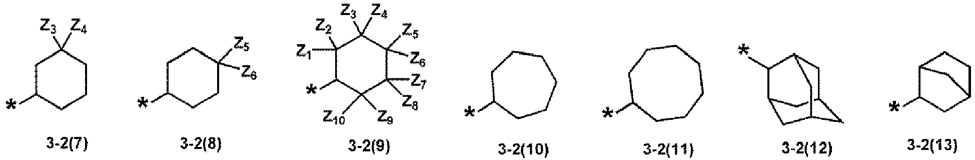

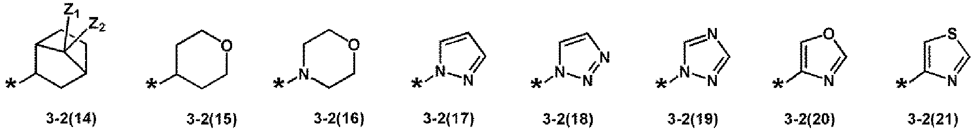

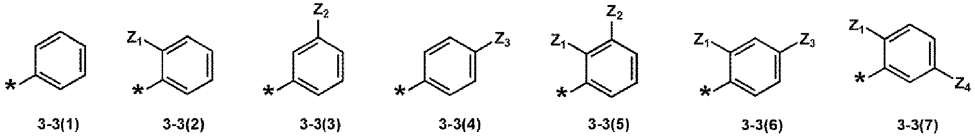

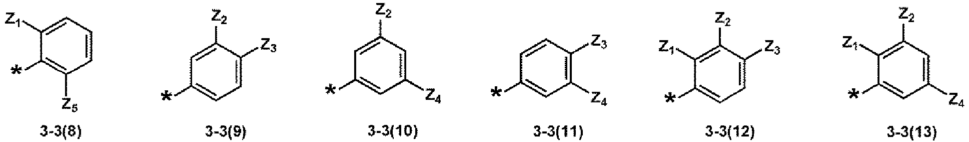

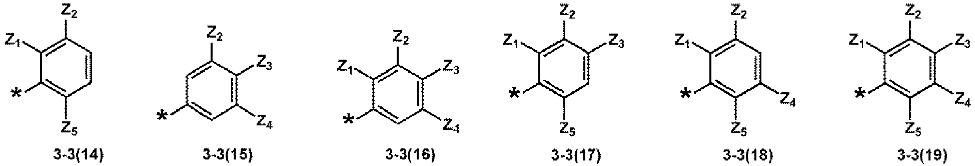

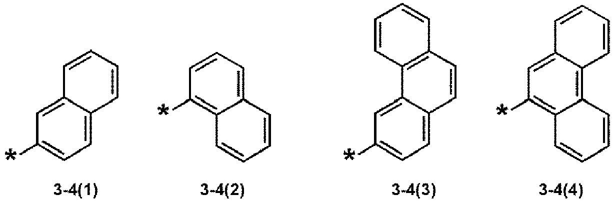

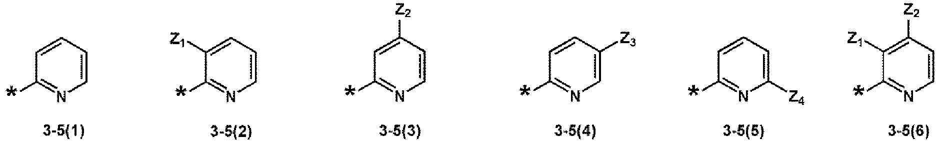

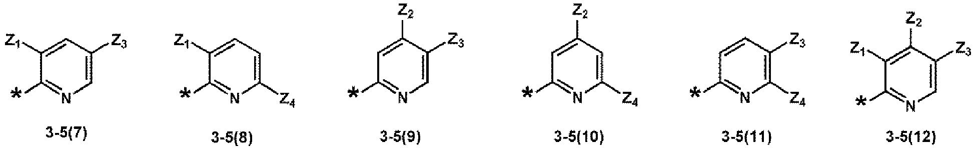

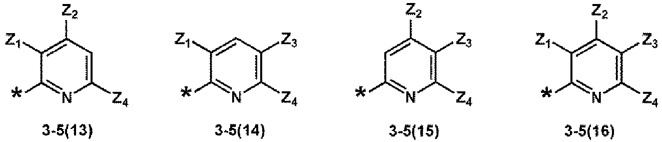

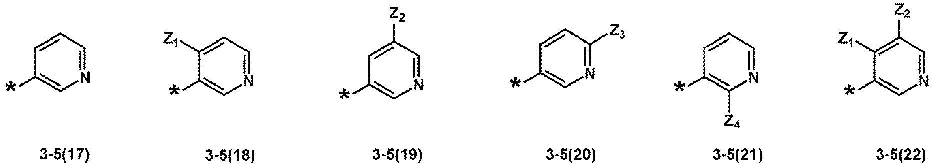

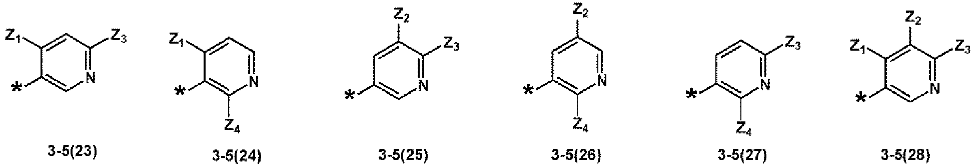

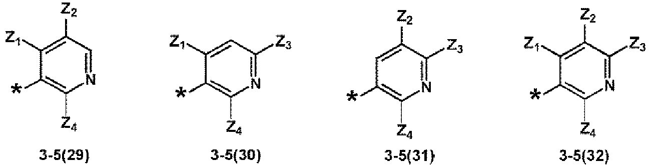

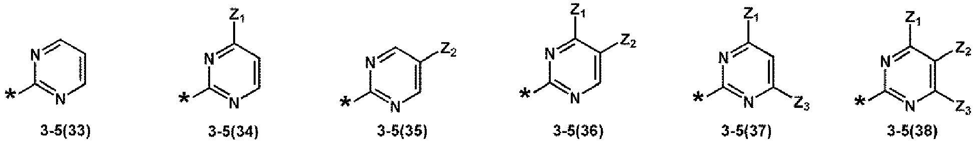

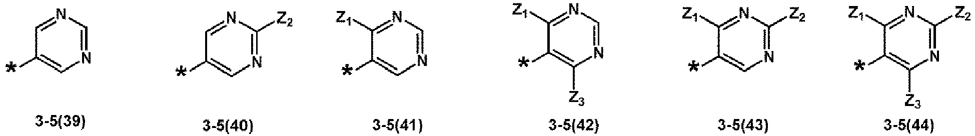

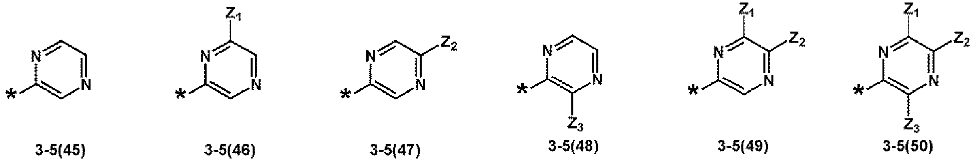

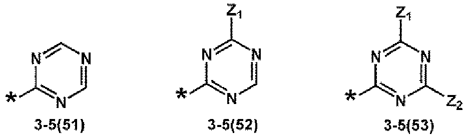

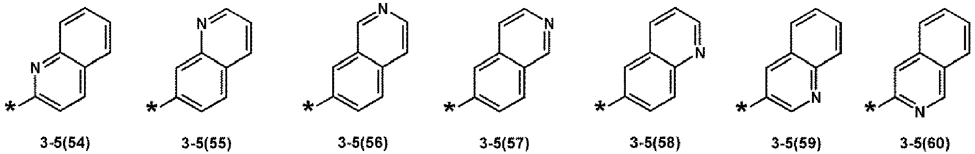

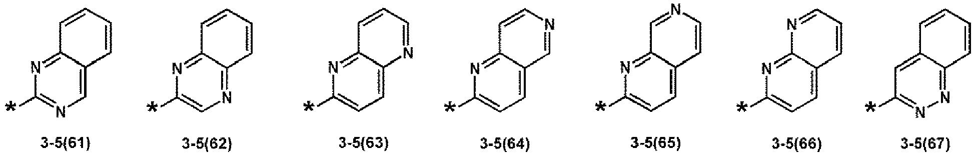

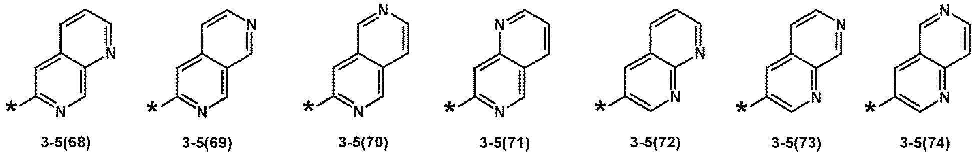

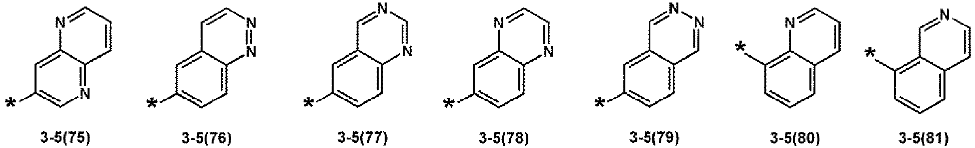

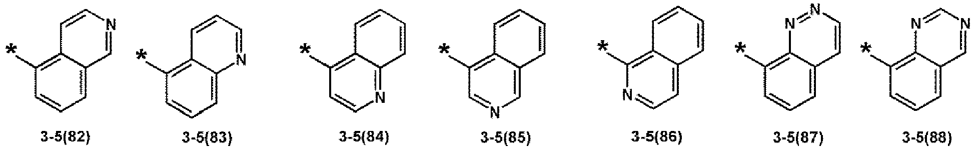

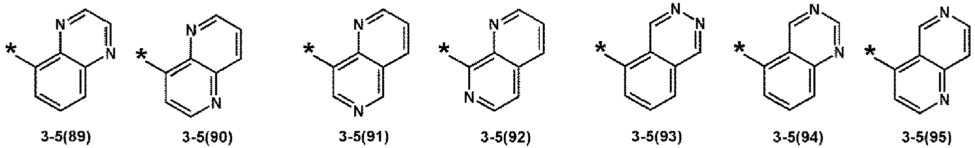

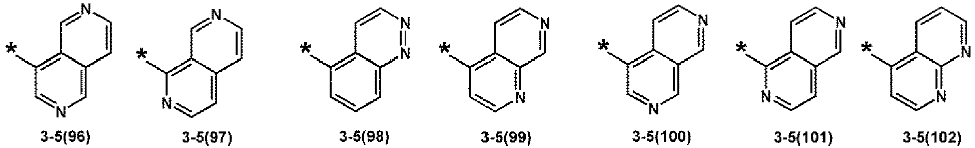

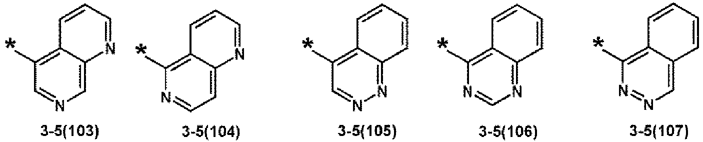

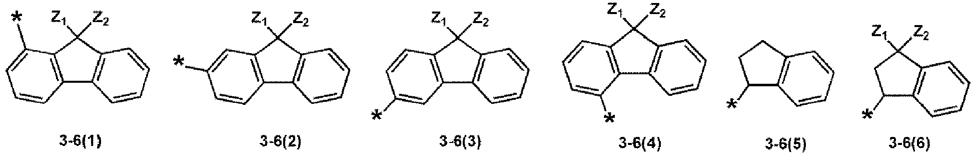

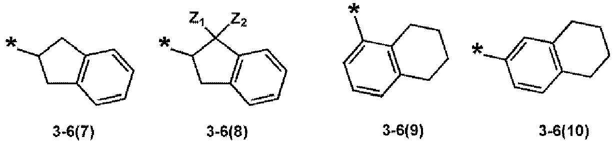

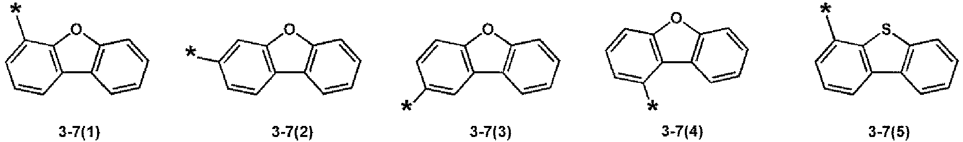

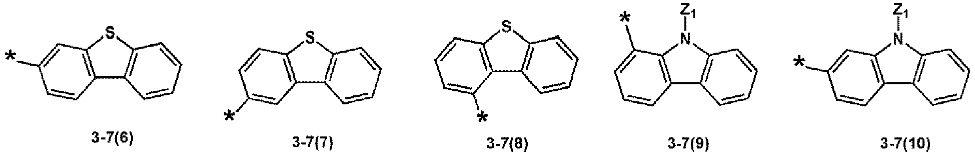

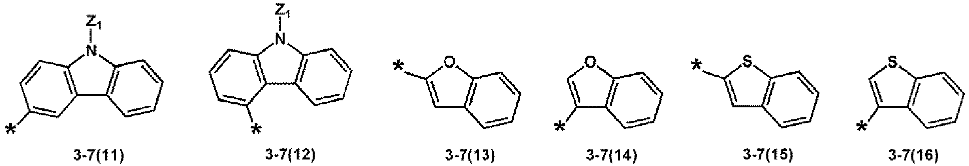

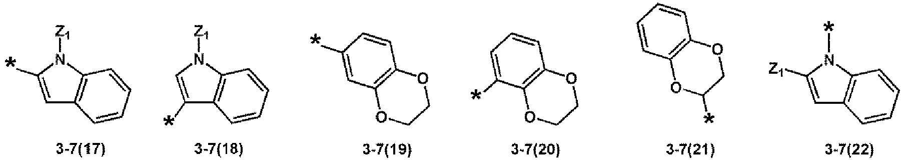

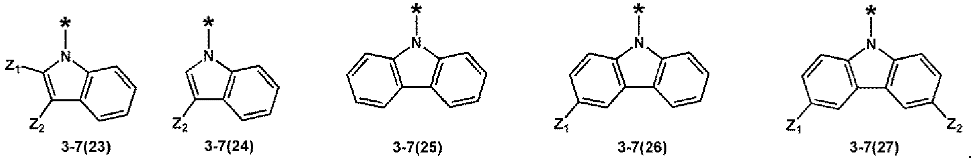

In Formulae 3-1(1) to 3-1(29), 3-2(1) to 3-2(21), 3-3(1) to 3-3(19), 3-4(1) to 3-4(4), 3-5(1) to 3-5(107), 3-6(1) to 3-6(10), and 3-7(1) to 3-7(27),
Z₁ to Z₁₀ are each independently selected from deuterium, -F, -Cl, -Br, -I,-CD₃, -CD₂H, -CDH₂, -CF₃, -CF₂H, -CFH₂, a hydroxyl group, a cyano group, a nitro group, an amidino group, a hydrazine group (a hydrazino group), a hydrazone group (a hydrazono group), a C₁-C₁₀ alkyl group, a C₂-C₁₀ alkenyl group, a C₂-C₁₀ alkynyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a tetrahydro-2H-pyranyl group, a morpholinyl group, a dioxanyl group, a phenyl group, a (C₁-C₁₀ alkyl)phenyl group, a di(C₁-C₁₀ alkyl)phenyl group, a biphenyl group, a di(phenyl)phenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, a 2,3-dihydro-1H-indenyl group, a 1,2,3,4-tetrahydronaphthalenyl group, a 2,3-dihydrobenzodioxinyl group, a benzodioxanyl group, -O(Q₃₁), -S(Q₃₁), -Si(Q₃₁)(Q₃₂)(Q₃₃), -N(Q₃₁)(Q₃₂),-B(Q₃₁)(Q₃₂), -P(Q₃₁)(Q₃₂), -C(=O)(Q₃₁), -S(=O)₂(Q₃₁), and -P(=O)(Q₃₁)(Q₃₂), and
Q₃₁ to Q₃₃ may each independently be the same as defined above, and
* indicates a binding site to a neighboring atom.

In Formula 1, a1 to a4 each indicates the number of R₁ to R₄, and are each independently an integer from 0 to 10 (for example, an integer from 0 to 3). When a1 is two or more, two or more of R₁(s) may be identical to or different from each other, when a2 is two or more, two or more of R₂(s) may be identical to or different from each other, when a3 is two or more, two or more of R₃(s) may be identical to or different from each other, and when a4 is two or more, two or more of R₄(s) may be identical to or different from each other.

In one embodiment, R₁ to R₄ may each independently be selected from hydrogen, deuterium, -F, -Cl, -Br, -I, -CD₃, -CD₂H, -CDH₂, -CF₃, -CF₂H, -CFH₂, C₁-C₁₀ alkyl group, a C₂-C₁₀ alkenyl group, a C₂-C₁₀ alkynyl group, a C₁-C₁₀ alkoxy group, a cyclopentyl group, a cyclohexyl group, a cycloheptyl group, a phenyl group, a (C₁-C₁₀ alkyl)phenyl group, a di(C₁-C₁₀ alkyl)phenyl group, a biphenyl group, a di(phenyl)phenyl group, a terphenyl group, -O(Q₁), and -S(Q₁), and
Q₁ may be selected from a C₁-C₁₀ alkyl group, a C₂-C₁₀ alkenyl group, a C₂-C₁₀ alkynyl group, a C₁-C₁₀ alkoxy group, a cyclopentyl group, a cyclohexyl group, a cycloheptyl group, a phenyl group, a (C₁-C₁₀ alkyl)phenyl group, a di(C₁-C₁₀ alkyl)phenyl group, a biphenyl group, a di(phenyl)phenyl group, and a terphenyl group.

In one or more embodiments, at least one selected from R₁₁ to R₁₄ (for example, all of R₁₁ to R₁₄) may each independently be selected from a C₂-C₁₀ alkyl group, a C₂-C₁₀ alkenyl group, a C₂-C₁₀ alkynyl group, and a C₁-C₁₀ alkoxy group, each unsubstituted or substituted with at least one selected from deuterium, -F, -Cl, -Br, -I, - CD₃, -CD₂H, -CDH₂, -CF₃, -CF₂H, -CFH₂, a hydroxyl group, a cyano group, a nitro group, an amidino group, a hydrazine group (a hydrazino group), a hydrazone group (a hydrazono group), a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a tetrahydro-2H-pyranyl group, a morpholinyl group, a phenyl group, a (C₁-C₁₀ alkyl)phenyl group, a di(C₁-C₁₀ alkyl)phenyl group, a biphenyl group, a di(phenyl)phenyl group, a terphenyl group, a naphthyl group, a pyridinyl group, a pyrimidinyl group, -O(Q₃₁), -S(Q₃₁),-Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁), -S(=O)₂(Q₃₁), and -P(=O)(Q₃₁)(Q₃₂).

In one or more embodiments, at least one selected from R₁₁ to R₁₄ may be neither of hydrogen, a phenyl group, a mesityl group, or a methyl group.

In one or more embodiments, at least one selected from R₁₁ to R₁₄ may each independently be selected from -O(Q₁), -S(Q₁), -Si(Q₁)(Q₂)(Q₃), -N(Q₁)(Q₂), -B(Q₁)(Q₂), -P(Q₁)(Q₂), -C(=O)(Q₁), -S(=O)₂(Q₁), -P(=O)(Q₁)(Q₂), and a group represented by Formula A5: 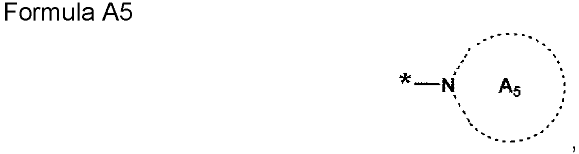
wherein, Q₁ to Q₃ may each independently be the same as defined above, and
A₅ in Formula A5 is a substituted or unsubstituted C₁-C₁₀ heterocycloalkyl group, a substituted or unsubstituted C₁-C₁₀ heterocycloalkenyl group, a substituted or unsubstituted C₁-C₆₀ (*e.g.* C₁-C₂₀) heteroaryl group, or a substituted or unsubstituted monovalent non-aromatic condensed heteropolycyclic group, and may be defined the same as R₁₁ above.

For example, a group belonging to Formula A5 may be represented by one selected from Formulae 3-2(16) to 3-2(19) and 3-7(22) to 3-7(27), but embodiments of the present disclosure are not limited thereto.

In one or more embodiments, R₁₁ to R₁₄ may be identical to each other.

In one or more embodiments, R₁₁ and R₁₂ may be different from each other, or R₁₁ and R₁₃ may be different from each other.

In one or more embodiments, in Formula 1,
a) R₁₁ and R₁₂ may be different from each other, R₁₃ and R₁₄ may be different from each other, R₁₁ and R₁₃ may be identical to each other, and R₁₂ and R₁₄ may be identical to each other;
b) R₁₁ and R₁₃ may be different from each other, R₁₂ and R₁₄ may be different from each other, R₁₁ and R₁₄ may be identical to each other, and R₁₂ and R₁₃ may be identical to each other;
c) R₁₁ and R₁₂ may be different from each other, and R₁₁, R₁₃, and R₁₄ may be identical to each other; or
d) R₁₁ to R₁₄ may be all different from each other, but embodiments of the present disclosure are not limited thereto.

For example, the organometallic compound represented by Formula 1 may be selected from Compounds 1 to 79, 82 to 116, 118 to 169 and 172 to 180, but embodiments of the present disclosure are not limited thereto:

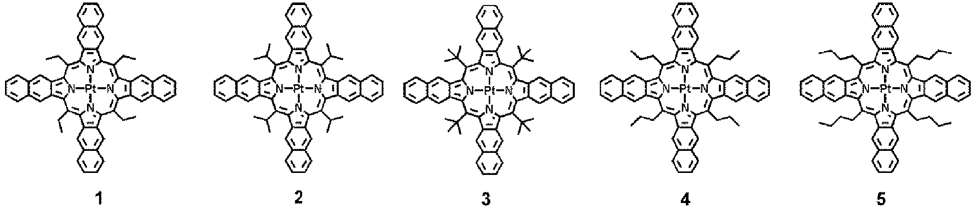

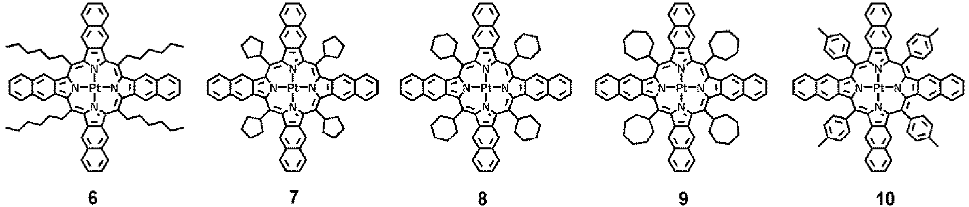

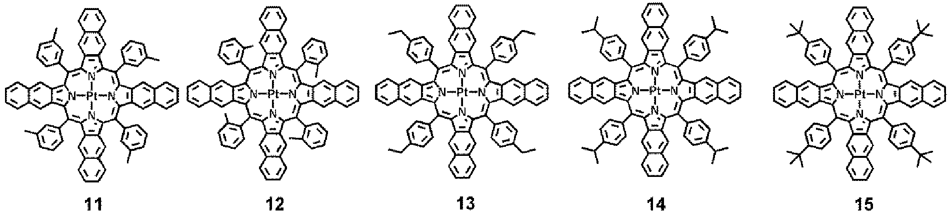

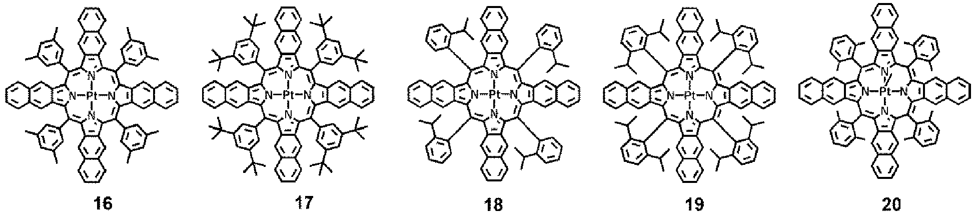

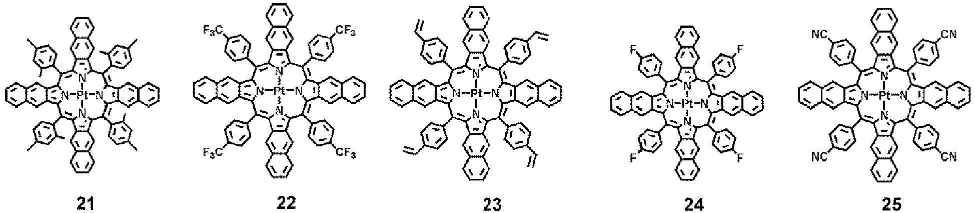

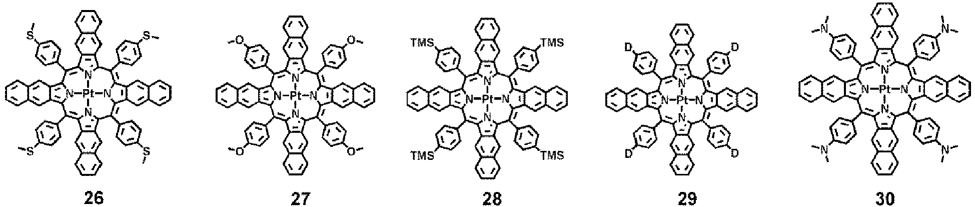

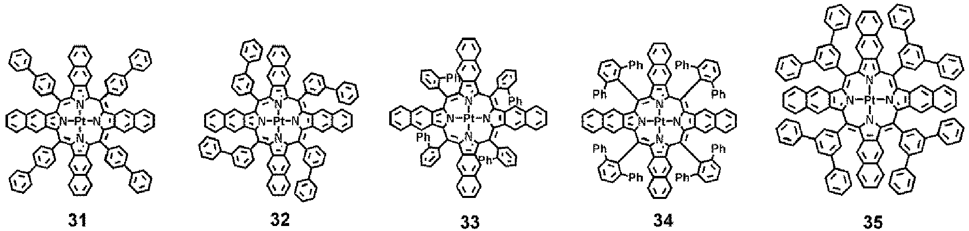

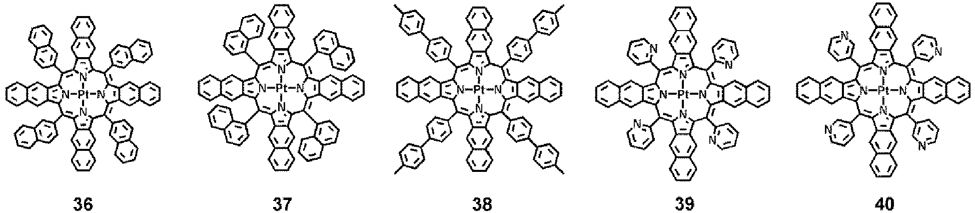

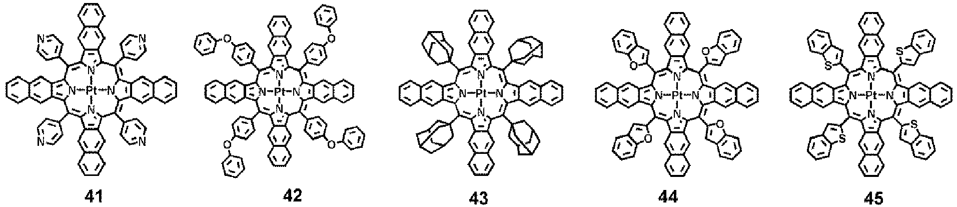

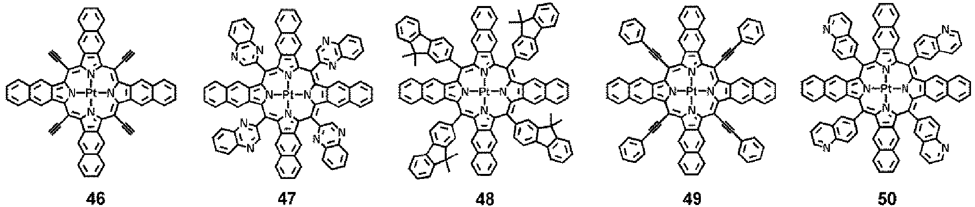

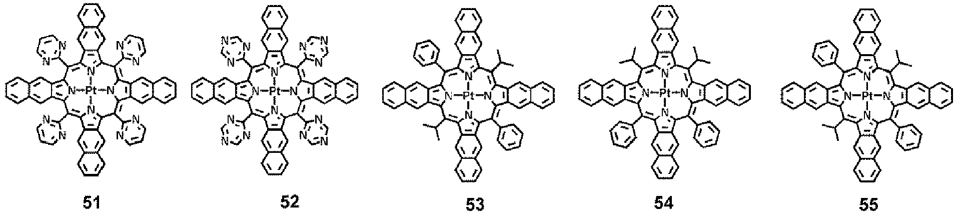

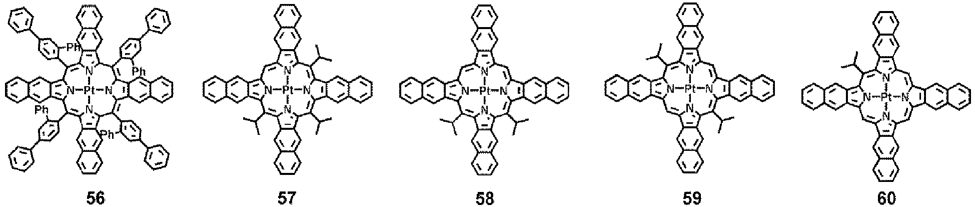

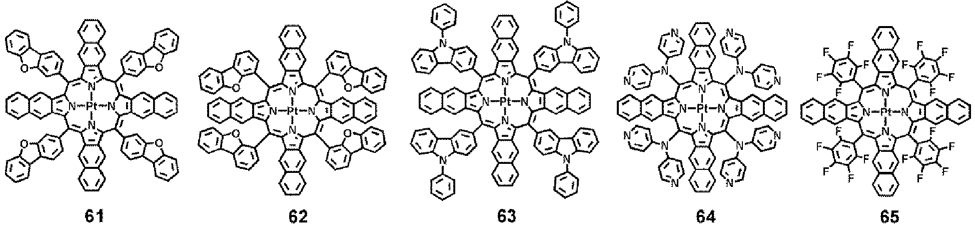

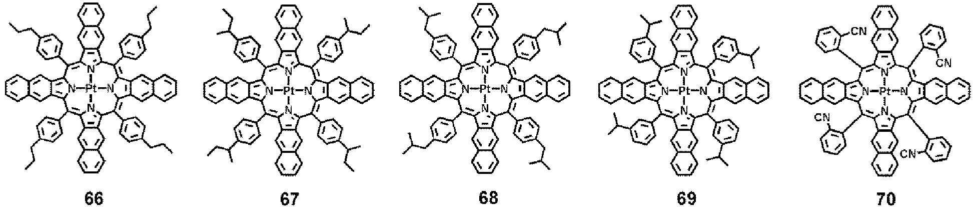

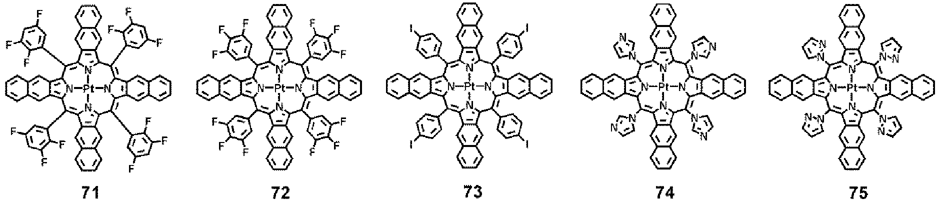

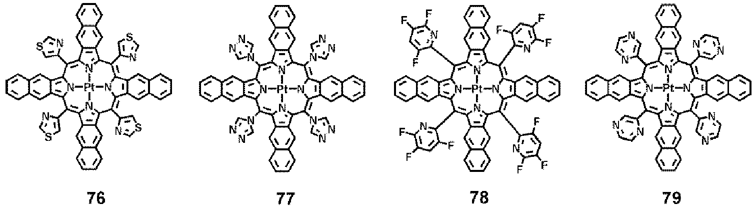

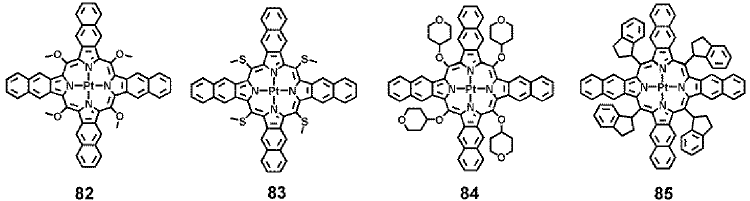

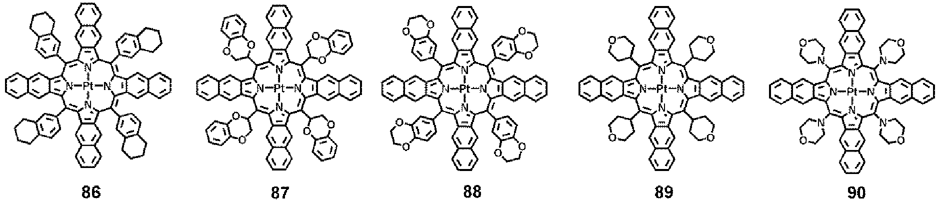

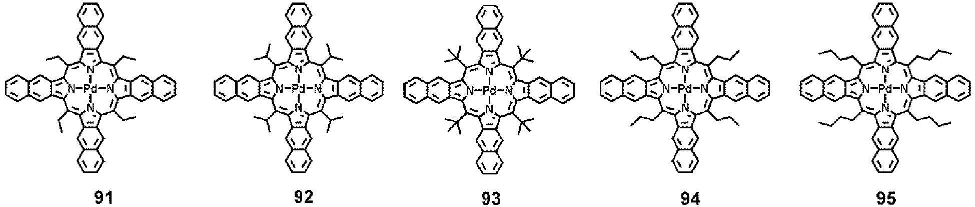

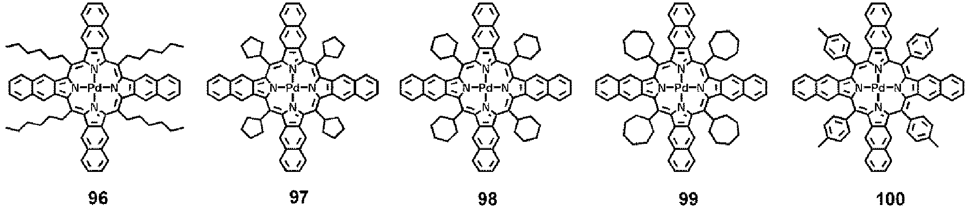

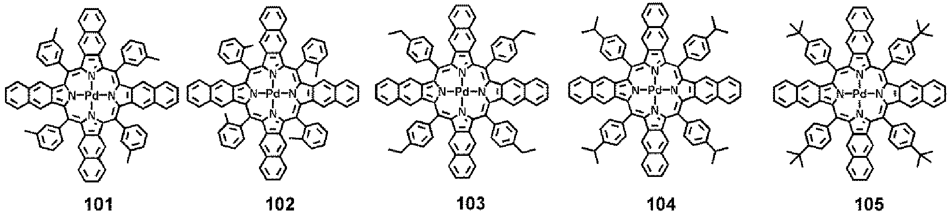

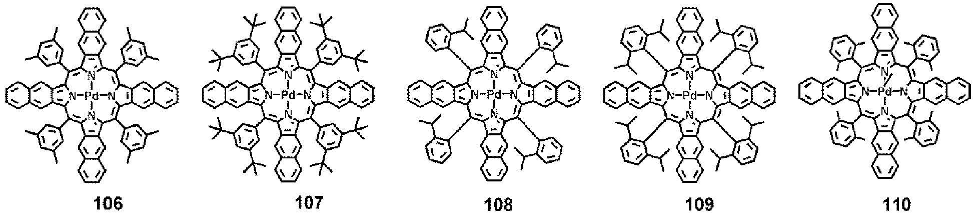

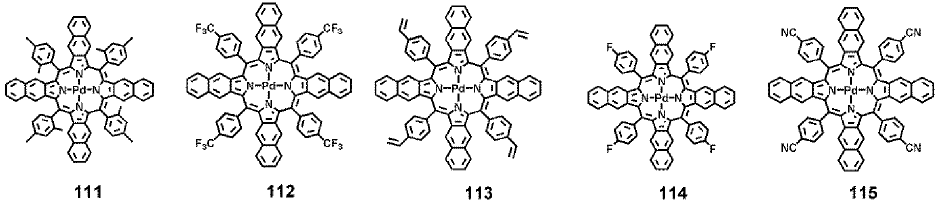

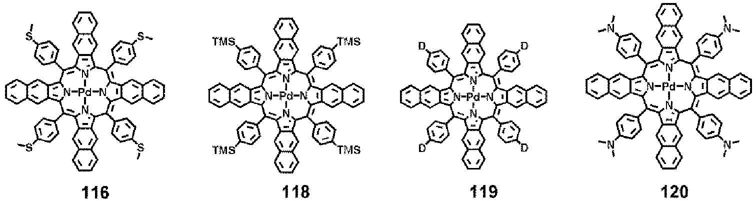

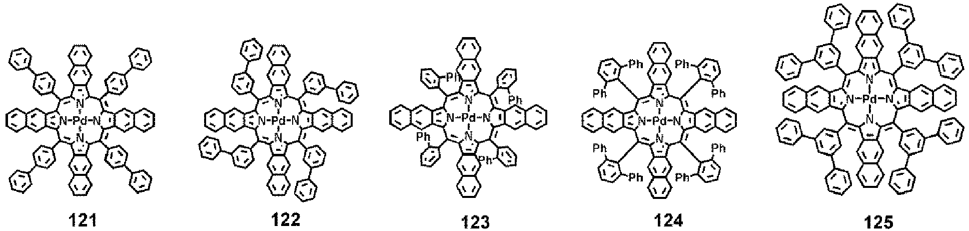

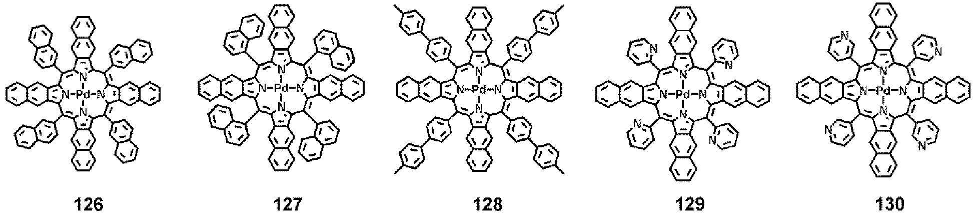

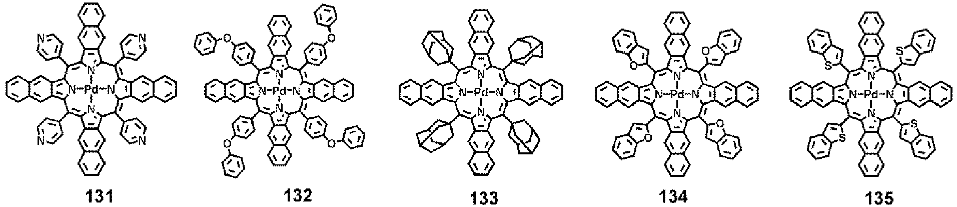

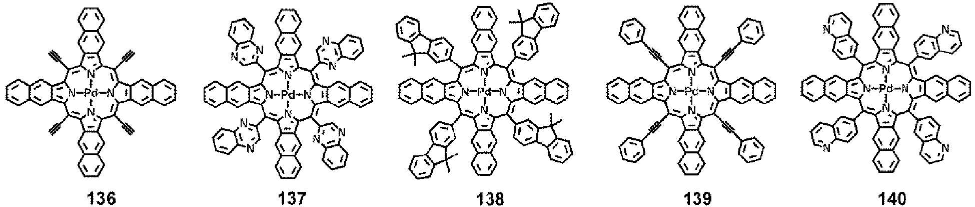

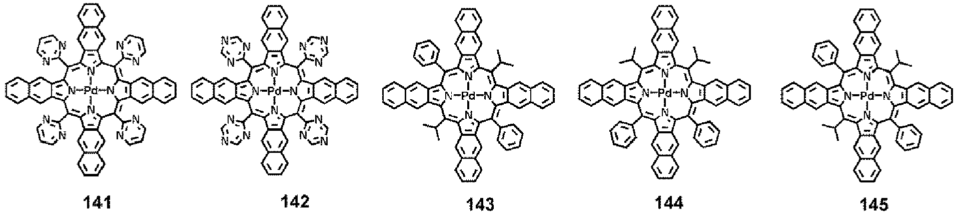

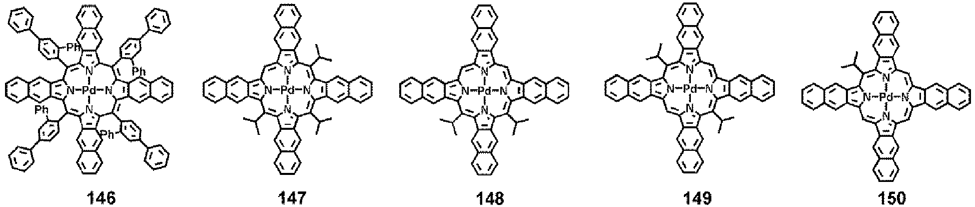

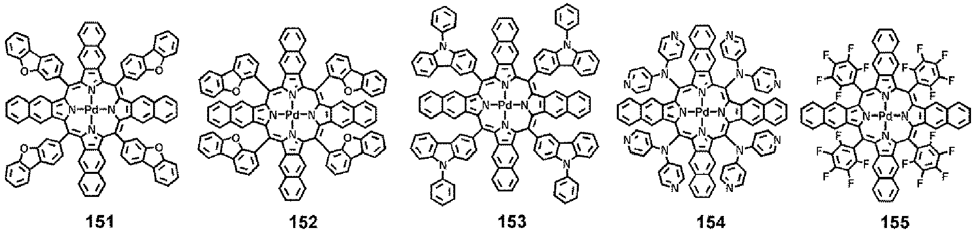

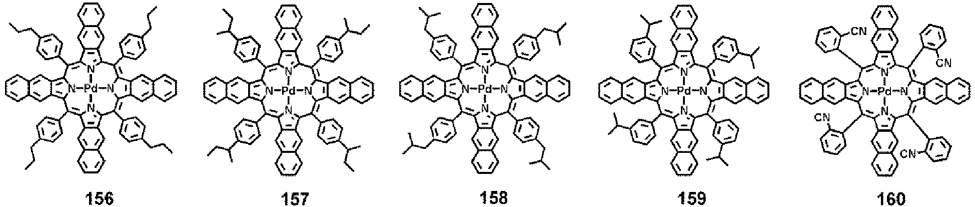

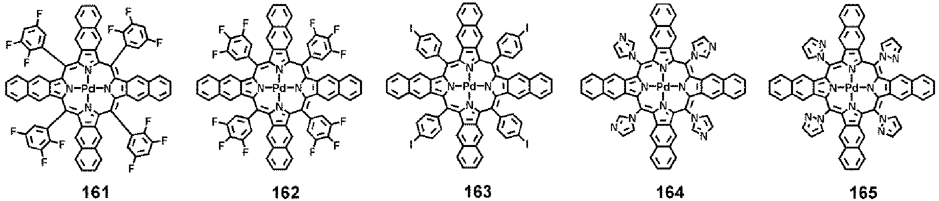

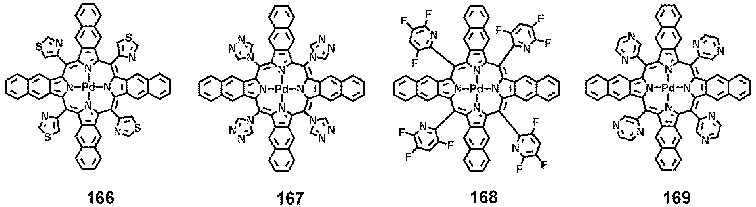

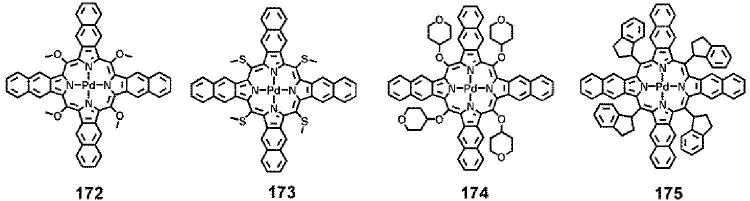

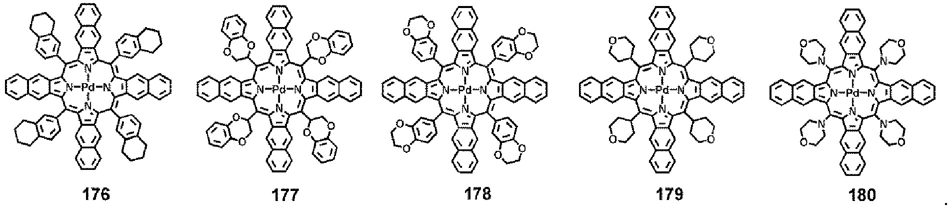

In Compounds 1 to 79, 82 to 116, 118 to 169 and 172 to 180, TMS indicates a trimethylsilyl group, and Ph indicates a phenyl group.

In one embodiment, in Formula 1, M is selected from a transition metal (for example, a first-row transition metal, a second-row transition metal, or a third-row transition metal, of the Periodic Table of Elements), and ring A₁ to ring A₄ are each independently a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, a pyrrole group, an indole group, an indene group, a benzosilole group, a benzothiophene group, a benzofuran group, a carbazole group, a fluorene group, a dibenzosilole group, a dibenzothiophene group, a dibenzofuran group, a pyridine group, a pyrimidine group, a pyrazine group, a pyridazine group, a triazine group, a quinoline group, an isoquinoline group, a quinazoline group, a phenanthroline group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group. Accordingly, the organometallic compound emits near-infrared phosphorescence (e.g. near-infrared phosphorescent light) having a maximum emission wavelength in a range from 720 nm to 2,500 nm.

In Formula 1, a) R₁₁ to R₁₄ are not simultaneously hydrogen, b) R₁₁ to R₁₄ are not simultaneously a phenyl group, c) R₁₁ to R₁₄ are not simultaneously a mesityl group, or d) R₁₁ to R₁₄ are not simultaneously a methyl group, wherein two selected from R₁₁ to R₁₄ are not linked to each other. Various embodiments of the groups R₁₁ to R₁₄ in Formula 1 are described above in the present specification. As R₁₁ to R₁₄ in Formula 1 are the same as defined above, the organometallic compound represented by Formula 1 may be synthesized with high purity and may have high heat resistance. In this regard, a film formed by depositing the organometallic compound may have a high film density. Thus, when such a film is exposed to heat generated by operation of an electronic device including the film, such as an organic light-emitting device, the decomposition of the organometallic compound included in the film may be substantially prevented or reduced. Therefore, an electronic device, such as an organic light-emitting device, including the organometallic compound represented by Formula 1, may have low driving voltage, high emission efficiency, and/or long lifespan.

A synthesis method for the organometallic compound represented by Formula 1 would be apparent to those of ordinary skill in the art by referring to the following examples.

At least one of the organometallic compound of Formula 1 may be used between a pair of electrodes of an organic light-emitting device. Accordingly, another aspect of an embodiment provides an organic light-emitting device including: a first electrode; a second electrode facing the first electrode; and an organic layer between the first electrode and the second electrode, the organic layer including an emission layer, wherein the emission layer includes at least one organometallic compound represented by Formula 1 as described herein and emits near-infrared phosphorescent light having a maximum emission wavelength in a range from 720 nm to 2,500 nm.

The expression "(an organic layer) includes at least one organometallic compound," as used herein, may include a case in which "(an organic layer) includes identical organometallic compounds represented by Formula 1" and a case in which "(an organic layer) includes two or more different organometallic compounds represented by Formula 1."

For example, the organic layer may include, as the organometallic compound, only Compound 1. In this regard, Compound 1 may exist in an emission layer of the organic light-emitting device. In one or more embodiments, the organic layer may include, as the organometallic compound, Compound 1 and Compound 2. In this regard, Compound 1 and Compound 2 may exist in an identical layer (for example, Compound 1 and Compound 2 may all exist in an emission layer), or different layers (for example, Compound 1 may exist in an emission layer and Compound 2 may exist in an electron transport region).

In the organic light-emitting device according to one embodiment,
the first electrode of the organic light-emitting device may be an anode,
the second electrode of the organic light-emitting device may be a cathode,
the organic layer may further include a hole transport region between the first electrode and the emission layer and an electron transport region between the emission layer and the second electrode,
the hole transport region may include a hole injection layer, a hole transport layer, an emission auxiliary layer, an electron blocking layer, or any combination thereof, and
the electron transport region may include a hole blocking layer, an electron transport layer, an electron injection layer, or any combination thereof.

The term "organic layer," as used herein, refers to a single layer and/or a plurality of layers between the first electrode and the second electrode of an organic light-emitting device. A material included in the "organic layer" is not limited to an organic material.

The emission layer includes the organometallic compound represented by Formula 1 as described herein. The emission layer may further include, in addition to the organometallic compound, a host, wherein an amount of the organometallic compound may be smaller than that of the host in the emission layer. Here, the emission layer emits near-infrared phosphorescent light having a maximum emission wavelength in a range from 720 nm to 2,500 nm.

In one or more embodiments, the hole transport region may include an electron blocking layer, and the electron blocking layer may include the organometallic compound; and/or the electron transport region may include a hole blocking layer, and the hole blocking layer may include the organometallic compound. The organometallic compound included in the electron blocking layer or the hole blocking layer does not serve as an emitter.

In one or more embodiments,
at least one selected from the hole transport region and the emission layer may include at least one selected from an arylamine group-containing compound, an acridine ring-containing compound, and a carbazole ring-containing compound; and/or
at least one selected from the emission layer and the electron transport region may include at least one selected from a silicon-containing compound, a phosphine oxide group-containing compound, a sulfur oxide group-containing compound, a triazine ring-containing compound, a pyrimidine ring-containing compound, a pyridine ring-containing compound, a dibenzofuran ring-containing compound, and a dibenzothiophene ring-containing compound.

### Description of the Drawing

The accompanying drawing is a schematic view of an organic light-emitting device 10 according to an embodiment. The organic light-emitting device 10 includes a first electrode 110, an organic layer 150, and a second electrode 190.

Hereinafter, the structure of the organic light-emitting device 10 according to an embodiment and a method of manufacturing the organic light-emitting device 10 will be described in connection with the accompanying drawing.

### First electrode 110

In the accompanying drawing, a substrate may be additionally disposed under the first electrode 110 or above the second electrode 190. The substrate may be a glass substrate or a plastic substrate, each having excellent mechanical strength, thermal stability, transparency, surface smoothness, ease of handling, and water resistance.

The first electrode 110 may be formed by depositing or sputtering a material for forming the first electrode 110 on the substrate. When the first electrode 110 is an anode, the material for forming the first electrode 110 may be selected from materials with a high work function to facilitate hole injection.

The first electrode 110 may be a reflective electrode, a semi-transmissive electrode, or a transmissive electrode. When the first electrode 110 is a transmissive electrode, a material for forming a first electrode 110 may be selected from indium tin oxide (ITO), indium zinc oxide (IZO), tin oxide (SnO₂), zinc oxide (ZnO), and any combinations thereof, but embodiments of the present disclosure are not limited thereto. When the first electrode 110 is a semi-transmissive electrode or a reflective electrode, as a material for forming the first electrode 110, magnesium (Mg), silver (Ag), aluminium (Al), aluminium-lithium (Al-Li), calcium (Ca), magnesium-indium (Mg-In), magnesium-silver (Mg-Ag), or any combination thereof may be used. However, the material for forming the first electrode 110 is not limited thereto.

The first electrode 110 may have a single-layered structure, or a multi-layered structure including two or more layers. For example, the first electrode 110 may have a three-layered structure of ITO/Ag/ITO, but the structure of the first electrode 110 is not limited thereto.

### Organic layer 150

The organic layer 150 is disposed on the first electrode 110. The organic layer 150 may include an emission layer.

The organic layer 150 may further include a hole transport region between the first electrode 110 and the emission layer, and an electron transport region between the emission layer and the second electrode 190.

### [Hole transport region in organic layer 150]

The hole transport region may have i) a single-layered structure including a single layer including a single material, ii) a single-layered structure including a single layer including a plurality of different materials, or iii) a multi-layered structure having a plurality of layers including a plurality of different materials.

The hole transport region may include at least one layer selected from a hole injection layer, a hole transport layer, an emission auxiliary layer, and an electron blocking layer.

For example, the hole transport region may have a single-layered structure including a single layer including a plurality of different materials, or a multi-layered structure having a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron blocking layer structure, wherein for each structure, constituting layers are sequentially stacked from the first electrode 110 in this stated order, but the structure of the hole transport region is not limited thereto.

The hole transport region may include at least one selected from m-MTDATA, TDATA, 2-TNATA, NPB(NPD), β-NPB, TPD, Spiro-TPD, Spiro-NPB, methylated-NPB, TAPC, HMTPD, 4,4',4"-tris(N-carbazolyl)triphenylamine (TCTA), polyaniline/dodecylbenzenesulfonic acid (PANI/DBSA), PEDOT/PSS (poly(3,4-ethylenedioxythiophene)/poly(4-styrenesulfonate)), polyaniline/camphor sulfonic acid (PANI/CSA), polyaniline/poly(4-styrenesulfonate) (PANI/PSS), a compound represented by Formula 201, and a compound represented by Formula 202:

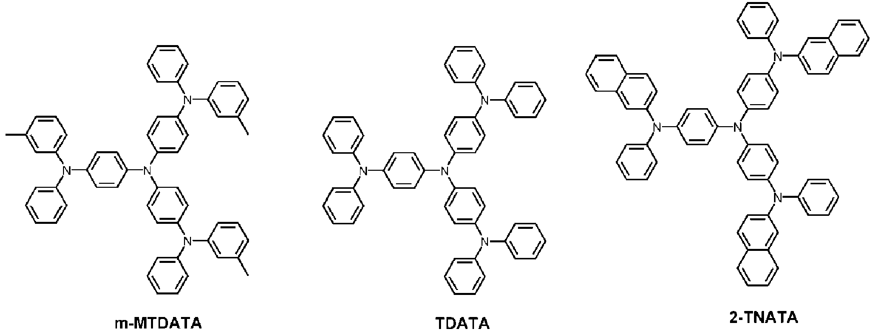

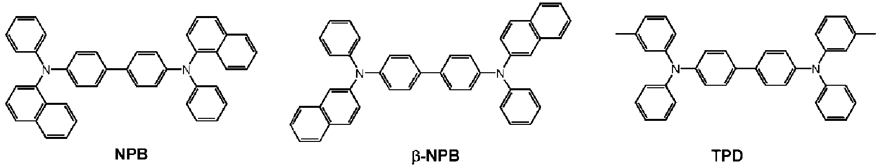

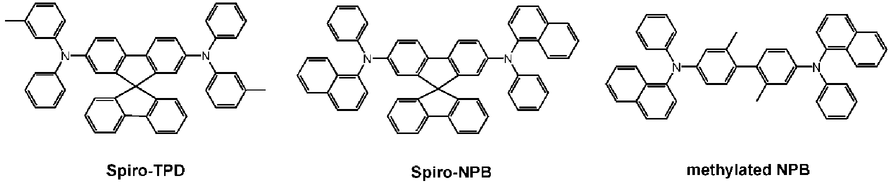

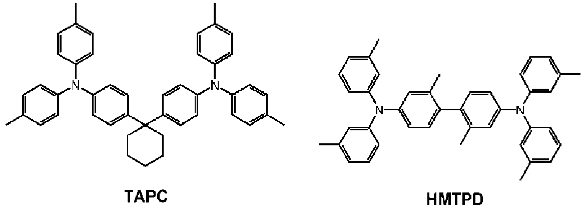

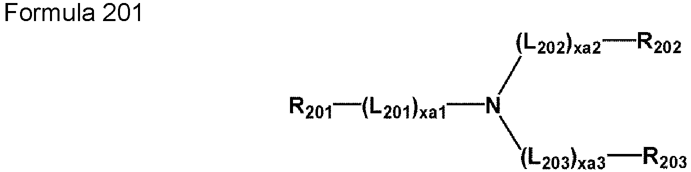

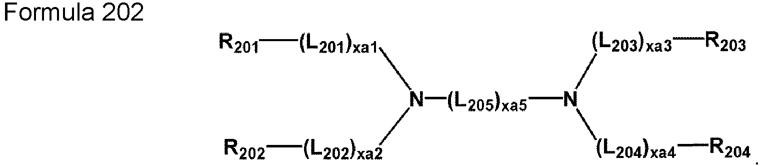

In Formulae 201 and 202,
L₂₀₁ to L₂₀₄ may each independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ (e.g. C₆-C₃₂) arylene group, a substituted or unsubstituted C₁-C₆₀ (e.g. C₁-C₂₀) heteroarylene group, a substituted or unsubstituted divalent non-aromatic condensed polycyclic group, and a substituted or unsubstituted divalent non-aromatic condensed heteropolycyclic group,
L₂₀₅ may be selected from *-O-*', *-S-*', *-N(Q₂₀₁)-*', a substituted or unsubstituted C₁-C₂₀ alkylene group, a substituted or unsubstituted C₂-C₂₀ alkenylene group, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ (e.g. C₆-C₃₂) arylene group, a substituted or unsubstituted C₁-C₆₀ (e.g. C₁-C₂₀) heteroarylene group, a substituted or unsubstituted divalent non-aromatic condensed polycyclic group, and a substituted or unsubstituted divalent non-aromatic condensed heteropolycyclic group,
xa1 to xa4 may each independently be an integer from 0 to 3,
xa5 may be an integer from 1 to 10, and
R₂₀₁ to R₂₀₄ and Q₂₀₁ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (*e.g.* C₆-C₃₂) aryl group, a substituted or unsubstituted C₆-C₆₀ (*e.g.* C₆-C₃₂) aryloxy group, a substituted or unsubstituted C₆-C₆₀ (e.g. C₆-C₃₂) arylthio group, a substituted or unsubstituted C₁-C₆₀ (*e.g.* C₁-C₂₀) heteroaryl group, a substituted or unsubstituted monovalent non-aromatic condensed polycyclic group, and a substituted or unsubstituted monovalent non-aromatic condensed heteropolycyclic group.

For example, in Formula 202, R₂₀₁ and R₂₀₂ may optionally be linked to each other via a single bond, a dimethyl-methylene group, or a diphenyl-methylene group, and R₂₀₃ and R₂₀₄ may optionally be linked to each other via a single bond, a dimethyl-methylene group, or a diphenyl-methylene group.

In one or more embodiments, in Formulae 201 and 202,
L₂₀₁ to L₂₀₅ may each independently be selected from:
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group; and
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group, each substituted with at least one selected from deuterium, -F, -Cl, -Br, -I, a hydroxyl group, a cyano group, a nitro group, an amidino group, a hydrazine group (a hydrazino group), a hydrazone group (a hydrazono group), a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, -Si(Q₃₁)(Q₃₂)(Q₃₃), and -N(Q₃₁)(Q₃₂), and
Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

In one or more embodiments, xa1 to xa4 may each independently be 0, 1, or 2.

In one or more embodiments, xa5 may be 1, 2, 3 ,or 4.

In one or more embodiments, R₂₀₁ to R₂₀₄ and Q₂₀₁ may each independently be selected from:
a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group; and
a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group, each substituted with at least one selected from deuterium, -F, -Cl, -Br, -I, a hydroxyl group, a cyano group, a nitro group, an amidino group, a hydrazine group (a hydrazino group), a hydrazone group (a hydrazono group), a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, -Si(Q₃₁)(Q₃₂)(Q₃₃), and -N(Q₃₁)(Q₃₂), and
Q₃₁ to Q₃₃ may each independently be the same as defined above.

In one or more embodiments, in Formula 201, at least one selected from R₂₀₁ to R₂₀₃ may each independently be selected from:
a fluorenyl group, a spiro-bifluorenyl group, a carbazolyl group, a dibenzofuranyl group, and a dibenzothiophenyl group; and
a fluorenyl group, a spiro-bifluorenyl group, a carbazolyl group, a dibenzofuranyl group, and a dibenzothiophenyl group, each substituted with at least one selected from deuterium, -F, -Cl, -Br, -I, a hydroxyl group, a cyano group, a nitro group, an amidino group, a hydrazine group (a hydrazino group), a hydrazone group (a hydrazono group), a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a naphthyl group, a fluorenyl group, a spiro-bifluorenyl group, a carbazolyl group, a dibenzofuranyl group, and a dibenzothiophenyl group,
but embodiments of the present disclosure are not limited thereto.

In one or more embodiments, in Formula 202, i) R₂₀₁ and R₂₀₂ may be linked via a single bond, and/or ii) R₂₀₃ and R₂₀₄ may be linked via a single bond.

In one or more embodiments, in Formula 202, at least one selected from R₂₀₁ to R₂₀₄ may be selected from:
a carbazolyl group; and
a carbazolyl group substituted with at least one selected from deuterium, -F, -Cl, -Br, -I, a hydroxyl group, a cyano group, a nitro group, an amidino group, a hydrazine group (a hydrazino group), a hydrazone group (a hydrazono group), a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a naphthyl group, a fluorenyl group, a spiro-bifluorenyl group, a carbazolyl group, a dibenzofuranyl group, and a dibenzothiophenyl group,
but embodiments of the present disclosure are not limited thereto.

The compound represented by Formula 201 may be represented by Formula 201A:

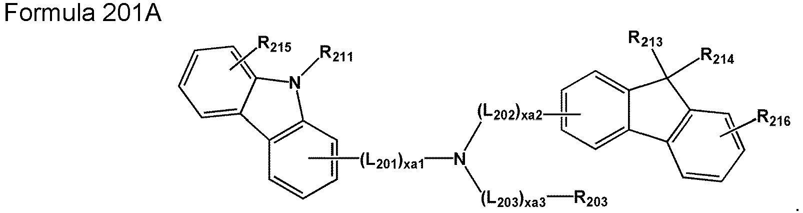

In one embodiment, the compound represented by Formula 201 may be represented by Formula 201A(1) below, but embodiments of the present disclosure are not limited thereto:

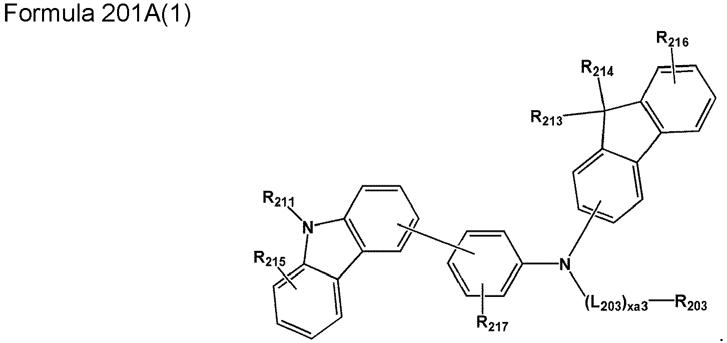

In one embodiment, the compound represented by Formula 201 may be represented by Formula 201A-1 below, but embodiments of the present disclosure are not limited thereto:

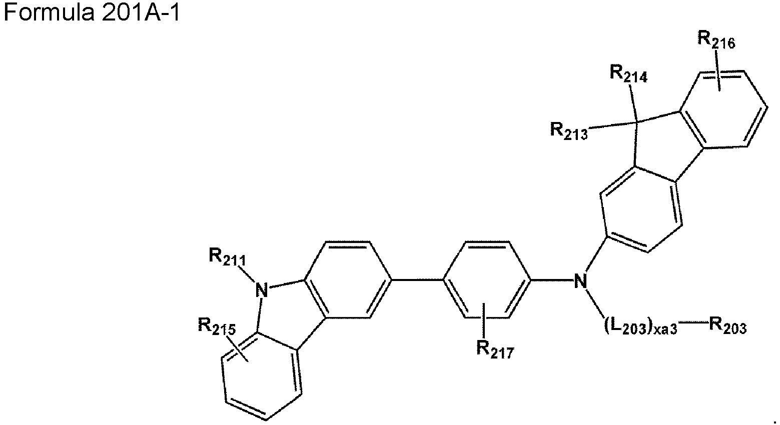

In one embodiment, the compound represented by Formula 202 may be represented by Formula 202A:

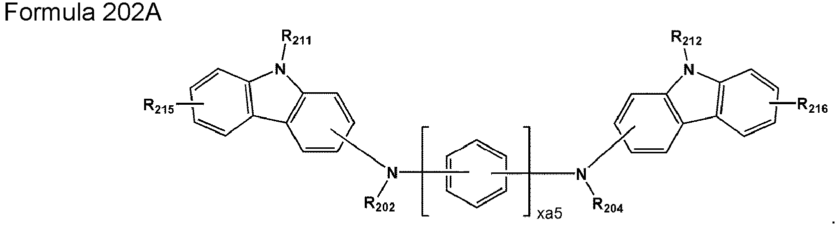

In one embodiment, the compound represented by Formula 202 may be represented by Formula 202A-1:

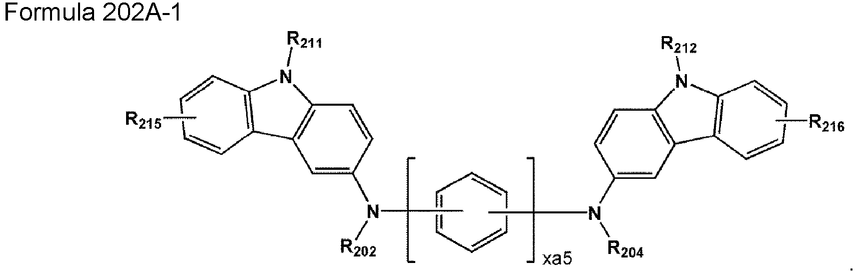

In Formulae 201A, 201A(1), 201A-1, 202A, and 202A-1,
L₂₀₁ to L₂₀₃, xa1 to xa3, xa5, and R₂₀₂ to R₂₀₄ may each independently be the same as defined above,
R₂₁₁ and R₂₁₂ may each independently be defined the same as R₂₀₃, and
R₂₁₃ to R₂₁₇ may each independently be selected from hydrogen, deuterium, -F, -Cl, -Br, -I, a hydroxyl group, a cyano group, a nitro group, an amidino group, a hydrazine group (a hydrazino group), a hydrazone group (a hydrazono group), a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group.

The hole transport region may include at least one compound selected from Compounds HT1 to HT39, but embodiments of the present disclosure are not limited thereto:

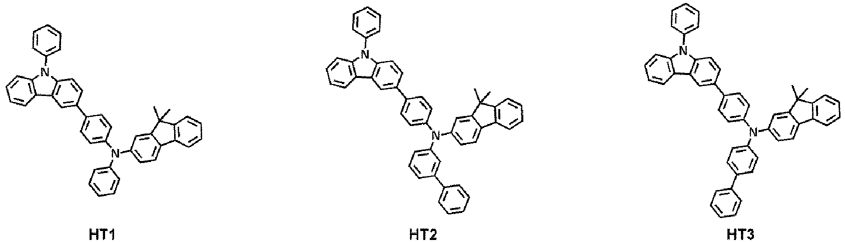

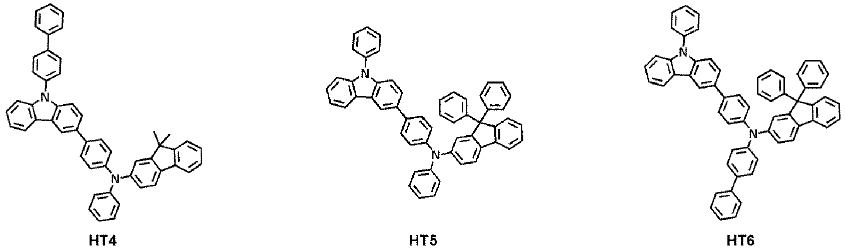

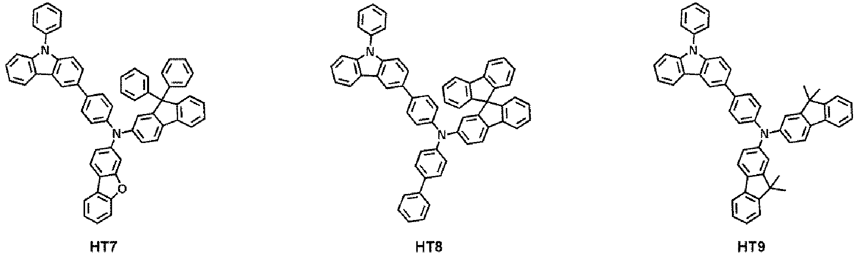

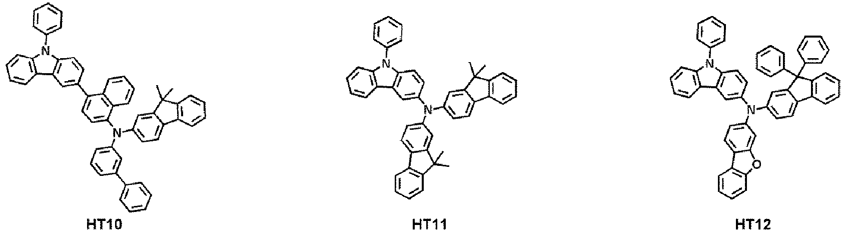

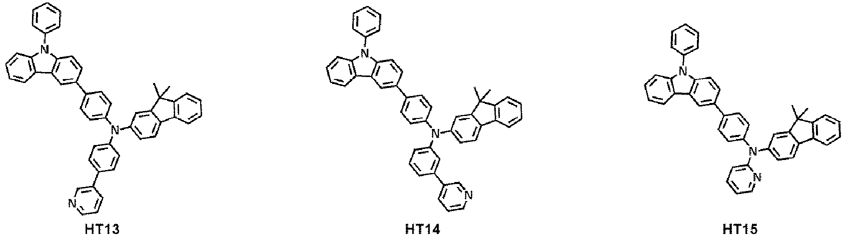

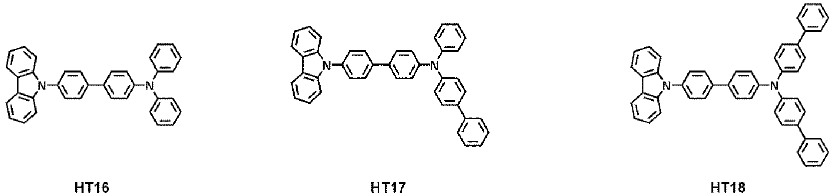

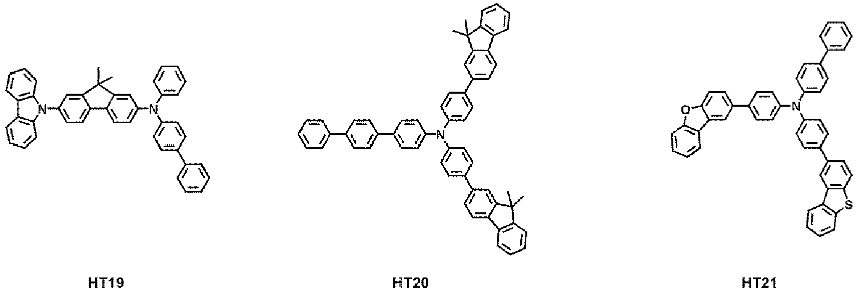

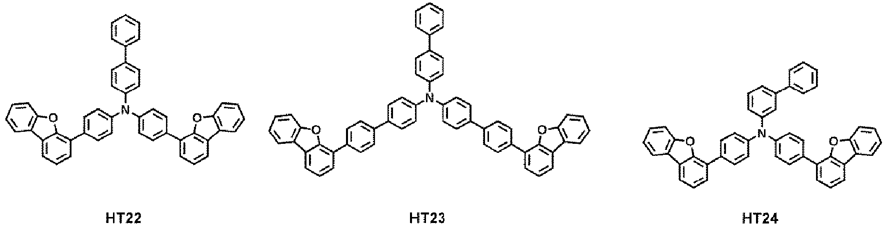

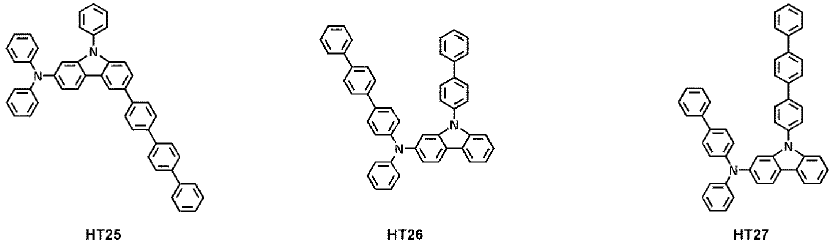

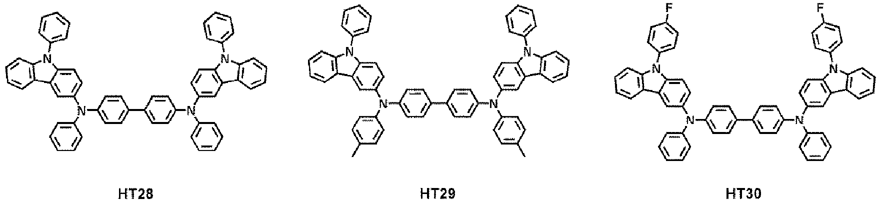

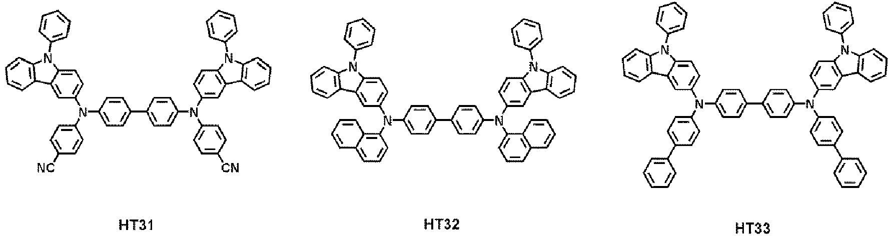

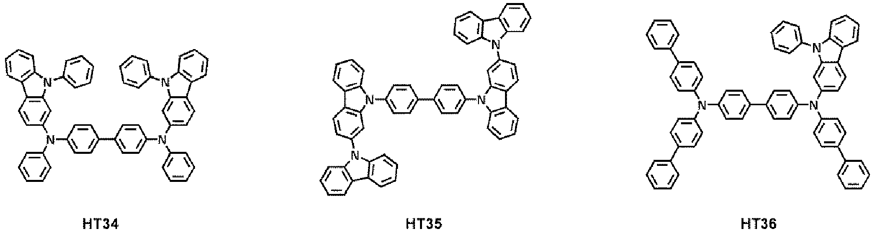

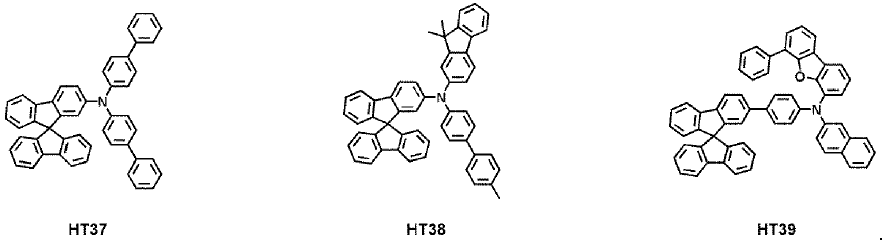

A thickness of the hole transport region may be in a range of about 100 Å to about 10,000 Å, for example, about 100 Å to about 7000 Å, about 100 Å to about 5000 Å, about 100 Å to about 3000 Å, or about 100 Å to about 1,000 Å. When the hole transport region includes at least one of a hole injection layer and a hole transport layer, the thickness of the hole injection layer may be in a range of about 100 Å to about 9,000 Å, and for example, about 100 Å to about 7,000 Å, about 100 Å to about 5,000 Å, about 100 Å to about 3,000 Å, about 100 Å to about 2,000 Å, about 100 Å to about 1,000 Å or about 500 Å to about 700 Å, and the thickness of the hole transport layer may be in a range of about 50 Å to about 2,000 Å, and for example, about 100 Å to about 1,500 Å, about 100 Å to about 1,000 Å, about 100 Å to about 800 Å, about 100 Å to about 500 Å, or about 200 Å to about 400 Å. When the thicknesses of the hole transport region, the hole injection layer, and the hole transport layer are within these ranges, suitable or satisfactory hole transporting characteristics may be obtained without a substantial increase in driving voltage.

The emission auxiliary layer may increase light-emission efficiency by compensating for an optical resonance distance according to the wavelength of light emitted by an emission layer, and the electron blocking layer may block the flow of electrons from an electron transport region. The emission auxiliary layer and the electron blocking layer may include the materials as described above.

### p-dopant

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant.

In one embodiment, the lowest unoccupied molecular orbital (LUMO) energy level of the p-dopant is lower than -3.5eV.

The p-dopant may include at least one selected from a quinone derivative, a metal oxide, and a cyano group-containing compound, but embodiments of the present disclosure are not limited thereto.

For example, the p-dopant may include at least one selected from:
a quinone derivative, such as tetracyanoquinodimethane (TCNQ) and 2,3,5,6-tetrafluoro-7,7,8,8-tetracyanoquinodimethane (F4-TCNQ);
a metal oxide, such as tungsten oxide or molybdenum oxide;
1,4,5,8,9,11-hexaazatriphenylene-hexacarbonitrile (HAT-CN); and
a compound represented by Formula 221,
but embodiments of the present disclosure are not limited thereto:

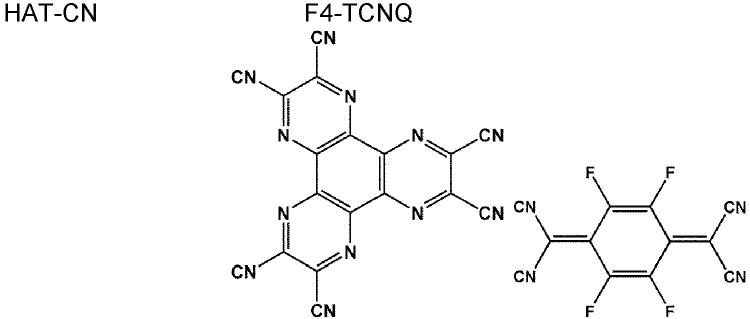

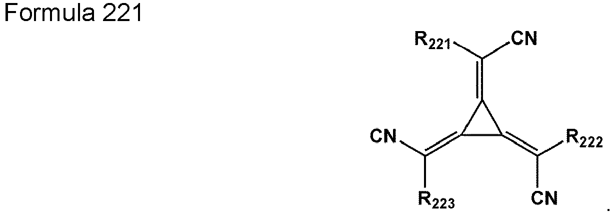

In Formula 221,
R₂₂₁ to R₂₂₃ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ (e.g. C₆-C₃₂) aryl group, a substituted or unsubstituted C₁-C₆₀ (e.g. C₁-C₂₀) heteroaryl group, a substituted or unsubstituted monovalent non-aromatic condensed polycyclic group, and a substituted or unsubstituted monovalent non-aromatic condensed heteropolycyclic group, wherein at least one selected from R₂₂₁ to R₂₂₃ may have at least one substituent selected from a cyano group, -F, -Cl, -Br, -I, a C₁-C₂₀ alkyl group substituted with -F, a C₁-C₂₀ alkyl group substituted with -Cl, a C₁-C₂₀ alkyl group substituted with -Br, and a C₁-C₂₀ alkyl group substituted with -I.

### Emission layer in organic layer 150

When the organic light-emitting device 10 is a full-color organic light-emitting device, the emission layer may be patterned into a red emission layer, a green emission layer, or a blue emission layer, according to a sub-pixel. In one or more embodiments, the emission layer may have a stacked structure of two or more layers selected from a red emission layer, a green emission layer, and a blue emission layer, in which the two or more layers contact each other or are separated from each other. In one or more embodiments, the emission layer may include two or more materials selected from a red light-emitting material, a green light-emitting material, and a blue light-emitting material, in which the two or more materials are mixed with each other in a single layer to emit white light.

The emission layer may include a host and a dopant. The dopant may include at least one selected from a phosphorescent dopant and a fluorescent dopant.

In the emission layer, an amount of the dopant may be in a range of about 0.01 parts by weight to about 15 parts by weight based on 100 parts by weight of the host, but embodiments of the present disclosure are not limited thereto.

A thickness of the emission layer may be in a range of about 100 A to about 1,000 Å, for example, about 100 Å to about 800 Å, about 150 Å to about 800 Å, about 150 Å to about 700 Å, about 150 Å to about 650 Å, about 200 Å to about 600 Å or about 200 Å to about 400 Å. When the thickness of the emission layer is within this range, excellent light-emission characteristics may be obtained without a substantial increase in driving voltage.

### Host in emission layer

In one or more embodiments, the host may include a compound represented by Formula 301:

Formula 301 [Ar₃₀₁]_{xb11-}[(L₃₀₁)_{xb1}-R₃₀₁]_{xb21}.

In Formula 301,
Ar₃₀₁ may be a substituted or unsubstituted C₅-C₆₀ carbocyclic group or a substituted or unsubstituted C₁-C₆₀ heterocyclic group,
xb11 may be 1, 2, or 3,
L₃₀₁ may independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
xb1 may be an integer from 0 to 5,
R₃₀₁ may be selected from deuterium, -F, -Cl, -Br, -I, a hydroxyl group, a cyano group, a nitro group, an amidino group, a hydrazine group (a hydrazino group), a hydrazone group (a hydrazono group), a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -Si(Q₃₀₁)(Q₃₀₂)(Q₃₀₃), -N(Q₃₀₁)(Q₃₀₂), -B(Q₃₀₁)(Q₃₀₂),-C(=O)(Q₃₀₁), -S(=O)₂(Q₃₀₁), and -P(=O)(Q₃₀₁)(Q₃₀₂),
xb21 may be an integer from 1 to 5, and
Q₃₀₁ to Q₃₀₃ may each independently be selected from a C₁-C₁₀ alkyl group, a C₁-C₁₀ alkoxy group, a phenyl group, a biphenyl group, a terphenyl group, and a naphthyl group, but embodiments of the present disclosure are not limited thereto.

For example, Ar₃₀₁ may be a substituted or unsubstituted C₆-C₃₂ carbocyclic group or a substituted or unsubstituted C₁-C₁₂ heterocyclic group, but embodiments are not limited thereto.

In one embodiment, in Formula 301, Ar₃₀₁ may be selected from:
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group; and
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group, each substituted with at least one selected from deuterium, - F, -Cl, -Br, -I, a hydroxyl group, a cyano group, a nitro group, an amidino group, a hydrazine group (a hydrazino group), a hydrazone group (a hydrazono group), a C₁-C₂₀ alkyl group, a C₁-C₂₀ alkoxy group, a phenyl group, a biphenyl group, a terphenyl group, a naphthyl group, -Si(Q₃₁)(Q₃₂)(Q₃₃), -N(Q₃₁)(Q₃₂), -B(Q₃₁)(Q₃₂), -C(=O)(Q₃₁), - S(=O)₂(Q₃₁), and -P(=O)(Q₃₁)(Q₃₂), and
Q₃₁ to Q₃₃ may each independently be selected from a C₁-C₁₀ alkyl group, a C₁-C₁₀ alkoxy group, a phenyl group, a biphenyl group, a terphenyl group, and a naphthyl group. However, embodiments of the present disclosure are not limited thereto.

When xb11 in Formula 301 is two or more, two or more Ar₃₀₁ (s) may be linked via a single bond.

In one or more embodiments, the compound represented by Formula 301 may be represented by Formula 301-1 or 301-2:

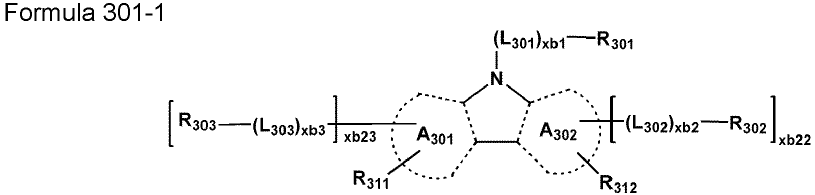

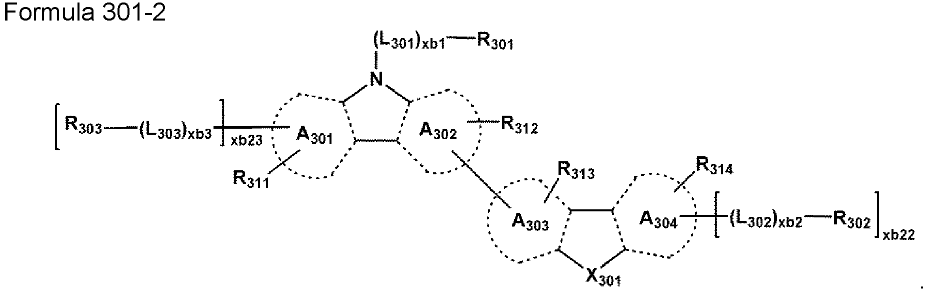

In Formulae 301-1 and 301-2,
A₃₀₁ to A₃₀₄ may each independently be selected from a benzene group, a naphthalene group, a phenanthrene group, a fluoranthene group, a triphenylene group, a pyrene group, a chrysene group, a pyridine group, a pyrimidine group, an indene group, a fluorene group, a spiro-bifluorene group, a benzofluorene group, a dibenzofluorene group, an indole group, a carbazole group, a benzocarbazole group, a dibenzocarbazole group, a furan group, a benzofuran group, a dibenzofuran group, a naphthofuran group, a benzonaphthofuran group, a dinaphthofuran group, a thiophene group, a benzothiophene group, a dibenzothiophene group, a naphthothiophene group, a benzonaphthothiophene group, and a dinaphthothiophene group,
X₃₀₁ may be O, S, or N-[(L₃₀₄)_{xb4}-R₃₀₄],
R₃₁₁ to R₃₁₄ may each independently be selected from hydrogen, deuterium, -F, -Cl, -Br, -I, a hydroxyl group, a cyano group, a nitro group, an amidino group, a hydrazine group (a hydrazino group), a hydrazone group (a hydrazono group), a C₁-C₂₀ alkyl group, a C₁-C₂₀ alkoxy group, a phenyl group, a biphenyl group, a terphenyl group, a naphthyl group -Si(Q₃₁)(Q₃₂)(Q₃₃), -N(Q₃₁)(Q₃₂), -B(Q₃₁)(Q₃₂), -C(=O)(Q₃₁),-S(=O)₂(Q₃₁), and -P(=O)(Q₃₁)(Q₃₂),
xb22 and xb23 may each independently be 0, 1, or 2,
L₃₀₁, xb1, R₃₀₁, and Q₃₁ to Q₃₃ may each independently be the same as defined above,
L₃₀₂ to L₃₀₄ may each independently be defined the same as L₃₀₁,
xb2 to xb4 may each independently be defined the same as xb1, and
R₃₀₂ to R₃₀₄ may each independently be defined the same as R₃₀₁.

For example, L₃₀₁ to L₃₀₄ in Formulae 301, 301-1, and 301-2 may each independently be selected from: a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₃₀ arylene group, a substituted or unsubstituted C₁-C₂₀ heteroarylene group, a substituted or unsubstituted divalent non-aromatic condensed polycyclic group, and a substituted or unsubstituted divalent non-aromatic condensed heteropolycyclic group, but embodiments are not limited thereto.

For example, in Formulae 301, 301-1, and 301-2, L₃₀₁ to L₃₀₄ may each independently be selected from:
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, a benzoisothiazolylene group, a benzoxazolylene group, a benzoisoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, a benzoisothiazolylene group, a benzoxazolylene group, a benzoisoxazolylene group, a triazolylene group, a tetrazolylene group, an imidazopyridinylene group, an imidazopyrimidinylene group, and an azacarbazolylene group, each substituted with at least one selected from deuterium, -F, -Cl, -Br, -I, a hydroxyl group, a cyano group, a nitro group, an amidino group, a hydrazine group (a hydrazino group), a hydrazone group (a hydrazono group), a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzoisothiazolyl group, a benzoxazolyl group, a benzoisoxazolyl group, a triazolyl group, a tetrazolyl group, an imidazopyridinyl group, an imidazopyrimidinyl group, an azacarbazolyl group, -Si(Q₃₁)(Q₃₂)(Q₃₃), -N(Q₃₁)(Q₃₂), -B(Q₃₁)(Q₃₂), -C(=O)(Q₃₁), - S(=O)₂(Q₃₁), and -P(=O)(Q₃₁)(Q₃₂), and
Q₃₁ to Q₃₃ may each independently be the same as defined above.

As another example, R₃₀₁ to R₃₀₄ in Formulae 301, 301-1, and 301-2 may each independently be selected from: deuterium, -F, -Cl, -Br, -I, a hydroxyl group, a cyano group, a nitro group, an amidino group, a hydrazino group, a hydrazono group, a substituted or unsubstituted C₁-C₆₀ (*e.g.* C₁-C₁₀) alkyl group, a substituted or unsubstituted C₂-C₆₀ (*e.g.* C₂-C₁₀) alkenyl group, a substituted or unsubstituted C₂-C₆₀ (*e.g.* C₂-C₁₀) alkynyl group, a substituted or unsubstituted C₁-C₆₀ (*e.g.* C₁-C₁₀) alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₃₀ aryl group, a substituted or unsubstituted C₆-C₃₀ aryloxy group, a substituted or unsubstituted C₆-C₃₀ arylthio group, a substituted or unsubstituted C₁-C₂₀ heteroaryl group, a substituted or unsubstituted monovalent non-aromatic condensed polycyclic group, a substituted or unsubstituted monovalent non-aromatic condensed heteropolycyclic group, -Si(Q₃₀₁)(Q₃₀₂)(Q₃₀₃), -N(Q₃₀₁)(Q₃₀₂),-B(Q₃₀₁)(Q₃₀₂), -C(=O)(Q₃₀₁), -S(=O)₂(Q₃₀₁), and -P(=O)(Q₃₀₁)(Q₃₀₂), but embodiments are not limited thereto.

In one embodiment, in Formulae 301, 301-1, and 301-2, R₃₀₁ to R₃₀₄ may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzoisothiazolyl group, a benzoxazolyl group, a benzoisoxazolyl group, a triazolyl group, a tetrazolyl group, an imidazopyridinyl group, an imidazopyrimidinyl group, and an azacarbazolyl group; and
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzoisothiazolyl group, a benzoxazolyl group, a benzoisoxazolyl group, a triazolyl group, a tetrazolyl group, an imidazopyridinyl group, an imidazopyrimidinyl group, and an azacarbazolyl group, each substituted with at least one selected from deuterium, -F, -Cl, -Br, -I, a hydroxyl group, a cyano group, a nitro group, an amidino group, a hydrazine group (a hydrazino group), a hydrazone group (a hydrazono group), a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzoisothiazolyl group, a benzoxazolyl group, a benzoisoxazolyl group, a triazolyl group, a tetrazolyl group, an imidazopyridinyl group, an imidazopyrimidinyl group, an azacarbazolyl group, -Si(Q₃₁)(Q₃₂)(Q₃₃), -N(Q₃₁)(Q₃₂),-B(Q₃₁)(Q₃₂), -C(=O)(Q₃₁), -S(=O)₂(Q₃₁), and -P(=O)(Q₃₁)(Q₃₂), and
Q₃₁ to Q₃₃ may each independently be the same as defined above.

In one or more embodiments, the host may include an alkaline earth metal complex. For example, the host may include a complex selected from a Be complex (for example, Compound H55), a Mg complex, and a Zn complex. For example, the host may be selected from a Be complex (for example, Compound H55), a Mg complex, and a Zn complex.

The host may include at least one selected from 9,10-di(2-naphthyl)anthracene (ADN), 2-methyl-9,10-bis(naphthalen-2-yl)anthracene (MADN), 9,10-di-(2-naphthyl)-2-t-butyl-anthracene (TBADN), a 4,4'-bis(N-carbazolyl)-1,1'-biphenyl (CBP), 1,3-di-9-carbazolylbenzene (mCP), 1,3,5-tri(carbazol-9-yl)benzene (TCP), and Compounds H1 to H55, but embodiments of the present disclosure are not limited thereto:

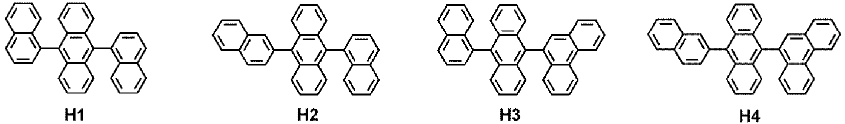

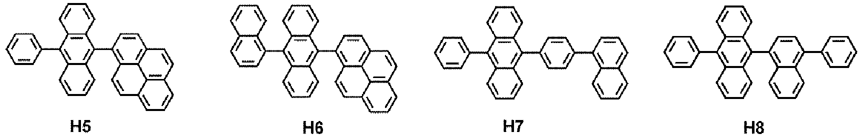

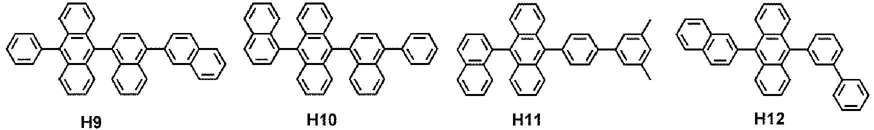

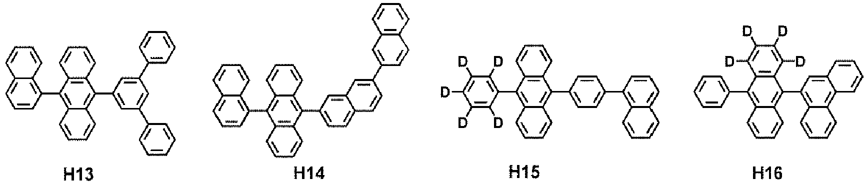

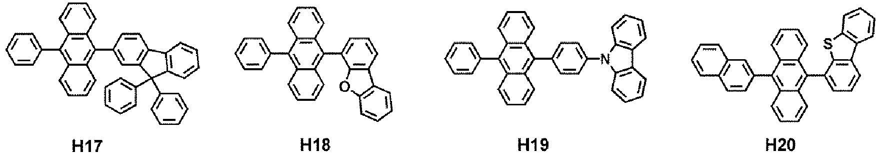

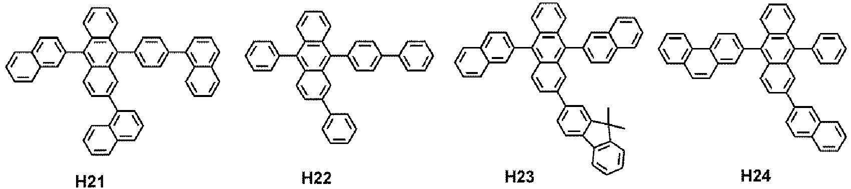

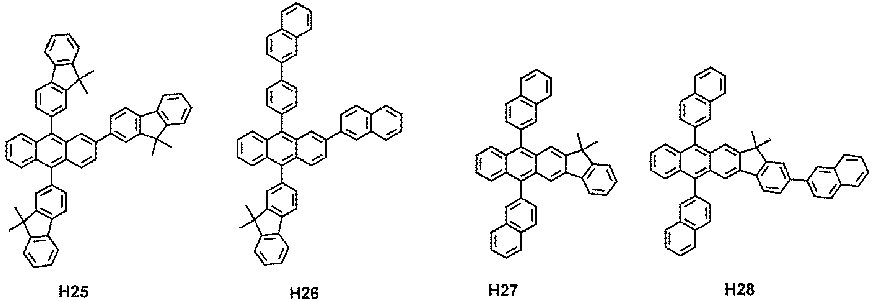

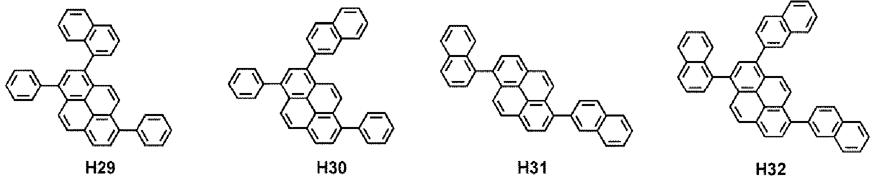

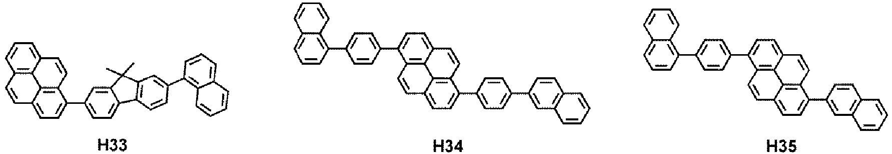

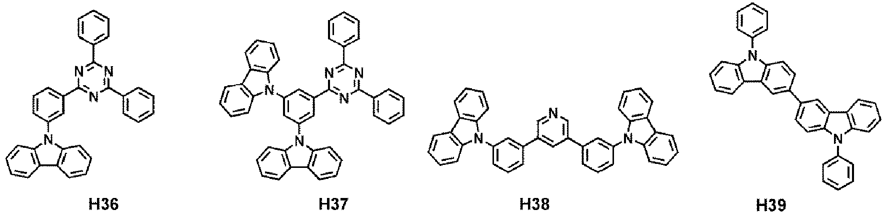

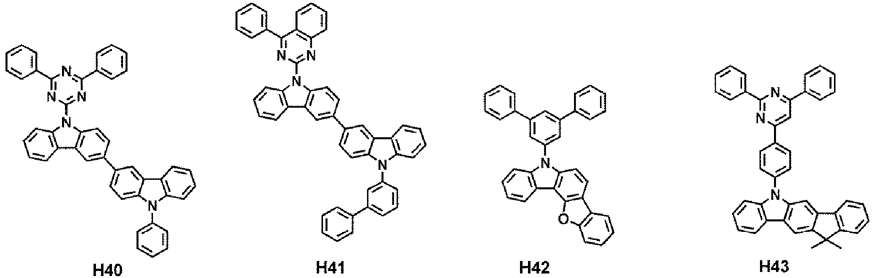

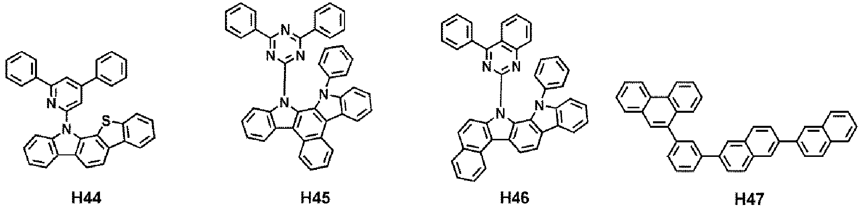

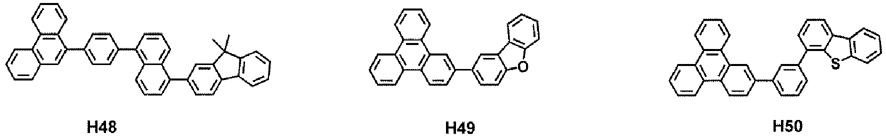

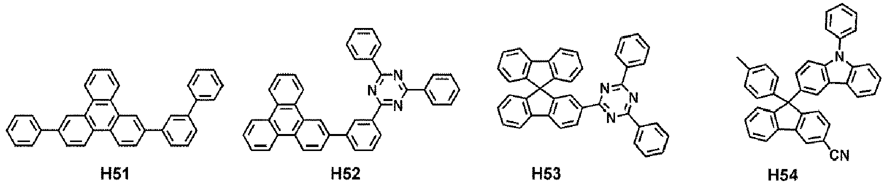

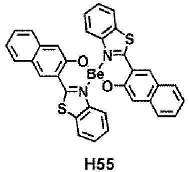

In one embodiment, the host may include at least one selected from a silicon-containing compound (for example, BCPDS used in the following examples or the like) and a phosphine oxide-containing compound (for example, POPCPA used in the following examples or the like).

However, embodiments of the present disclosure are not limited thereto. In one embodiment, the host may include only one compound, or two or more different compounds (for example, a host used in the following examples includes BCPDS and POPCPA).

### Dopant included in emission layer in organic layer 150

The dopant may include an organometallic compound represented by Formula 1. The organometallic compound is the same as described above.

### Electron transport region in organic layer 150

The electron transport region may have i) a single-layered structure including a single layer including a single material, ii) a single-layered structure including a single layer including a plurality of different materials, or iii) a multi-layered structure having a plurality of layers including a plurality of different materials.

The electron transport region may include at least one selected from a buffer layer, a hole blocking layer, an electron control layer, an electron transport layer, and an electron injection layer, but embodiments of the present disclosure are not limited thereto.

For example, the electron transport region may have an electron transport layer/electron injection layer structure, a hole blocking layer/electron transport layer/electron injection layer structure, an electron control layer/electron transport layer/electron injection layer structure, or a buffer layer/electron transport layer/electron injection layer structure, wherein for each structure, constituting layers are sequentially stacked from an emission layer. However, embodiments of the structure of the electron transport region are not limited thereto.

The electron transport region (for example, a buffer layer, a hole blocking layer, an electron control layer, or an electron transport layer in the electron transport region) may include a metal-free compound containing at least one π electron-depleted nitrogen-containing ring.

The term "π electron-depleted nitrogen-containing ring," as used herein, refers to a C₁-C₆₀ (e.g. a C₁-C₃₀) heterocyclic group having at least one *-N=*' moiety as a ring-forming moiety.

For example, the "π electron-depleted nitrogen-containing ring" may be i) a 5-membered to 7-membered heteromonocyclic group having at least one *-N=*' moiety, ii) a heteropolycyclic group in which two or more 5-membered to 7-membered heteromonocyclic groups each having at least one *-N=*' moiety are condensed with each other, or iii) a heteropolycyclic group in which at least one of 5-membered to 7-membered heteromonocyclic groups, each having at least one *-N=*' moiety, is condensed with at least one C₅-C₆₀ (e.g. a C₅-C₃₀) carbocyclic group.

Examples of the π electron-depleted nitrogen-containing ring include an imidazole, a pyrazole, a thiazole, an isothiazole, an oxazole, an isoxazole, a pyridine, a pyrazine, a pyrimidine, a pyridazine, an indazole, a purine, a quinoline, an isoquinoline, a benzoquinoline, a phthalazine, a naphthyridine, a quinoxaline, a quinazoline, a cinnoline, a phenanthridine, an acridine, a phenanthroline, a phenazine, a benzimidazole, a benzoisothiazole, a benzoxazole, a benzoisoxazole, a triazole, a tetrazole, an oxadiazole, a triazine, a thiadiazole, an imidazopyridine, an imidazopyrimidine, and an azacarbazole, but are not limited thereto.

For example, the electron transport region may include a compound represented by Formula 601:

Formula 601 [Ar₆₀₁]ₓₑ₁₁-[(L₆₀₁)ₓₑ₁-R₆₀₁]ₓₑ₂₁.

In Formula 601,
Ar₆₀₁ may be a substituted or unsubstituted C₅-C₆₀ carbocyclic group or a substituted or unsubstituted C₁-C₆₀ heterocyclic group,
xe11 may be 1, 2, or 3,
L₆₀₁ may be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group;
xe1 may be an integer from 0 to 5,
R₆₀₁ may be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, - Si(Q₆₀₁)(Q₆₀₂)(Q₆₀₃), -C(=O)(Q₆₀₁), -S(=O)₂(Q₆₀₁), and -P(=O)(Q₆₀₁)(Q₆₀₂),
Q₆₀₁ to Q₆₀₃ may each independently be a C₁-C₁₀ alkyl group, a C₁-C₁₀ alkoxy group, a phenyl group, a biphenyl group, a terphenyl group, or a naphthyl group, and
xe21 may be an integer from 1 to 5.

In one embodiment, at least one of Ar₆₀₁(s) in the number of xe11 and/or at least one of R₆₀₁(s) in the number of xe21 may include the π electron-depleted nitrogen-containing ring.

For example, Ar₆₀₁ may be a substituted or unsubstituted C₅-C₃₀ carbocyclic group or a substituted or unsubstituted C₁-C₂₀ heterocyclic group, but embodiments are not limited thereto.

In one embodiment, Ar₆₀₁ in Formula 601 may be selected from:
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, a benzoisothiazole group, a benzoxazole group, a benzoisoxazole group, a triazole group, a tetrazole group, an oxadiazole group, a triazine group, a thiadiazole group, an imidazopyridine group, an imidazopyrimidine group, and an azacarbazole group; and
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, a benzoisothiazole group, a benzoxazole group, a benzoisoxazole group, a triazole group, a tetrazole group, an oxadiazole group, a triazine group, a thiadiazole group, an imidazopyridine group, an imidazopyrimidine group, and an azacarbazole group, each substituted with at least one selected from deuterium, -F, -Cl, -Br, -I, a hydroxyl group, a cyano group, a nitro group, an amidino group, a hydrazine group (a hydrazino group), a hydrazone group (a hydrazono group), a C₁-C₂₀ alkyl group, a C₁-C₂₀ alkoxy group, a phenyl group, a biphenyl group, a terphenyl group, a naphthyl group, -Si(Q₃₁)(Q₃₂)(Q₃₃), -S(=O)₂(Q₃₁), and -P(=O)(Q₃₁)(Q₃₂), and
Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

When xe11 in Formula 601 is two or more, two or more of Ar₆₀₁(s) may be linked to each other via a single bond.

In one or more embodiments, Ar₆₀₁ in Formula 601 may be an anthracene group.

In one or more embodiments, a compound represented by Formula 601 may be represented by Formula 601-1:

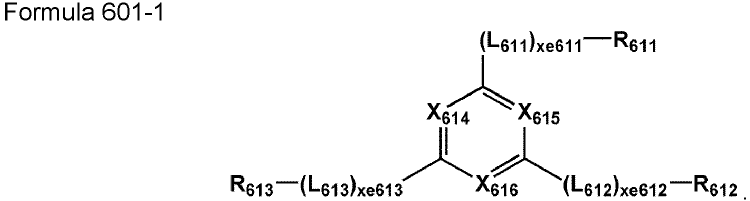

In Formula 601-1,
X₆₁₄ may be N or C(R₆₁₄), X₆₁₅ may be N or C(R₆₁₅), X₆₁₆ may be N or C(R₆₁₆), and at least one selected from X₆₁₄ to X₆₁₆ may be N,
L₆₁₁ to L₆₁₃ may each independently be defined the same as L₆₀₁,
xe611 to xe613 may each independently be defined the same as xe1,
R₆₁₁ to R₆₁₃ may each independently be defined the same as R₆₀₁, and
R₆₁₄ to R₆₁₆ may each independently be selected from hydrogen, deuterium, -F, -Cl, -Br, -I, a hydroxyl group, a cyano group, a nitro group, an amidino group, a hydrazine group (a hydrazino group), a hydrazone group (a hydrazono group), a C₁-C₂₀ alkyl group, a C₁-C₂₀ alkoxy group, a phenyl group, a biphenyl group, a terphenyl group, and a naphthyl group.

For example, L₆₀₁ and L₆₁₁ to L₆₁₃ in Formulae 601 and 601-1 may each independently be selected from: a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₃₀ arylene group, a substituted or unsubstituted C₁-C₂₀ heteroarylene group, a substituted or unsubstituted divalent non-aromatic condensed polycyclic group, and a substituted or unsubstituted divalent non-aromatic condensed heteropolycyclic group, but embodiments are not limited thereto.

In one embodiment, L₆₀₁ and L₆₁₁ to L₆₁₃ in Formulae 601 and 601-1 may each independently be selected from:
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, a benzoisothiazolylene group, a benzoxazolylene group, a benzoisoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, a benzoisothiazolylene group, a benzoxazolylene group, a benzoisoxazolylene group, a triazolylene group, a tetrazolylene group, an imidazopyridinylene group, an imidazopyrimidinylene group, and an azacarbazolylene group, each substituted with at least one selected from deuterium, -F, -Cl, -Br, -I, a hydroxyl group, a cyano group, a nitro group, an amidino group, a hydrazine group (a hydrazino group), a hydrazone group (a hydrazono group), a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzoisothiazolyl group, a benzoxazolyl group, a benzoisoxazolyl group, a triazolyl group, a tetrazolyl group, an imidazopyridinyl group, an imidazopyrimidinyl group, and an azacarbazolyl group,
but embodiments of the present disclosure are not limited thereto.

In one or more embodiments, xe1 and xe611 to xe613 in Formulae 601 and 601-1 may each independently be 0, 1, or 2.

For example, R₆₀₁ and R₆₁₁ to R₆₁₃ in Formulae 601 and 601-1 may each independently be selected from: a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₃₀ aryl group, a substituted or unsubstituted C₆-C₃₀ aryloxy group, a substituted or unsubstituted C₆-C₃₀ arylthio group, a substituted or unsubstituted C₁-C₂₀ heteroaryl group, a substituted or unsubstituted monovalent non-aromatic condensed polycyclic group, a substituted or unsubstituted monovalent non-aromatic condensed heteropolycyclic group, - Si(Q₆₀₁)(Q₆₀₂)(Q₆₀₃), -C(=O)(Q₆₀₁), -S(=O)₂(Q₆₀₁), and -P(=O)(Q₆₀₁)(Q₆₀₂).

In one or more embodiments, R₆₀₁ and R₆₁₁ to R₆₁₃ in Formula 601 and 601-1 may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzoisothiazolyl group, a benzoxazolyl group, a benzoisoxazolyl group, a triazolyl group, a tetrazolyl group, an imidazopyridinyl group, an imidazopyrimidinyl group, and an azacarbazolyl group;
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzoisothiazolyl group, a benzoxazolyl group, a benzoisoxazolyl group, a triazolyl group, a tetrazolyl group, an imidazopyridinyl group, an imidazopyrimidinyl group, and an azacarbazolyl group, each substituted with at least one selected from deuterium, -F, -Cl, -Br, -I, a hydroxyl group, a cyano group, a nitro group, an amidino group, a hydrazine group (a hydrazino group), a hydrazone group (a hydrazono group), a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzoisothiazolyl group, a benzoxazolyl group, a benzoisoxazolyl group, a triazolyl group, a tetrazolyl group, an imidazopyridinyl group, an imidazopyrimidinyl group, and an azacarbazolyl group; and
-S(=O)₂(Q₆₀₁) and -P(=O)(Q₆₀₁)(Q₆₀₂), and

Q₆₀₁ and Q₆₀₂ may each independently be defined the same as described above.

The electron transport region may include at least one compound selected from Compounds ET1 to ET36, but embodiments of the present disclosure are not limited thereto:

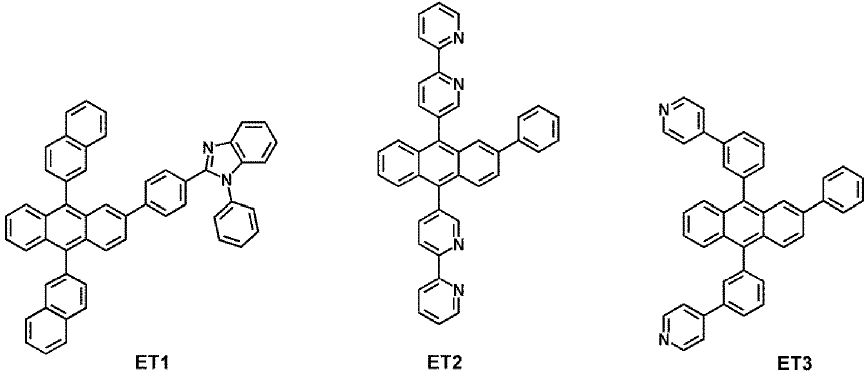

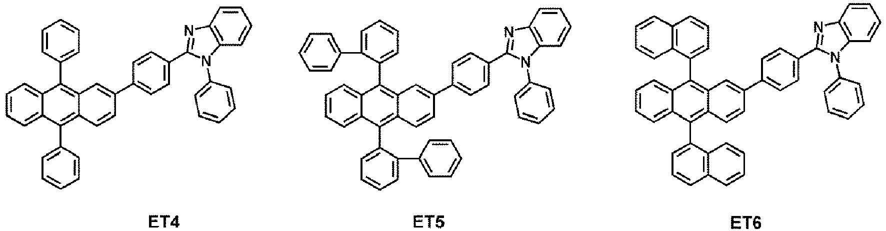

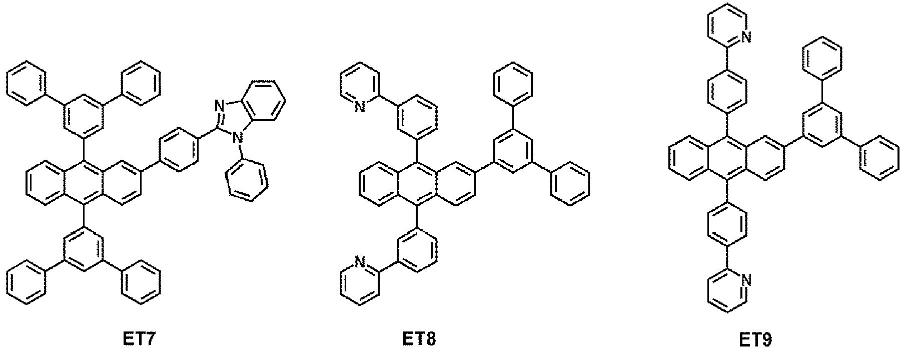

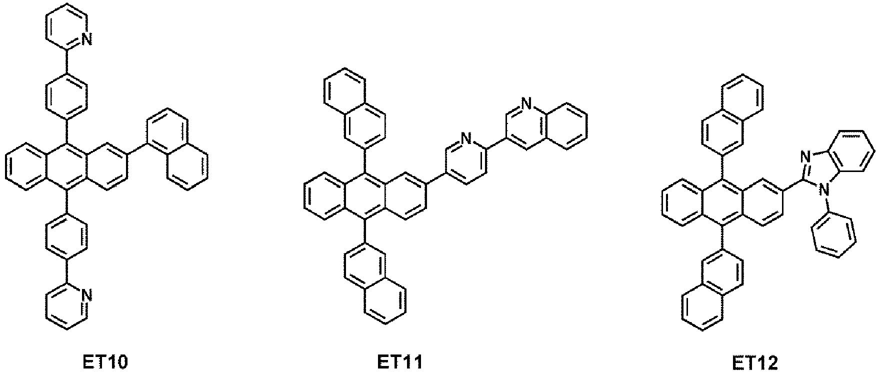

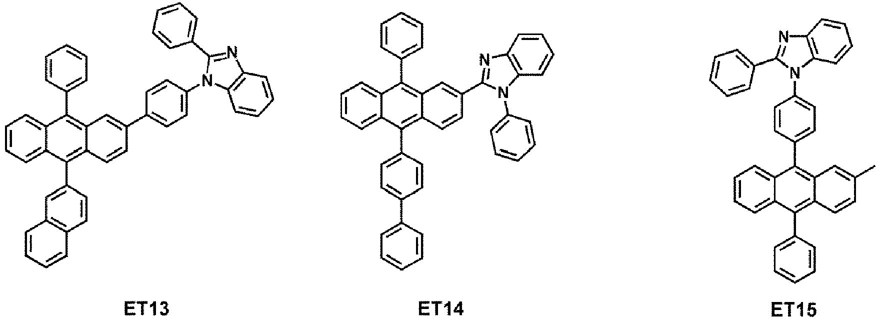

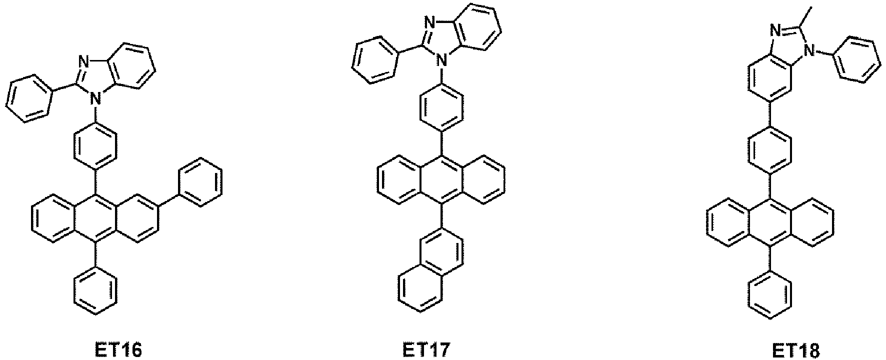

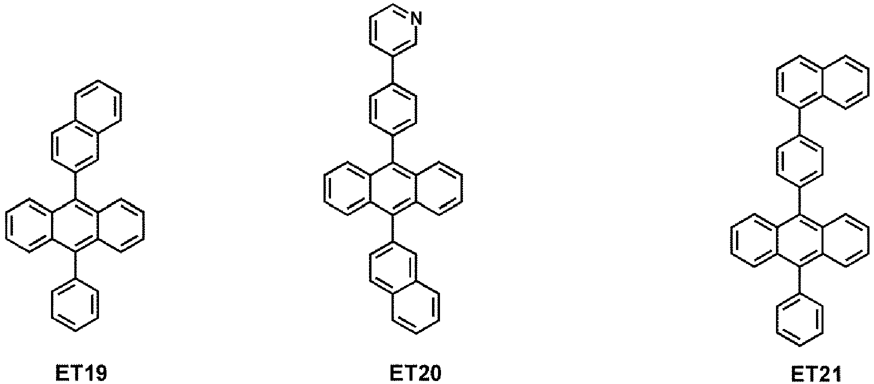

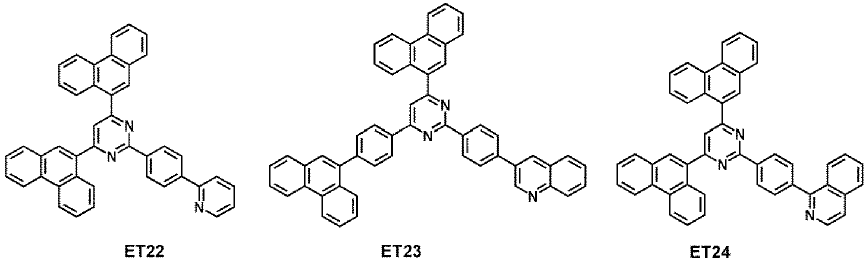

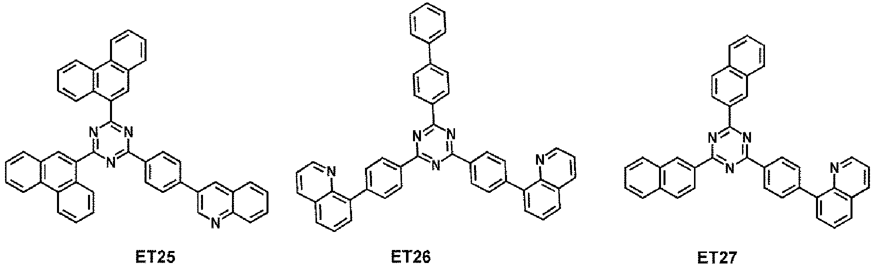

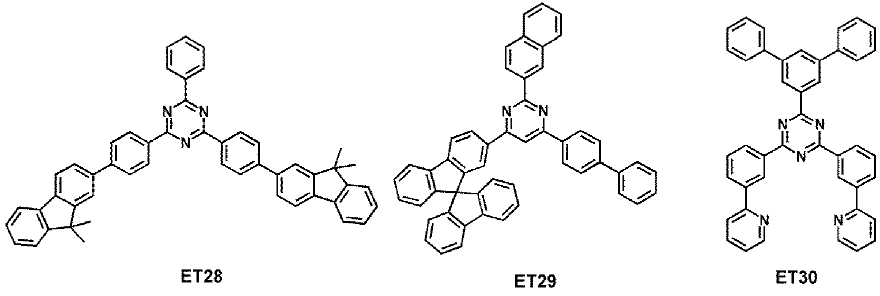

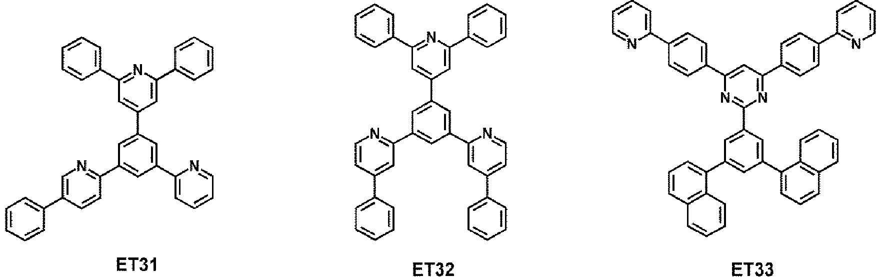

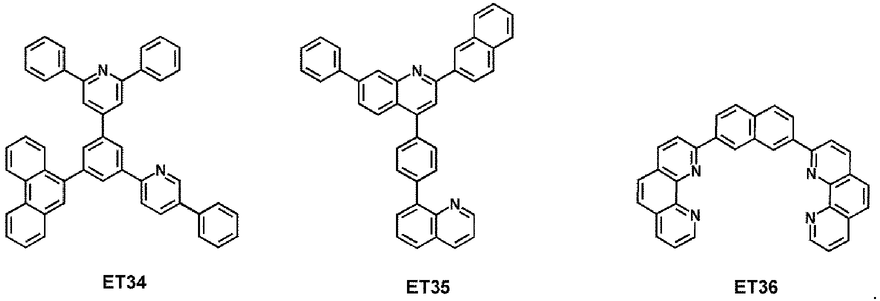

In one or more embodiments, the electron transport region may include at least one selected from 2,9-dimethyl-4,7-diphenyl-1,10-phenanthroline (BCP), 4,7-diphenyl-1,10-phenanthroline (Bphen), Alq₃, BAlq, 3-(biphenyl-4-yl)-5-(4-tert-butylphenyl)-4-phenyl-4H-1,2,4-triazole (TAZ), and NTAZ:

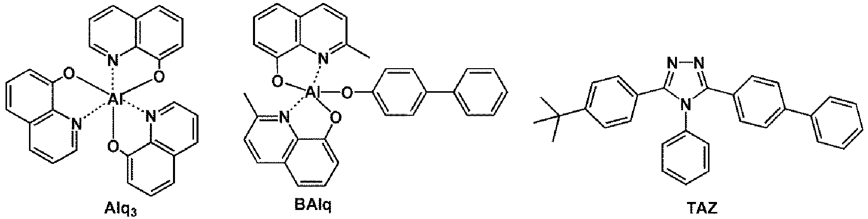

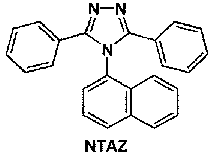

In one embodiment, the electron transport region may include a phosphine oxide-containing compound (for example, TSPO1 used in the following examples or the like), but embodiments of the present disclosure are not limited thereto. In one embodiment, the phosphine oxide-containing compound may be used in a hole blocking layer in the electron transport region, but embodiments of the present disclosure are not limited thereto.

The thickness of the buffer layer, the hole blocking layer, or the electron controlling layer may each independently be in a range of about 20 Å to about 1,000 Å, for example, about 20 Å to about 700 Å, about 20 Å to about 500 Å, about 20 Å to about 400 Å, about 30 Å to about 400 Å or about 30 Å to about 300 Å. When the thicknesses of the buffer layer, the hole blocking layer, and the electron control layer are within these ranges, the electron blocking layer may have excellent electron blocking characteristics or electron control characteristics without a substantial increase in driving voltage.

A thickness of the electron transport layer may be in a range of about 100 Å to about 1,000 Å, for example, about 100 Å to about 700 Å, about 100 Å to about 600 Å, about 150 Å to about 600 Å, about 150 Å to about 500 Å or about 250 Å to about 350 Å. When the thickness of the electron transport layer is within the range described above, the electron transport layer may have suitable or satisfactory electron transport characteristics without a substantial increase in driving voltage.

The electron transport region (for example, the electron transport layer in the electron transport region) may further include, in addition to the materials described above, a metal-containing material.

The metal-containing material may include at least one selected from alkali metal complex and alkaline earth-metal complex. The alkali metal complex may include a metal ion selected from a Li ion, a Na ion, a K ion, a Rb ion, and a Cs ion, and the alkaline earth-metal complex may include a metal ion selected from a Be ion, a Mg ion, a Ca ion, a Sr ion, and a Ba ion. A ligand coordinated with the metal ion of the alkali metal complex or the alkaline earth-metal complex may be selected from a hydroxy quinoline, a hydroxy isoquinoline, a hydroxy benzoquinoline, a hydroxy acridine, a hydroxy phenanthridine, a hydroxy phenyloxazole, a hydroxy phenylthiazole, a hydroxy phenyloxadiazole, a hydroxy phenylthiadiazole, a hydroxy phenylpyridine, a hydroxy phenylbenzimidazole, a hydroxy phenylbenzothiazole, a bipyridine, a phenanthroline, and a cyclopentadiene, but embodiments of the present disclosure are not limited thereto.

For example, the metal-containing material may include a Li complex. The Li complex may include, for example, Compound ET-D1 (8-hydroxyquinolinolato-lithium, LiQ) or ET-D2:

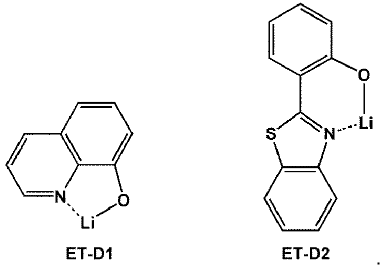

The electron transport region may include an electron injection layer that facilitates injection of electrons from the second electrode 190. The electron injection layer may directly contact the second electrode 190.

The electron injection layer may have i) a single-layered structure including a single layer including a single material, ii) a single-layered structure including a single layer including a plurality of different materials, or iii) a multi-layered structure having a plurality of layers including a plurality of different materials.

The electron injection layer may include an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof.

The alkali metal may be selected from Li, Na, K, Rb, and Cs. In one embodiment, the alkali metal may be Li, Na, or Cs. In one or more embodiments, the alkali metal may be Li or Cs, but embodiments of the present disclosure are not limited thereto.

The alkaline earth metal may be selected from Mg, Ca, Sr, and Ba.

The rare earth metal may be selected from Sc, Y, Ce, Tb, Yb, and Gd.

The alkali metal compound, the alkaline earth-metal compound, and the rare earth metal compound may be selected from oxides and halides (for example, fluorides, chlorides, bromides, or iodides) of the alkali metal, the alkaline earth-metal, and the rare earth metal.

The alkali metal compound may be selected from alkali metal oxides, such as Li₂O, Cs₂O, or K₂O, and alkali metal halides, such as LiF, NaF, CsF, KF, Lil, Nal, CsI, or KI. In one embodiment, the alkali metal compound may be selected from LiF, Li₂O, NaF, Lil, Nal, CsI, and KI, but embodiments of the present disclosure are not limited thereto.

The alkaline earth-metal compound may be selected from alkaline earth-metal oxides, such as BaO, SrO, CaO, BaₓSr₁₋ₓO (0<x<1), or BaₓCa₁₋ₓO (0<x<1). In one embodiment, the alkaline earth-metal compound may be selected from BaO, SrO, and CaO, but embodiments of the present disclosure are not limited thereto.

The rare earth metal compound may be selected from YbF₃, ScF₃, Sc₂O₃, Y₂O₃, Ce₂O₃, GdF₃, and TbF₃. In one embodiment, the rare earth metal compound may be selected from YbF₃, ScF₃, TbF₃, YbI₃, ScI₃, and TbI₃, but embodiments of the present disclosure are not limited thereto.

The alkali metal complex, the alkaline earth-metal complex, and the rare earth metal complex may include an ion of alkali metal, alkaline earth-metal, and rare earth metal as described above, and a ligand coordinated with a metal ion of the alkali metal complex, the alkaline earth-metal complex, or the rare earth metal complex may be selected from hydroxy quinoline, hydroxy isoquinoline, hydroxy benzoquinoline, hydroxy acridine, hydroxy phenanthridine, hydroxy phenyloxazole, hydroxy phenylthiazole, hydroxy phenyloxadiazole, hydroxy phenylthiadiazole, hydroxy phenylpyridine, hydroxy phenylbenzimidazole, hydroxy phenylbenzothiazole, bipyridine, phenanthroline, and cyclopentadiene, but embodiments of the present disclosure are not limited thereto.

The electron injection layer may include (e.g., consist of) an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof, as described above. In one or more embodiments, the electron injection layer may further include an organic material. When the electron injection layer further includes an organic material, an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof may be homogeneously or non-homogeneously dispersed in a matrix including the organic material.

A thickness of the electron injection layer may be in a range of about 1 Å to about 100 Å, for example, about 3 Å to about 90 Å or about 3 Å to about 20 Å. When the thickness of the electron injection layer is within the range described above, the electron injection layer may have suitable or satisfactory electron injection characteristics without a substantial increase in driving voltage.

### Second electrode 190

The second electrode 190 may be disposed on the organic layer 150 having such a structure. The second electrode 190 may be a cathode that is an electron injection electrode, and in this regard, a material for forming the second electrode 190 may be a material having a low work function, and such a material may be metal, alloy, an electrically conductive compound, or a combination thereof.

The second electrode 190 may include at least one selected from lithium (Li), silver (Ag), magnesium (Mg), aluminium (Al), aluminium-lithium (Al-Li), calcium (Ca), magnesium-indium (Mg-In), magnesium-silver (Mg-Ag), ITO, and IZO, but embodiments of the present disclosure are not limited thereto. The second electrode 190 may be a transmissive electrode, a semi-transmissive electrode, or a reflective electrode.

The second electrode 190 may have a single-layered structure, or a multi-layered structure including two or more layers.

Layers constituting the hole transport region, an emission layer, and layers constituting the electron transport region may be formed in a certain region by using one or more suitable methods selected from vacuum deposition, spin coating, casting, Langmuir-Blodgett (LB) deposition, ink-jet printing, laser-printing, and laser-induced thermal imaging.

When layers constituting the hole transport region, an emission layer, and layers constituting the electron transport region are formed by vacuum deposition, the deposition may be performed at a deposition temperature of about 100 °C to about 500 °C, a vacuum degree of about 10⁻⁸ torr to about 10⁻³ torr (wherein 1 torr = 133.322 Pa), and a deposition speed of about 0.01 Å/sec to about 100 Å/sec by taking into account a material to be included in a layer to be formed, and the structure of a layer to be formed.

When layers constituting the hole transport region, an emission layer, and layers constituting the electron transport region are formed by spin coating, the spin coating may be performed at a coating speed of about 2,000 rpm to about 5,000 rpm and at a heat treatment temperature of about 80 °C to 200 °C, depending on a material to be included in a layer and the structure of each layer to be formed.

### Apparatus

The organic light-emitting device may be used in various suitable apparatuses.

Thus, another aspect of an embodiment provides an apparatus including the organic light-emitting device.

The apparatus may be, for example, a light-emitting apparatus, an authentication apparatus, or any other suitable electronic device, but embodiments of the present disclosure are not limited thereto.

The light-emitting apparatus may be used as various suitable displays, light sources, and the like.

The authentication apparatus may be, for example, a biometric authentication apparatus for authenticating an individual by using biometric information of a biometric body (for example, a finger tip, a pupil, or the like).

The authentication apparatus may further include, in addition to the organic light-emitting device, a biometric information collector.

The electronic apparatus may be applied to personal computers (for example, a mobile personal computer), mobile phones, digital cameras, electronic organizers, electronic dictionaries, electronic game machines, medical instruments (for example, electronic thermometers, sphygmomanometers, blood glucose meters, pulse measurement devices, pulse wave measurement devices, electrocardiogram (ECG) displays, ultrasonic diagnostic devices, or endoscope displays), fish finders, various suitable measuring instruments, meters (for example, meters for a vehicle, an aircraft, and a vessel), projectors, and the like, but embodiments of the present disclosure are not limited thereto.

The apparatus may further include, in addition to the organic light-emitting device, a thin film transistor. The thin film transistor may include a source electrode and a drain electrode, and the first electrode of the organic light-emitting device may be electrically coupled (e.g., electrically connected) with at least one selected from of the source electrode and the drain electrode of the thin film transistor.

### General definition of some of the substituents

The term "C₁-C₆₀ alkyl group," as used herein, refers to a linear or branched saturated aliphatic hydrocarbon monovalent group having 1 to 60 carbon atoms, and non-limiting examples thereof include a methyl group, an ethyl group, a propyl group, an isobutyl group, a sec-butyl group, a tert-butyl group, a pentyl group, an isoamyl group, and a hexyl group. The term "C₁-C₆₀ alkylene group," as used herein, refers to a divalent group having the same (e.g., substantially the same) structure as the C₁-C₆₀ alkyl group. Corresponding definitions apply to other ranges given for the number of carbon atoms in an alkyl/alkylene group.

The term "C₂-C₆₀ alkenyl group," as used herein, refers to a hydrocarbon group formed by substituting at least one carbon-carbon double bond at a main chain (e.g., in the middle) or at a terminus of the C₂-C₆₀ alkyl group, and non-limiting examples thereof include an ethenyl group, a propenyl group, and a butenyl group. The term "C₂-C₆₀ alkenylene group," as used herein, refers to a divalent group having the same (e.g., substantially the same) structure as the C₂-C₆₀ alkenyl group. Corresponding definitions apply to other ranges given for the number of carbon atoms in an alkenyl/alkenylene group.

The term "C₂-C₆₀ alkynyl group," as used herein, refers to a hydrocarbon group formed by substituting at least one carbon-carbon triple bond at a main chain (e.g., in the middle) or at a terminus of the C₂-C₆₀ alkyl group, and non-limiting examples thereof include an ethynyl group, and a propynyl group. The term "C₂-C₆₀ alkynylene group," as used herein, refers to a divalent group having the same (e.g., substantially the same) structure as the C₂-C₆₀ alkynyl group. Corresponding definitions apply to other ranges given for the number of carbon atoms in an alkynyl/alkynylene group.

The term "C₁-C₆₀ alkoxy group," as used herein, refers to a monovalent group represented by -OA₁₀₁ (wherein A₁₀₁ is the C₁-C₆₀ alkyl group), and non-limiting examples thereof include a methoxy group, an ethoxy group, and an isopropyloxy group. Corresponding definitions apply to other ranges given for the number of carbon atoms in an alkoxy group.

The term "C₃-C₁₀ cycloalkyl group," as used herein, refers to a monovalent saturated hydrocarbon monocyclic group having 3 to 10 carbon atoms, and non-limiting examples thereof include a cyclopropyl group, a cyclobutyl group, a cyclopentyl group, a cyclohexyl group, and a cycloheptyl group. The term "C₃-C₁₀ cycloalkylene group," as used herein, refers to a divalent group having the same (e.g., substantially the same) structure as the C₃-C₁₀ cycloalkyl group.

The term "C₁-C₁₀ heterocycloalkyl group," as used herein, refers to a monovalent saturated monocyclic group having at least one heteroatom selected from N, O, Si, P, and S as a ring-forming atom and 1 to 10 carbon atoms, and non-limiting examples thereof include a 1,2,3,4-oxatriazolidinyl group, a tetrahydrofuranyl group, a tetrahydrothiophenyl group. The term "C₁-C₁₀ heterocycloalkylene group," as used herein, refers to a divalent group having the same (e.g., substantially the same) structure as the C₁-C₁₀ heterocycloalkyl group.

The term "C₃-C₁₀ cycloalkenyl group," as used herein, refers to a monovalent monocyclic group that has 3 to 10 carbon atoms and at least one carbon-carbon double bond in the ring thereof and no aromaticity (e.g., the entire ring and/or group is not aromatic), and non-limiting examples thereof include a cyclopentenyl group, a cyclohexenyl group, and a cycloheptenyl group. The term "C₃-C₁₀ cycloalkenylene group," as used herein, refers to a divalent group having the same (e.g., substantially the same) structure as the C₃-C₁₀ cycloalkenyl group.

The term "C₁-C₁₀ heterocycloalkenyl group," as used herein, refers to a monovalent monocyclic group that has at least one heteroatom selected from N, O, Si, P, and S as a ring-forming atom, 1 to 10 carbon atoms, and at least one double bond in its ring. Non-limiting examples of the C₁-C₁₀ heterocycloalkenyl group include a 4,5-dihydro-1,2,3,4-oxatriazolyl group, a 2,3-dihydrofuranyl group, and a 2,3-dihydrothiophenyl group. The term "C₁-C₁₀ heterocycloalkenylene group," as used herein, refers to a divalent group having the same (e.g., substantially the same) structure as the C₁-C₁₀ heterocycloalkenyl group.

The term "C₆-C₆₀ aryl group," as used herein, refers to a monovalent group having a carbocyclic aromatic system having 6 to 60 carbon atoms, and the term "C₆-C₆₀ arylene group," as used herein, refers to a divalent group having a carbocyclic aromatic system having 6 to 60 carbon atoms. Non-limiting examples of the C₆-C₆₀ aryl group include a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other (e.g., combined together). Corresponding definitions apply to other ranges given for the number of carbon atoms in an aryl/arylene group.

The term "C₁-C₆₀ heteroaryl group," as used herein, refers to a monovalent group having a heterocyclic aromatic system that has at least one heteroatom selected from N, O, Si, P, and S as a ring-forming atom, in addition to 1 to 60 carbon atoms. The term "C₁-C₆₀ heteroarylene group," as used herein refers to a divalent group having a heterocyclic aromatic system that has at least one heteroatom selected from N, O, Si, P, and S as a ring-forming atom, in addition to 1 to 60 carbon atoms. Non-limiting examples of the C₁-C₆₀ heteroaryl group include a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include two or more rings, the rings may be fused to each other. Corresponding definitions apply to other ranges given for the number of carbon atoms in an heteroaryl/heteroarylene group.

The term "C₆-C₆₀ aryloxy group," as used herein, refers to -OA₁₀₂ (wherein A₁₀₂ is the C₆-C₆₀ aryl group), and a C₆-C₆₀ arylthio group used herein indicates -SA₁₀₃ (wherein A₁₀₃ is the C₆-C₆₀ aryl group). Corresponding definitions apply to other ranges given for the number of carbon atoms in an aryloxy group and an arylthio group.

The term "monovalent non-aromatic condensed polycyclic group," as used herein, refers to a monovalent group (for example, having 8 to 60 carbon atoms) having two or more rings condensed with each other (e.g., combined together), only carbon atoms as ring-forming atoms, and no aromaticity in its entire molecular structure (e.g., the entire ring, group, or molecule is not aromatic). An example of the monovalent non-aromatic condensed polycyclic group is a fluorenyl group. The term "divalent non-aromatic condensed polycyclic group," as used herein, refers to a divalent group having the same (e.g., substantially the same) structure as the monovalent non-aromatic condensed polycyclic group.

The term "monovalent non-aromatic condensed heteropolycyclic group," as used herein, refers to a monovalent group (for example, having 1 to 60 carbon atoms) having two or more rings condensed to each other (e.g., combined together), at least one heteroatom selected from N, O, Si, P, and S, other than carbon atoms, as a ring-forming atom, and no aromaticity in its entire molecular structure (e.g., the entire ring, group, and/or molecule is not aromatic). An example of the monovalent non-aromatic condensed heteropolycyclic group is a carbazolyl group. The term "divalent non-aromatic condensed heteropolycyclic group," as used herein refers to a divalent group having the same (e.g., substantially the same) structure as the monovalent non-aromatic condensed heteropolycyclic group.

The term "C₅-C₆₀ carbocyclic group," as used herein, refers to a monocyclic or polycyclic group having 5 to 60 carbon atoms in which a ring-forming atom is a carbon atom only. The C₅-C₆₀ carbocyclic group may be an aromatic carbocyclic group or a non-aromatic carbocyclic group. The C₅-C₆₀ carbocyclic group may be a ring, such as benzene, a monovalent group, such as a phenyl group, or a divalent group, such as a phenylene group. In one or more embodiments, depending on the number of substituents coupled to (e.g., connected to) the C₅-C₆₀ carbocyclic group, the C₅-C₆₀ carbocyclic group may be a trivalent group or a quadrivalent group. Corresponding definitions apply to other ranges given for the number of carbon atoms in a carbocyclic group.

The term "C₁-C₆₀ heterocyclic group," as used herein, refers to a group having the same (e.g., substantially the same) structure as the C₅-C₆₀ carbocyclic group, except that as a ring-forming atom, at least one heteroatom selected from N, O, Si, P, and S is used in addition to carbon (the number of carbon atoms may be in a range of 1 to 60). Corresponding definitions apply to other ranges given for the number of carbon atoms in a heterocyclic group.

Apart from the definitions for R₁ to R₄ and R₁₁ to R₁₄ already described above, at least one selected from substituent of the substituted C₅-C₆₀ (*e.g.* C₅-C₃₀) carbocyclic group, the substituted C₁-C₆₀ (*e.g.* C₁-C₂₀) heterocyclic group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ (*e.g.* C₆-C₃₀) arylene group, the substituted C₁-C₆₀ (*e.g.* C₁-C₂₀) heteroarylene group, the substituted divalent non-aromatic condensed polycyclic group, the substituted divalent non-aromatic condensed heteropolycyclic group, the substituted C₁-C₆₀ (*e.g.* C₁-C₂₀) alkyl group, the substituted C₂-C₆₀ (*e.g.* C₂-C₂₀) alkenyl group, the substituted C₂-C₆₀ (*e.g.* C₂-C₂₀) alkynyl group, the substituted C₁-C₆₀ (*e.g.* C₁-C₂₀) alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ (*e.g.* C₆-C₃₀) aryl group, the substituted C₆-C₆₀ (*e.g.* C₆-C₃₀) aryloxy group, the substituted C₆-C₆₀ (*e.g.* C₆-C₃₀) arylthio group, the substituted C₁-C₆₀ (e*.g.* C₁-C₂₀) heteroaryl group, the substituted monovalent non-aromatic condensed polycyclic group, and the substituted monovalent non-aromatic condensed heteropolycyclic group may be selected from:
deuterium, -F, -Cl, -Br, -I, a hydroxyl group, a cyano group, a nitro group, an amidino group, a hydrazine group (a hydrazino group), a hydrazone group (a hydrazono group), a C₁-C₆₀ (e.g. C₁-C₂₀) alkyl group, a C₂-C₆₀ (*e.g.* C₂-C₂₀) alkenyl group, a C₂-C₆₀ (*e.g.* C₂-C₂₀) alkynyl group, and a C₁-C₆₀ (*e.g.* C₁-C₂₀) alkoxy group;
a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, and a C₁-C₆₀ (*e.g.* C₁-C₂₀) alkoxy group, each substituted with at least one selected from deuterium, -F, -Cl, -Br, -I, a hydroxyl group, a cyano group, a nitro group, an amidino group, a hydrazine group (a hydrazino group), a hydrazone group (a hydrazono group), a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryl group, a monovalent non-aromatic condensed polycyclic group, a monovalent non-aromatic condensed heteropolycyclic group, -O(Q₁₁), -S(Q₁₁), -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁), and -P(=O)(Q₁₁)(Q₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₆-C₆₀ (e*.g.* C₆-C₃₀) aryloxy group, a C₆-C₆₀ (*e.g.* C₆-C₃₀) arylthio group, a C₁-C₆₀ (*e.g.* C₁-C₂₀) heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₆-C₆₀ (e.g. C₆-C₃₀) aryloxy group, a C₆-C₆₀ (*e.g.* C₆-C₃₀) arylthio group, a C₁-C₆₀ (*e.g.* C₁-C₂₀) heteroaryl group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazine group (a hydrazino group), a hydrazone group (a hydrazono group), a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, a C₁-C₆₀ (*e.g.* C₁-C₂₀) alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryl group, a monovalent non-aromatic condensed polycyclic group, a monovalent non-aromatic condensed heteropolycyclic group, -O(Q₂₁), -S(Q₂₁), -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), and - P(=O)(Q₂₁)(Q₂₂); and
-O(Q₃₁), -S(Q₃₁), -Si(Q₃₁)(Q₃₂)(Q₃₃), -N(Q₃₁)(Q₃₂), -B(Q₃₁)(Q₃₂), -C(=O)(Q₃₁), - S(=O)₂(Q₃₁), and -P(=O)(Q₃₁)(Q₃₂), and
Q₁ to Q₃, Q₁₁ to Q₁₃, Q₂₁ to Q₂₃, and Q₃₁ to Q₃₃ may each independently be selected from hydrogen, deuterium, -F, -Cl, -Br, -I, a hydroxyl group, a cyano group, a nitro group, an amidino group, a hydrazine group (a hydrazino group), a hydrazone group (a hydrazono group), a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, a C₁-C₆₀ (*e.g.* C₁-C₂₀) alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ (*e.g.* C₆-C₃₀) aryl group, a C₁-C₆₀ (*e.g.* C₁-C₂₀) heteroaryl group, a monovalent non-aromatic condensed polycyclic group, a monovalent non-aromatic condensed heteropolycyclic group, a biphenyl group, a terphenyl group, a C₁-C₆₀ (*e.g.* C₁-C₂₀) alkyl group substituted with at least one selected from deuterium, -F, a cyano group, a C₁-C₆₀ (*e.g.* C₁-C₂₀) alkyl group, a phenyl group, and a biphenyl group, and a C₆-C₆₀ (*e.g.* C₆-C₃₀) aryl group substituted with at least one selected from deuterium, -F, a cyano group, a C₁-C₁₀ alkyl group, a phenyl group, and a biphenyl group.

The term "Ph," as used herein, may refer to a phenyl group; the term "Me," as used herein, may refer to a methyl group; the term "Et," as used herein, may refer to an ethyl group; the terms "ter-Bu" or "But," as used herein, may refer to a tert-butyl group; and the term "OMe," as used herein, may refer to a methoxy group.

The term "biphenyl group," as used herein, refers to "a phenyl group substituted with a phenyl group." In other words, the "biphenyl group" is a substituted phenyl group having a C₆-C₆₀ aryl group as a substituent.

The term "terphenyl group," as used herein, refers to "a phenyl group substituted with a biphenyl group." In other words, the "terphenyl group" is a phenyl group having, as a substituent, a C₆-C₆₀ aryl group substituted with a C₆-C₆₀ aryl group.

* and *' used herein, unless defined otherwise, each refer to a binding site to a neighboring atom in a corresponding formula.

Hereinafter, a compound according to embodiments and an organic light-emitting device according to embodiments will be described in more detail with reference to Synthesis Examples and Examples. The wording "B was used instead of A" used in describing Synthesis Examples refers to that an identical molar equivalent of B was used in place of A.

### Examples

### Synthesis Example 1 (Compound 2)

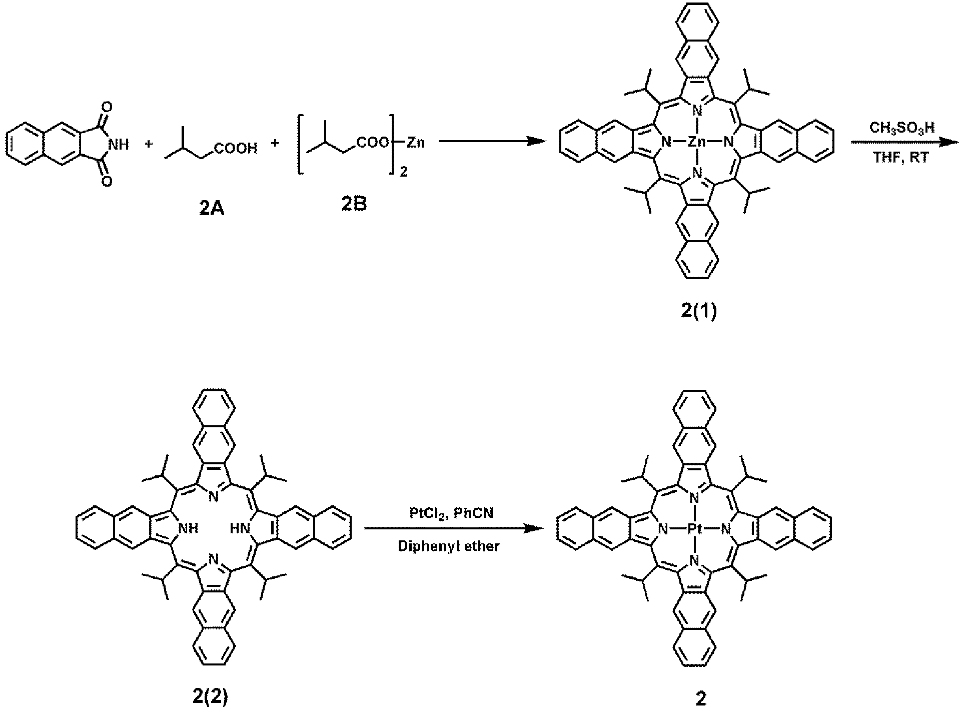

### Synthesis of Intermediate 2(2)

2,3-naphthalenedicarboxyimide (150 g, 0.76 mol), isopropylacetic acid (starting material 2A, 116 g, 1.14 mol), and zinc isopropylacetate (starting material 2B, 51 g, 0.19 mol) were mixed in a 250 ml reactor, and then, stirred under reflux at a temperature of 360 °C for 24 hours. After the reaction was completed, the temperature was cooled to room temperature, and a resulting product, Intermediate 2(1), was dissolved in 3 L of tetrahydrofuran without a further purification process, and then, 700 ml of methane sulfonic acid was added dropwise thereto. After 2 hours, an extraction process was performed thereon by using water and dichloromethane, and an organic layer extracted thereto was separated by silica gel column chromatography (using a mixed solvent of dichloromethane and methanol), thereby synthesizing Intermediate 2(2) (0.50 g, 0.57 mmol, yield: 0.3%).

### Synthesis of Compound 2

Dichloroplatinum(II)dichloride (0.30 g, 1.14 mmol) was added to in a 20 ml reactor, and 3 ml of benzonitrile was added thereto. The mixed solution was then stirred under reflux at a temperature of 160 °C for 30 minutes in a nitrogen atmosphere. A product obtained therefrom was added dropwise to a mixture containing 30 ml of phenyl diether and Intermediate 2(2) (0.50 g, 0.57 mmol), and the resulting mixed solution was stirred under reflux at a temperature of 190 °C for 3 hours. After the reaction was completed, the temperature was cooled to room temperature, and a resulting product was separated by silica gel column chromatography (using dichloromethane), thereby synthesizing Compound 2 (0.31 g, 0.29 mmol, yield: 50%). MS (MALDI-TOF) m/z: 1072 [M]+

### Synthesis Example 2 (Compound 8)

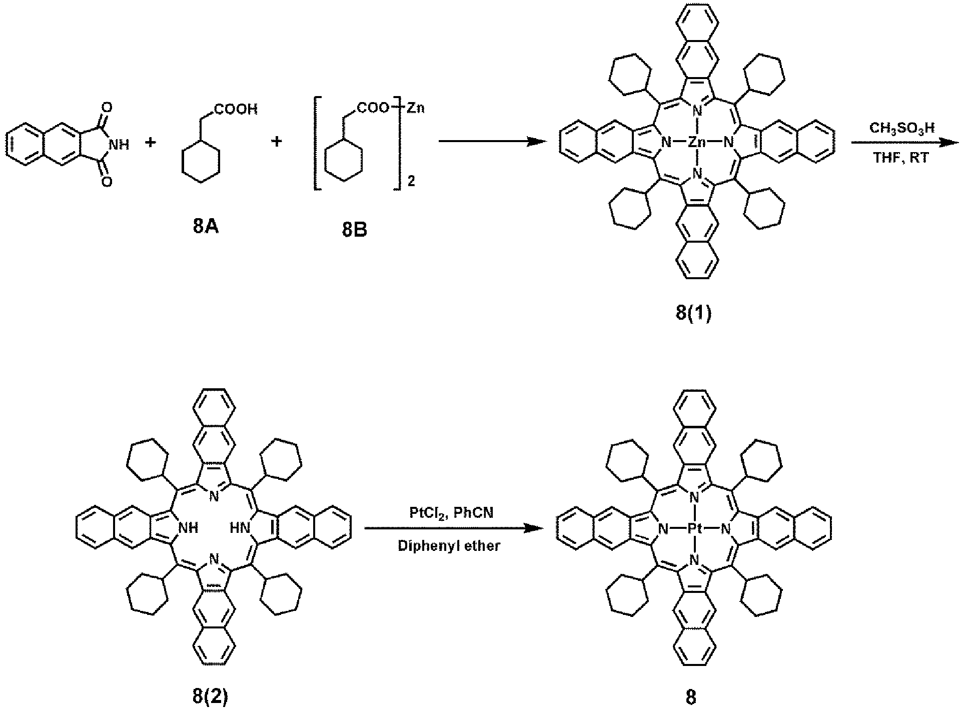

Compound 8 (0.28 g, 0.23 mmol, yield: 0.12%) was synthesized in substantially the same manner as in Synthesis Example 1, except that Intermediate 8(2) was synthesized by using starting materials 8A and 8B instead of starting materials 2A and 2B, respectively, in synthesizing Intermediate 2(2), and that Intermediate 8(2) was used instead of Intermediate 2(2) in synthesizing Compound 2.
MS (MALDI-TOF) m/z: 1232 [M]+

### Synthesis Example 3 (Compound 10)

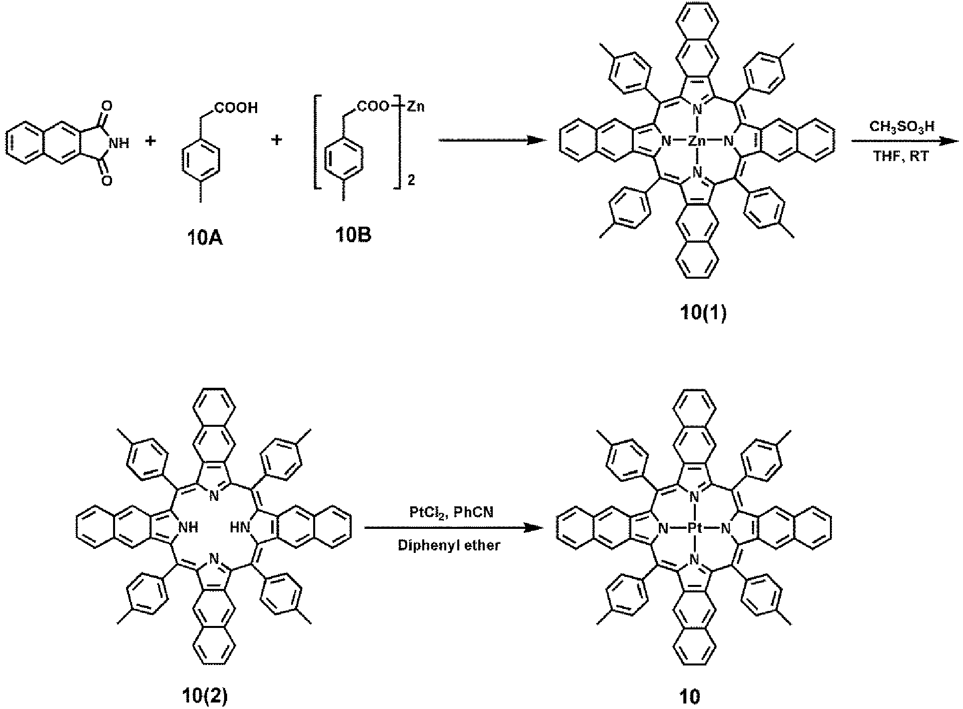

Compound 10 (0.43 g, 0.34 mmol, yield: 0.18%) was synthesized in substantially the same manner as in Synthesis Example 1, except that Intermediate 10(2) was synthesized by using starting materials 10A and 10B instead of starting materials 2A and 2B, respectively, in synthesizing Intermediate 2(2), and that Intermediate 10(2) was used instead of Intermediate 2(2) in synthesizing Compound 2.
MS (MALDI-TOF) m/z: 1264 [M]+

### Synthesis Example 4 (Compound 22)

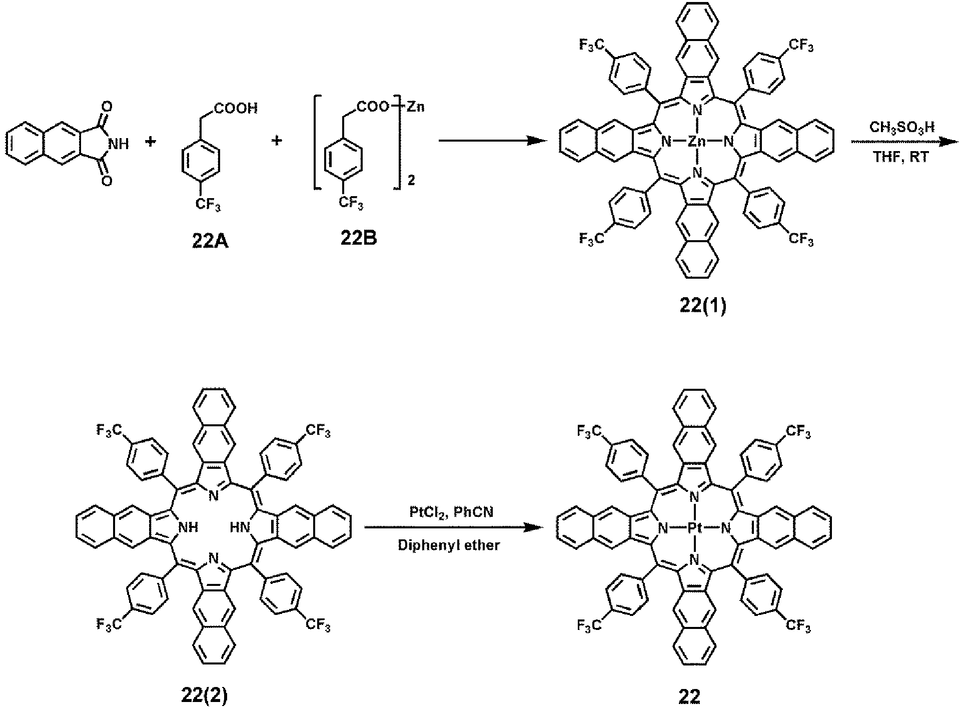

Compound 22 (0.23 g, 0.15 mmol, yield: 0.08%) was synthesized in substantially the same manner as in Synthesis Example 1, except that Intermediate 22(2) was synthesized by using starting materials 22A and 22B instead of starting materials 2A and 2B, respectively, in synthesizing Intermediate 2(2), and that Intermediate 22(2) was used instead of Intermediate 2(2) in synthesizing Compound 2.
MS (MALDI-TOF) m/z: 1480 [M]+

### Synthesis Example 5 (Compound 24)

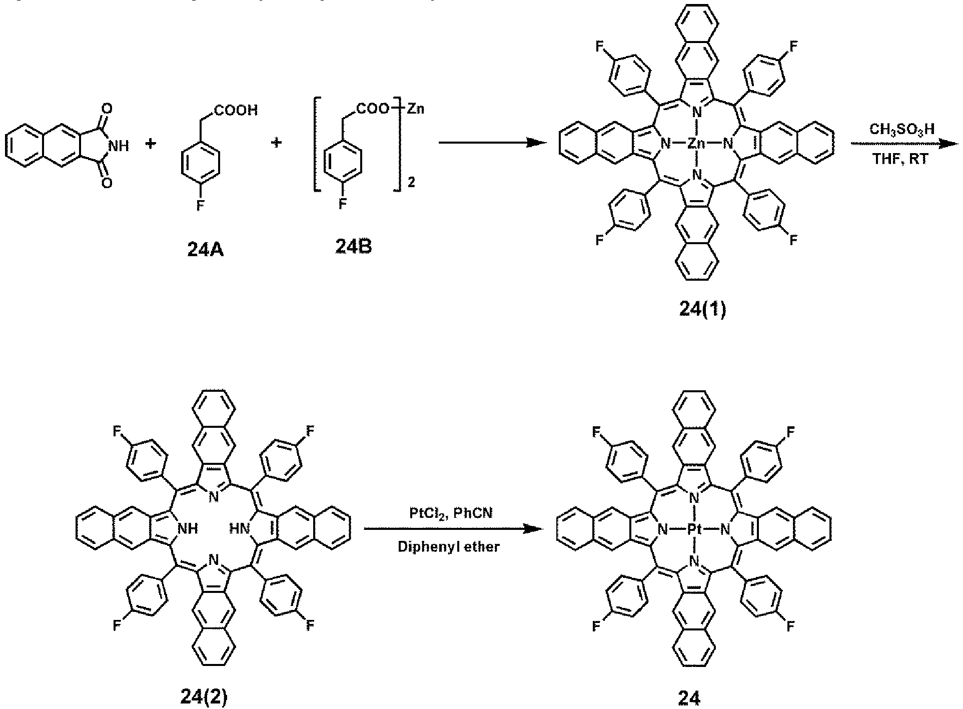

Compound 24 (0.27g, 0.21 mmol, yield: 0.11 %) was synthesized in substantially the same manner as in Synthesis Example 1, except that Intermediate 24(2) was synthesized by using starting materials 24A and 24B instead of starting materials 2A and 2B, respectively, in synthesizing Intermediate 2(2), and that Intermediate 24(2) was used instead of Intermediate 2(2) in synthesizing Compound 2.
MS (MALDI-TOF) m/z: 1280 [M]+

### Synthesis Example 6 (Compound 39)

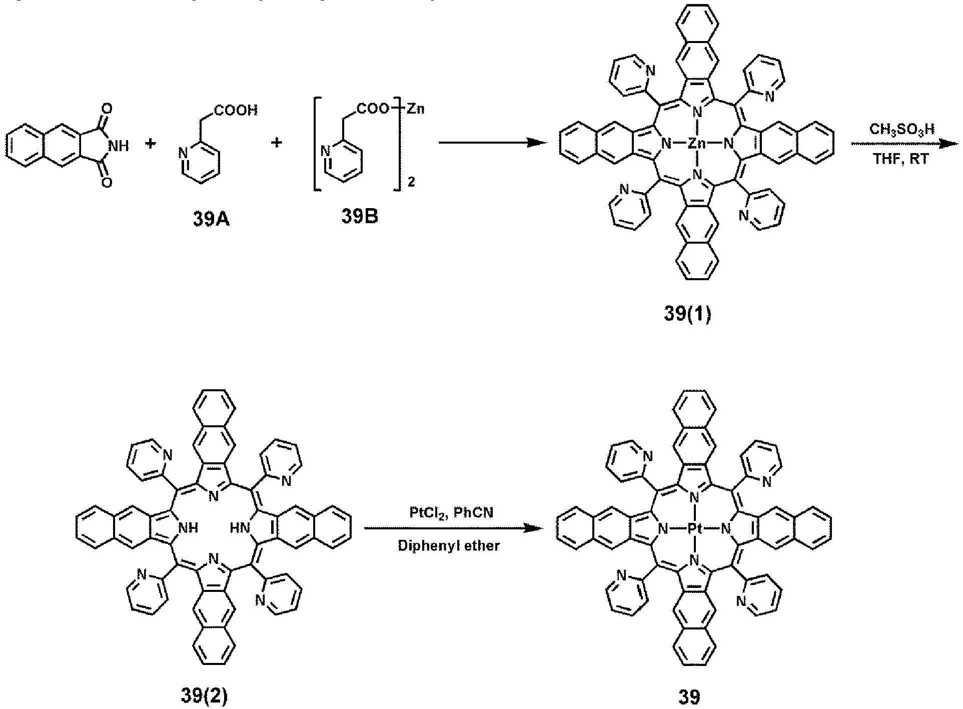

Compound 39 (0.14 g, 0.11 mmol, yield: 0.06%) was synthesized in substantially the same manner as in Synthesis Example 1, except that Intermediate 39(2) was synthesized by using starting materials 39A and 39B instead of starting materials 2A and 2B, respectively, in synthesizing Intermediate 2(2), and that Intermediate 39(2) was used instead of Intermediate 2(2) in synthesizing Compound 2.
MS (MALDI-TOF) m/z: 1212 [M]+

### Synthesis Example 7 (Compound 43)

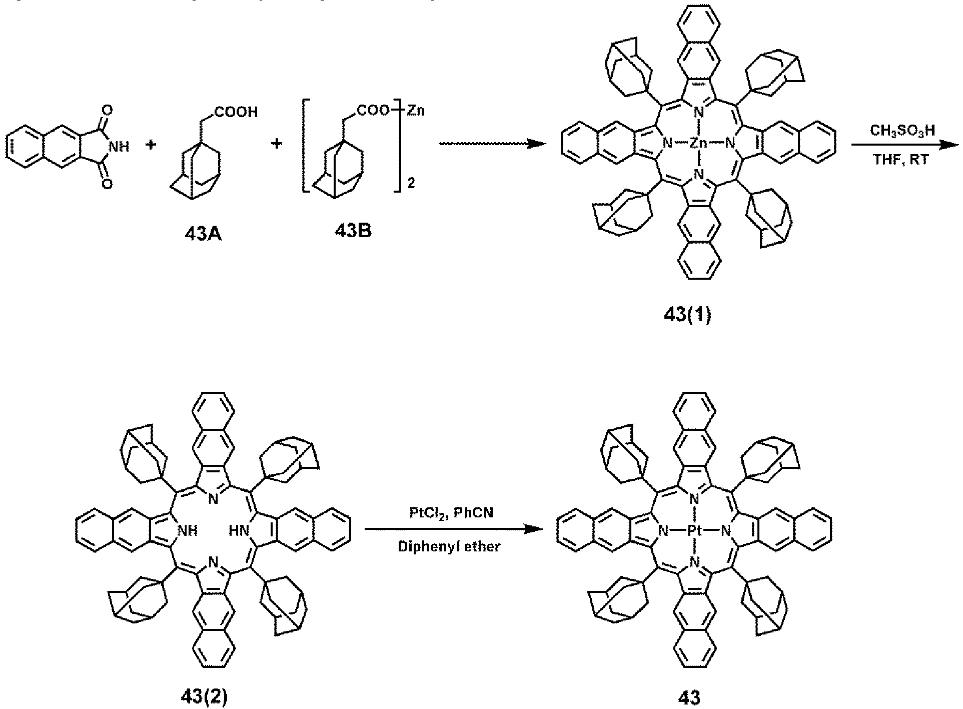

Compound 43 (0.25 g, 0.17 mmol, yield: 0.09%) was synthesized in substantially the same manner as in Synthesis Example 1, except that Intermediate 43(2) was synthesized by using starting materials 43A and 43B instead of starting materials 2A and 2B, respectively, in synthesizing Intermediate 2(2), and that Intermediate 43(2) was used instead of Intermediate 2(2) in synthesizing Compound 2.
MS (MALDI-TOF) m/z: 1440 [M]+

### Synthesis Example 8 (Compound 31)

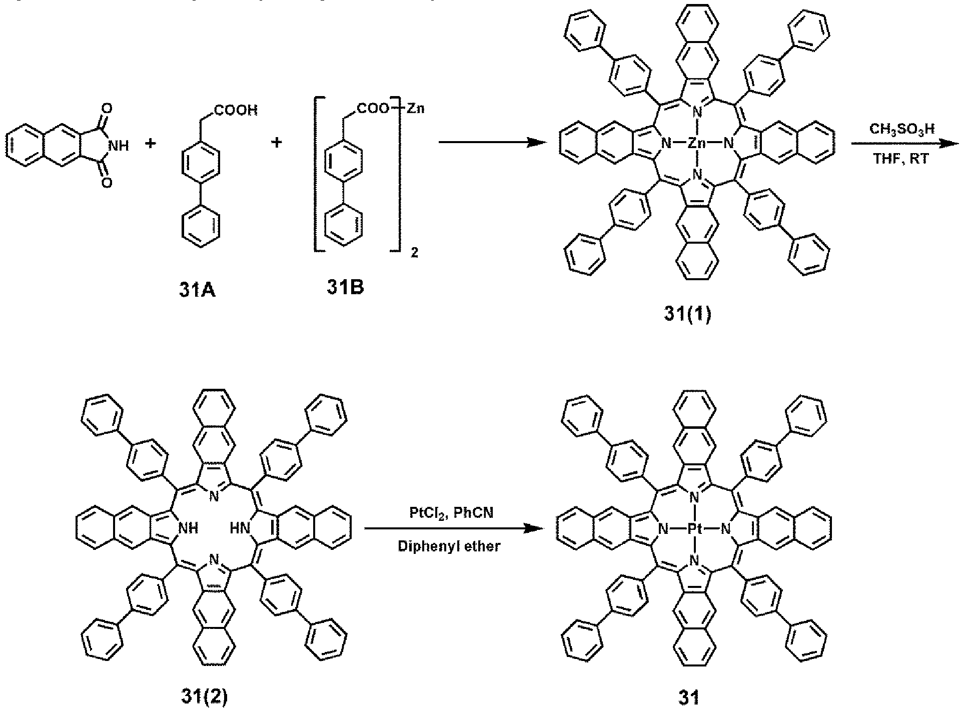

Compound 31 (0.29 g, 0.19 mmol, yield: 0.10%) was synthesized in substantially the same manner as in Synthesis Example 1, except that Intermediate 31(2) was synthesized by using starting materials 31A and 31B instead of starting materials 2A and 2B, respectively, in synthesizing Intermediate 2(2), and that Intermediate 31(2) was used instead of Intermediate 2(2) in synthesizing Compound 2.
MS (MALDI-TOF) m/z: 1512 [M]+

### Synthesis Example 9 (Compound 59)

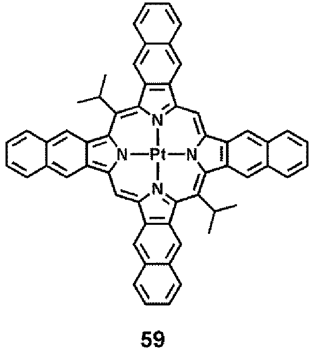

Compound 59 (0.06 g, 0.06 mmol, yield: 0.03%), which was produced as a by-product in Synthesis Example 1 was collected.
MS (MALDI-TOF) m/z: 988 [M]+

### Synthesis Example 10 (Compound 75)

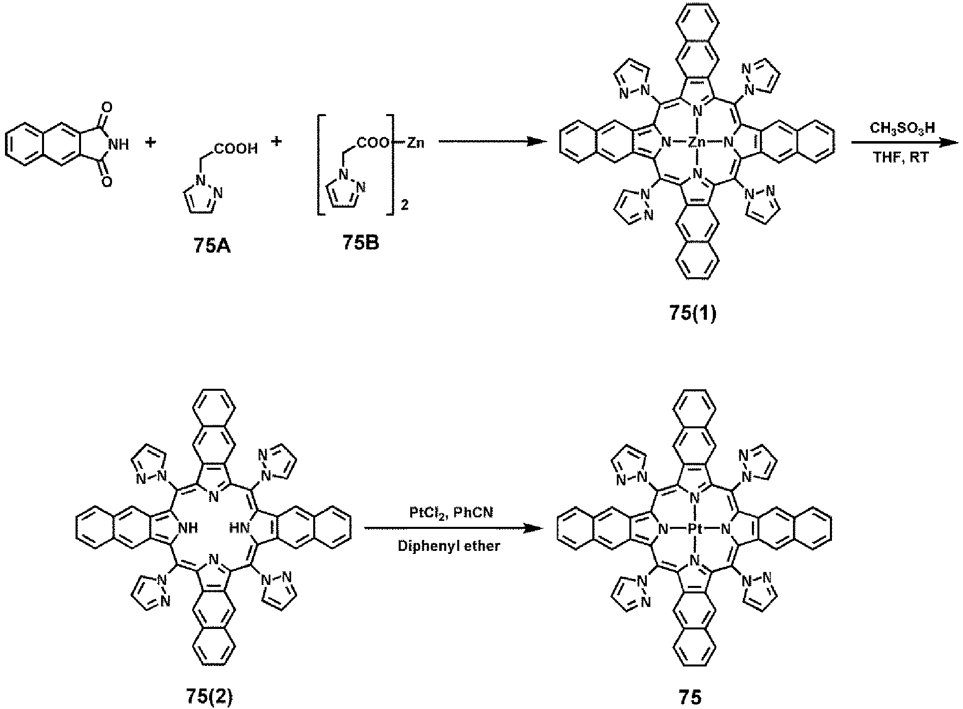

Compound 75 (0.18 g, 0.15 mmol, yield: 0.08%) was synthesized in substantially the same manner as in Synthesis Example 1, except that Intermediate 75(2) was synthesized by using starting materials 75A and 75B instead of starting materials 2A and 2B, respectively, in synthesizing Intermediate 2(2), and that Intermediate 75(2) was 2 used instead of Intermediate 2(2) in synthesizing Compound 2.
MS (MALDI-TOF) m/z: 1168 [M]+

### Comparative Synthesis Example A (Compound A)

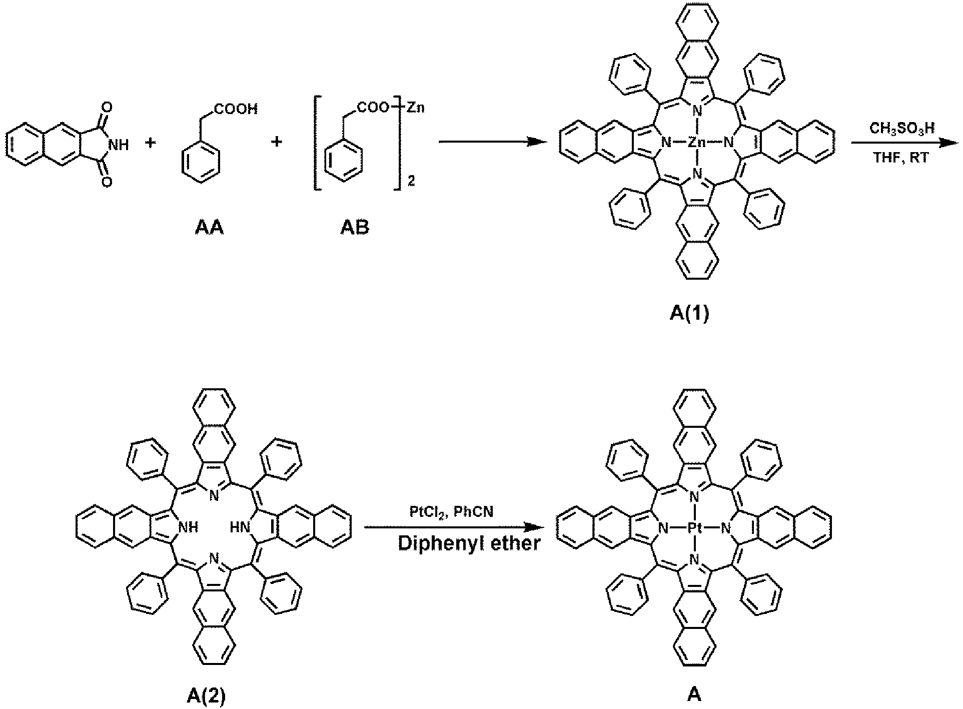

Compound A (0.46 g, 0.38 mmol, yield: 0.20%) was synthesized in substantially the same manner as in Synthesis Example 1, except that Intermediate A(2) was synthesized by using starting materials AA and AB instead of starting materials 2A and 2B, respectively, in synthesizing Intermediate 2(2), and that Intermediate A(2) was used instead of Intermediate 2(2) in synthesizing Compound 2.
MS (MALDI-TOF) m/z: 1208 [M]+

### Comparative Synthesis Example B (Compound B)

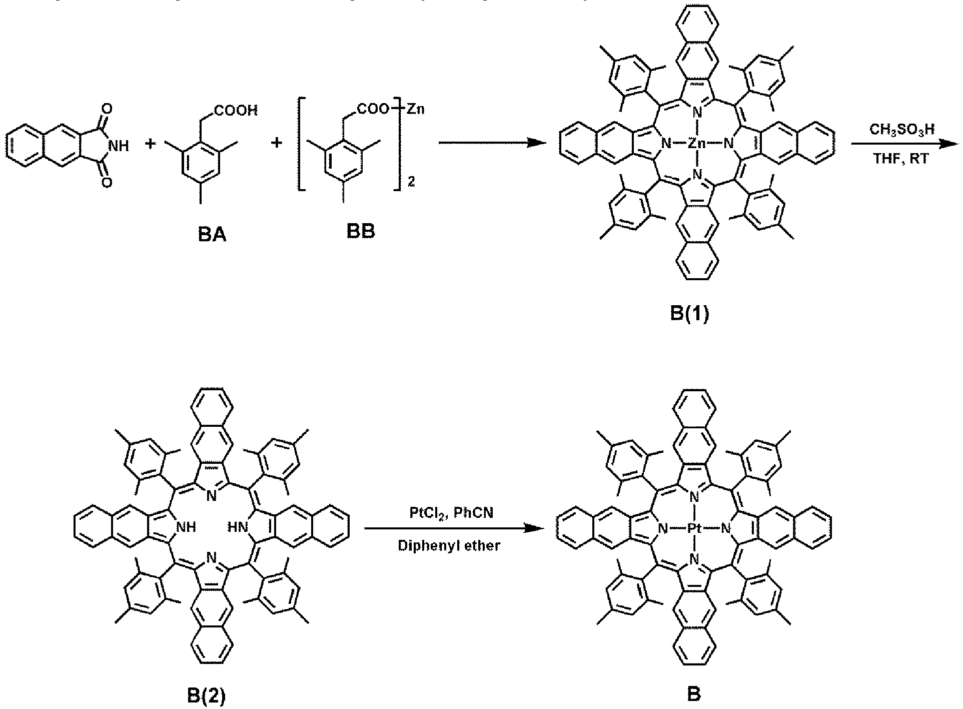

Compound B (0.26 g, 0.19 mmol, yield: 0.10%) was synthesized in substantially the same manner as in Synthesis Example 1, except that Intermediate B(2) was synthesized by using starting materials BA and BB instead of starting materials 2A and 2B, respectively, in synthesizing Intermediate 2(2), and that Intermediate B(2) was used instead of Intermediate 2(2) in synthesizing Compound 2.
MS (MALDI-TOF) m/z: 1376 [M]+

### Comparative Synthesis Example C (Compound C)

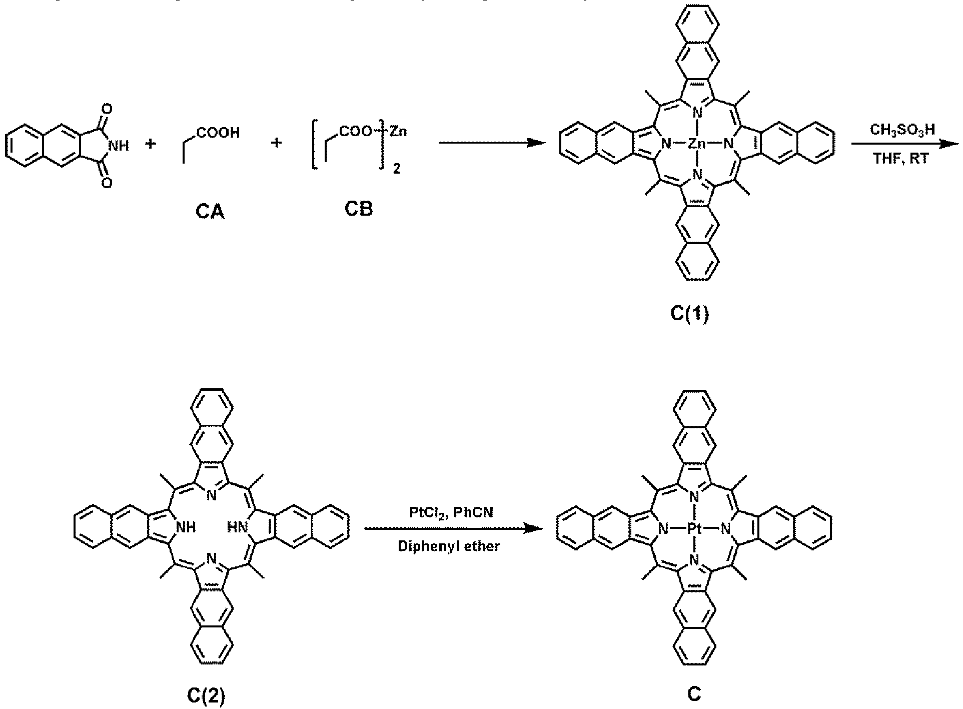

Compound C (0.11 g, 0.11 mmol, yield: 0.06%) was synthesized in substantially the same manner as in Synthesis Example 1, except that Intermediate C(2) was synthesized by using starting materials CA and CB instead of starting materials 2A and 2B, respectively, in synthesizing Intermediate 2(2), and that Intermediate C(2) was used instead of Intermediate 2(2) in synthesizing Compound 2.
MS (MALDI-TOF) m/z: 960 [M]+

### Example 1

As a substrate and an anode, a Corning 15 Ω/cm² (1,200 Å) ITO glass substrate was cut to a size of 50 mm x 50 mm x 0.7 mm, sonicated with isopropyl alcohol and pure water each for 5 minutes, and then cleaned by exposure to ultraviolet rays and ozone for 30 minutes. Then, the ITO glass substrate was provided to a vacuum deposition apparatus.

2-TNATA was vacuum-deposited on the ITO glass substrate to form a hole injection layer having a thickness of 600 Å, and NPB was vacuum-deposited on the hole injection layer to form a hole transport layer having a thickness of 300 Å.

BCPDS and POPCPA (at a weight ratio of 1 : 1 between BCPDS and POPCPA), which are co-hosts, and Compound 2, which is a dopant, were codeposited on the hole transport layer at a weight ratio of 97 : 3 to form an emission layer having a thickness of 300 Å.

TSPO1 was deposited on the emission layer to form a hole blocking layer having a thickness of 50 Å, and Alq₃ was deposited on the hole blocking layer to form an electron transport layer having a thickness of 300 Å. Then, LiF was deposited on the electron transport layer to form an electron injection layer having a thickness of 10 Å, and Al was vacuum-deposited on the electron injection layer to form a cathode having a thickness of 3,000 Å, thereby completing the manufacture of an organic light-emitting device.

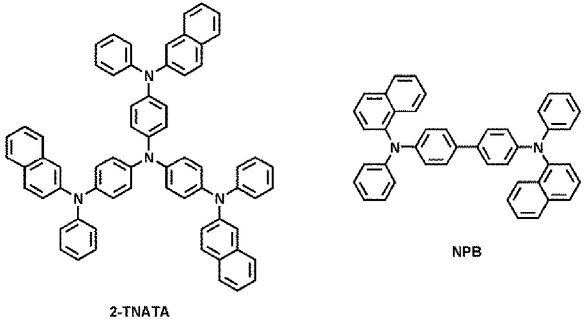

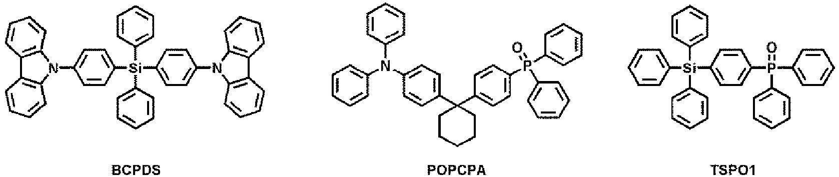

### Examples 2 to 10 and Comparative Examples A to C

Organic light-emitting devices were manufactured in substantially the same manner as in Example 1, except that compounds shown in Table 1 were each used as the dopant in forming an emission layer.

### Evaluation Example 1

The driving voltage, current density, external quantum luminescence efficiency (EQE), and maximum emission wavelength of the organic light-emitting devices manufactured according to Examples 1 to 10 and Comparative Examples A to C were measured by using Keithley SMU 236 and a luminance meter PR650, and results thereof are shown in Table 1. Here, the lifespan (T₉₅) indicates an amount of time that lapsed when luminance was 95% of initial luminance (100%).

**Table 1**

| | Dopant Compoun d No. | Driving voltage (V) | Current density (mA/cm²) | EQE (%) | Maximum emission wavelength (nm) |
|---|---|---|---|---|---|
| Example 1 | 2 | 6.5 | 100 | 2.21 | 900 |
| Example 2 | 8 | 6.4 | 100 | 2.20 | 905 |
| Example 3 | 10 | 6.1 | 100 | 2.10 | 900 |
| Example 4 | 22 | 6.0 | 100 | 2.05 | 901 |
| Example 5 | 24 | 6.1 | 100 | 2.20 | 905 |
| Example 6 | 39 | 6.3 | 100 | 2.15 | 905 |
| Example 7 | 43 | 6.1 | 100 | 2.10 | 904 |
| Example 8 | 31 | 6.2 | 100 | 2.06 | 900 |
| Example 9 | 59 | 6.3 | 100 | 2.05 | 901 |
| Example 10 | 75 | 6.5 | 100 | 2.20 | 900 |
| Comparative Example A | A | 7.2 | 100 | 1.90 | 900 |
| Comparative Example B | B | 6.9 | 100 | 2.0 | 901 |
| Comparative Example C | C | 7.1 | 100 | 1.87 | 900 |

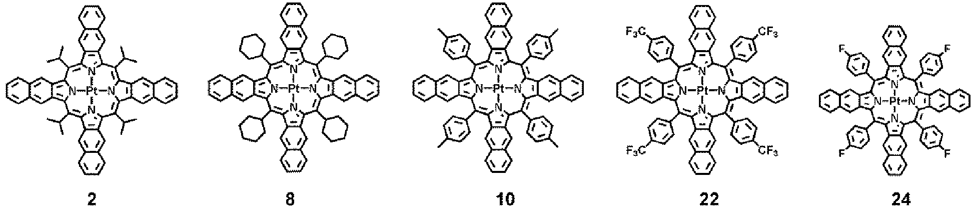

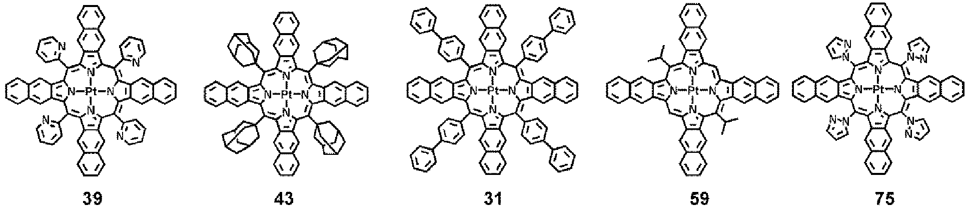

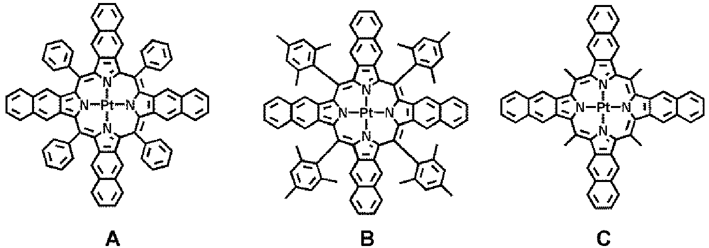

Referring to Table 1, it was confirmed that the organic light-emitting devices of Examples 1 to 10 had low driving voltage and high EQE, as compared with the organic light-emitting devices of Comparative Examples A to C. Accordingly, the organic light-emitting devices of Examples 1 to 10 were able to emit near-infrared light.

According to the one or more embodiments, an organic light-emitting device including an organometallic compound may have low driving voltage, high emission efficiency, and/or long lifespan, and in this regard, the organometallic compound may be utilized to implement a high-quality organic light-emitting device and an electronic device.

It should be understood that the embodiments described herein should be considered in a descriptive sense only and not for purposes of limitation. Descriptions of features or aspects within each embodiment should typically be considered as available for other similar features or aspects in other embodiments.

It will be understood that, although the terms "first," "second," "third," etc., may be used herein to describe various elements, components, regions, layers and/or sections, these elements, components, regions, layers and/or sections should not be limited by these terms. These terms are used to distinguish one element, component, region, layer or section from another element, component, region, layer or section. Thus, a first element, component, region, layer or section described above could be termed a second element, component, region, layer or section, without departing from the scope of the present disclosure.

Spatially relative terms, such as "beneath," "below," "lower," "under," "above," "upper," and the like, may be used herein for ease of explanation to describe one element or feature's relationship to another element(s) or feature(s) as illustrated in the figures. It will be understood that the spatially relative terms are intended to encompass different orientations of the device in use or in operation, in addition to the orientation depicted in the figures. For example, if the device in the figures is turned over, elements described as "below" or "beneath" or "under" other elements or features would then be oriented "above" the other elements or features. Thus, the example terms "below" and "under" can encompass both an orientation of above and below. The device may be otherwise oriented (e.g., rotated 90 degrees or at other orientations) and the spatially relative descriptors used herein should be interpreted accordingly.

It will be understood that when an element or layer is referred to as being "on," "connected to," or "coupled to" another element or layer, it can be directly on, connected to, or coupled to the other element or layer, or one or more intervening elements or layers may be present. In addition, it will also be understood that when an element or layer is referred to as being "between" two elements or layers, it can be the only element or layer between the two elements or layers, or one or more intervening elements or layers may also be present.

The terminology used herein is for the purpose of describing particular embodiments only and is not intended to be limiting of the present disclosure. As used herein, the singular forms "a" and "an" are intended to include the plural forms as well, unless the context clearly indicates otherwise. It will be further understood that the terms "comprises," "comprising," "includes," and "including," when used in this specification, specify the presence of the stated features, integers, acts, operations, elements, and/or components, but do not preclude the presence or addition of one or more other features, integers, acts, operations, elements, components, and/or groups thereof. As used herein, the term "and/or" includes any and all combinations of one or more of the associated listed items. Expressions such as "at least one of," when preceding a list of elements, modify the entire list of elements and do not modify the individual elements of the list.

As used herein, the terms "substantially," "about," and similar terms are used as terms of approximation and not as terms of degree, and are intended to account for the inherent deviations in measured or calculated values that would be recognized by those of ordinary skill in the art. Further, the use of "may" when describing embodiments of the present disclosure refers to "one or more embodiments of the present disclosure." As used herein, the terms "use," "using," and "used" may be considered synonymous with the terms "utilize," "utilizing," and "utilized," respectively.

Also, any numerical range recited herein is intended to include all subranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein, and any minimum numerical limitation recited in this specification is intended to include all higher numerical limitations subsumed therein.

While one or more embodiments have been described with reference to the accompanying drawing, it will be understood by those of ordinary skill in the art that various changes in form and details may be made therein without departing from the scope as defined by the following claims.

## Claims

1. An organometallic compound represented by Formula 1 and emitting near-infrared phosphorescent light having a maximum emission wavelength in a range from 720 nm to 2,500 nm: 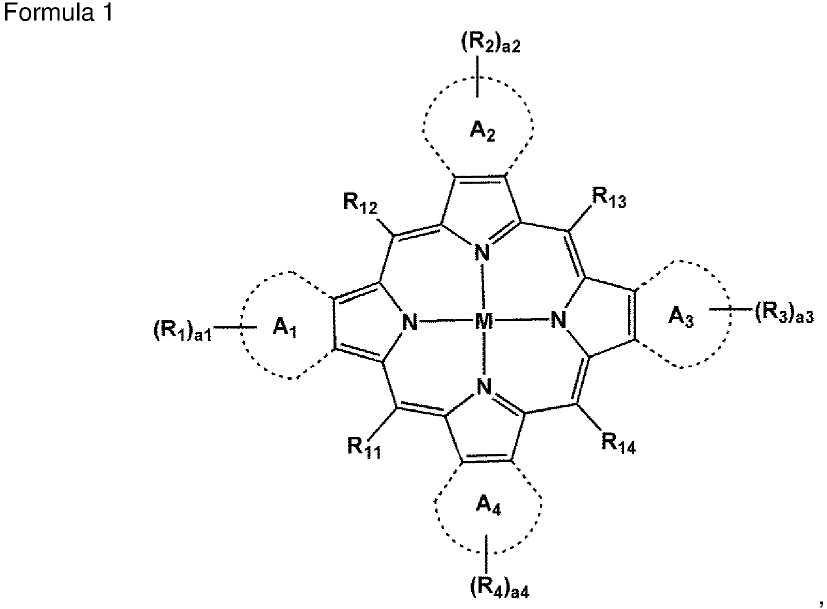 wherein, in Formula 1,
M is a transition metal,
ring A₁ to ring A₄ are each independently a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, a pyrrole group, an indole group, an indene group, a benzosilole group, a benzothiophene group, a benzofuran group, a carbazole group, a fluorene group, a dibenzosilole group, a dibenzothiophene group, a dibenzofuran group, a pyridine group, a pyrimidine group, a pyrazine group, a pyridazine group, a triazine group, a quinoline group, an isoquinoline group, a quinazoline group, a phenanthroline group, a pyrazole group, an imidazole group, a triazole group, an oxazole group, an isooxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group,
R₁ to R₄ and R₁₁ to R₁₄ are each independently selected from hydrogen, deuterium, -F, -Cl, -Br, -I, a hydroxyl group, a cyano group, a nitro group, an amidino group, a hydrazino group, a hydrazono group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -O(Q₁), -S(Q₁), -Si(Q₁)(Q₂)(Qₐ), -N(Q₁)(Q₂), -B(Q₁)(Q₂), -P(Q₁)(Q₂), -C(=O)(Q₁), -S(=O)₂(Q₁), and -P(=O)(Q₁)(Q₂),
a1 to a4 are each independently an integer from 0 to 10,
a) R₁₁ to R₁₄ are not simultaneously hydrogen, b) R₁₁ to R₁₄ are not simultaneously a phenyl group, c) R₁₁ to R₁₄ are not simultaneously a mesityl group, or d) R₁₁ to R₁₄ are not simultaneously a methyl group,
two selected from R₁₁ to R₁₄ are not linked to each other,
at least one substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₁-C₆₀ heteroaryl group, the substituted monovalent non-aromatic condensed polycyclic group, and the substituted monovalent non-aromatic condensed heteropolycyclic group is selected from:
deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, and a C₂-C₆₀ alkynyl group;
a C₁-C₆₀ alkyl group, a C₂-C₆₀ alkenyl group, and a C₂-C₆₀ alkynyl group, each substituted with at least one selected from deuterium, -F, -Cl, -Br, -I, a hydroxyl group, a cyano group, a nitro group, an amidino group, a hydrazino group, a hydrazono group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, a monovalent non-aromatic condensed heteropolycyclic group, -O(Q₁₁), -S(Q₁₁), -Si(Q₁₁)(Q₁₂)(Q₁₃), - N(Q₁₁)(Q₁₂), -B(Q₁₁)(Q₁₂), -P(Q₁₁)(Q₁₂), -C(=O)(Q₁₁), -S(=O)₂(Q₁₁), and - P(=O)(Q₁₁)(Q₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, a monovalent non-aromatic condensed heteropolycyclic group, -O(Q₂₁), -S(Q₂₁), -Si(Q₂₁)(Q₂₂)(Q₂₃), -N(Q₂₁)(Q₂₂), -B(Q₂₁)(Q₂₂), -P(Q₂₁)(Q₂₂), -C(=O)(Q₂₁), -S(=O)₂(Q₂₁), and -P(=O)(Q₂₁)(Q₂₂); and
-O(Q31), -S(Q₃₁), -Si(Q₃₁)(Q₃₂)(Q₃₃), -N(Q₃₁)(Q₃₂), -B(Q₃₁)(Q₃₂), -P(Q₃₁)(Q₃₂), - C(=O)(Q₃₁), -S(=O)₂(Q₃₁), and -P(=O)(Q₃₁)(Q₃₂), and
Q₁ to Q₃, Q₁₁ to Q₁₃, Q₂₁ to Q₂₃, and Q₃₁ to Q₃₃ are each independently selected from hydrogen, deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, a monovalent non-aromatic condensed heteropolycyclic group, a biphenyl group, a terphenyl group, a C₁-C₆₀ alkyl group substituted with at least one selected from deuterium, -F, a cyano group, a C₁-C₆₀ alkyl group, a phenyl group, and a biphenyl group, and a C₆-C₆₀ aryl group substituted with at least one selected from deuterium, -F, a cyano group, a C₁-C₁₀ alkyl group, a phenyl group, and a biphenyl group,
and the organometallic compound is not any of the following compounds: 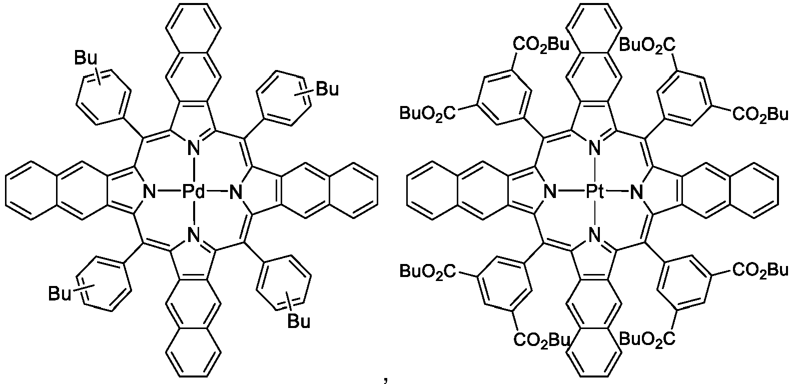 or

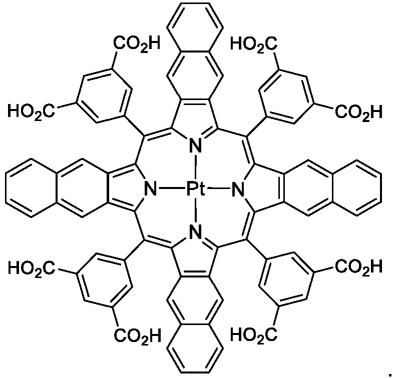

2. An organometallic compound according to claim 1, wherein M is platinum (Pt), palladium (Pd), or gold (Au).

3. An organometallic compound according to claim 1 or claim 2, wherein ring A₁ to ring A₄ are each independently selected from groups represented by Formulae 2-2 to 2-13: 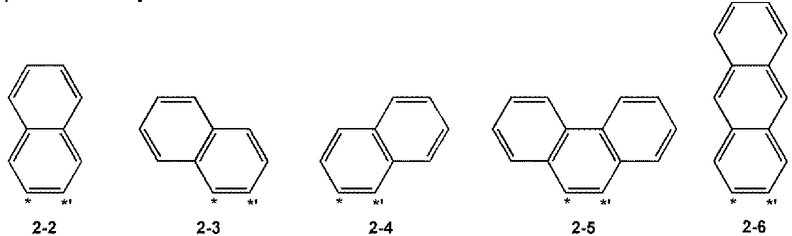 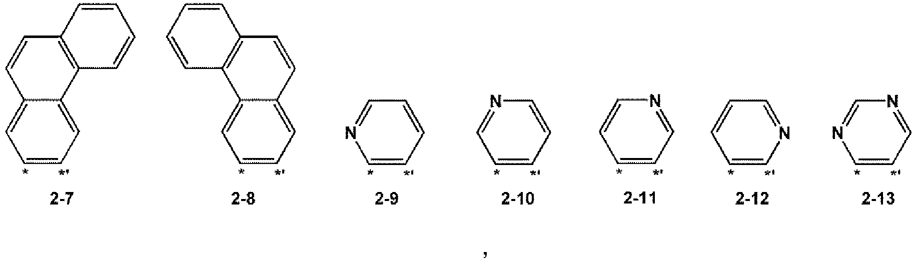 wherein, in Formulae 2-2 to 2-13, * and *' each indicate a position of a carbon atom shared with a neighboring 5-membered ring to which each of ring A₁ to ring A₄ is condensed, respectively.

4. An organometallic compound according to any one of claims 1 to 3, wherein:
R₁ to R₄ and R₁₁ to R₁₄ are each independently selected from:
hydrogen, deuterium, -F, -Cl, -Br, -I, a hydroxyl group, a cyano group, a nitro group, an amidino group, a hydrazino group, and a hydrazono group;
a C₁-C₁₀ alkyl group, a C₂-C₁₀ alkenyl group, a C₂-C₁₀ alkynyl group, and a C₁-C₁₀ alkoxy group, each unsubstituted or substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazino group, a hydrazono group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a tetrahydro-2H-pyranyl group, a morpholinyl group, a phenyl group, a (C₁-C₁₀ alkyl)phenyl group, a di(C₁-C₁₀ alkyl)phenyl group, a biphenyl group, a di(phenyl)phenyl group, a terphenyl group, a naphthyl group, a pyridinyl group, a pyrimidinyl group, -O(Q₃₁), -S(Q₃₁), - Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁), -S(=O)₂(Q₃₁), and -P(=O)(Q₃₁)(Q₃₂);
a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a tetrahydro-2H-pyranyl group, a morpholinyl group, a dioxanyl group, a phenyl group, a (C₁-C₁₀ alkyl)phenyl group, a di(C₁-C₁₀ alkyl)phenyl group, a biphenyl group, a di(phenyl)phenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, a 2,3-dihydro-1H-indenyl group, a 1,2,3,4-tetrahydronaphthalenyl group, a 2,3-dihydrobenzodioxinyl group, and a benzodioxanyl group, each unsubstituted or substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazino group, a hydrazono group, a C₁-C₁₀ alkyl group, a C₂-C₁₀ alkenyl group, a C₂-C₁₀ alkynyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a tetrahydro-2H-pyranyl group, a morpholinyl group, a dioxanyl group, a phenyl group, a (C₁-C₁₀ alkyl)phenyl group, a di(C₁-C₁₀ alkyl)phenyl group, a biphenyl group, a di(phenyl)phenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, a 2,3-dihydro-1H-indenyl group, a 1,2,3,4-tetrahydronaphthalenyl group, a 2,3-dihydrobenzodioxinyl group, a benzodioxanyl group, -O(Q₃₁), -S(Q₃₁), -Si(Q₃₁)(Q₃₂)(Q₃₃), -N(Q₃₁)(Q₃₂), - B(Q₃₁)(Q₃₂), -P(Q₃₁)(Q₃₂), -C(=O)(Q₃₁), -S(=O)₂(Q₃₁), and -P(=O)(Q₃₁)(Q₃₂); and
-O(Q₁), -S(Q₁), -Si(Q₁)(Q₂)(Q₃), -N(Q₁)(Q₂), -B(Q₁)(Q₂), -P(Q₁)(Q₂), -C(=O)(Q₁), - S(=O)₂(Q₁), and -P(=O)(Q₁)(Q₂), and
Q₁ to Q₃ and Q₃₁ to Q₃₃ are each independently selected from hydrogen, deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazino group, a hydrazono group, a C₁-C₁₀ alkyl group, a C₂-C₁₀ alkenyl group, a C₂-C₁₀ alkynyl group, a C₁-C₁₀ alkoxy group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a tetrahydro-2H-pyranyl group, a morpholinyl group, a dioxanyl group, a phenyl group, a (C₁-C₁₀ alkyl)phenyl group, a di(C₁-C₁₀ alkyl)phenyl group, a biphenyl group, a di(phenyl)phenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, a 2,3-dihydro-1H-indenyl group, a 1,2,3,4-tetrahydronaphthalenyl group, a 2,3-dihydrobenzodioxinyl group, and a benzodioxanyl group, preferably,
wherein:
R₁ to R₄ are each independently selected from hydrogen, deuterium, -F, -Cl, -Br, -I, -CD₃, -CD₂H, -CDH₂, -CF₃, -CF₂H, -CFH₂, C₁-C₁₀ alkyl group, a C₂-C₁₀ alkenyl group, a C₂-C₁₀ alkynyl group, a C₁-C₁₀ alkoxy group, a cyclopentyl group, a cyclohexyl group, a cycloheptyl group, a phenyl group, a (C₁-C₁₀ alkyl)phenyl group, a di(C₁-C₁₀ alkyl)phenyl group, a biphenyl group, a di(phenyl)phenyl group, a terphenyl group, - O(Q₁), and -S(Q₁), and
Q₁ is selected from a C₁-C₁₀ alkyl group, a C₂-C₁₀ alkenyl group, a C₂-C₁₀ alkynyl group, a C₁-C₁₀ alkoxy group, a cyclopentyl group, a cyclohexyl group, a cycloheptyl group, a phenyl group, a (C₁-C₁₀ alkyl)phenyl group, a di(C₁-C₁₀ alkyl)phenyl group, a biphenyl group, a di(phenyl)phenyl group, and a terphenyl group.

5. An organometallic compound according to any one of claims 1 to 4, wherein:
each of at least one selected from R₁₁ to R₁₄ is independently selected from a C₂-C₁₀ alkyl group, a C₂-C₁₀ alkenyl group, a C₂-C₁₀ alkynyl group, and a C₁-C₁₀ alkoxy group, each unsubstituted or substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazino group, a hydrazono group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a tetrahydro-2H-pyranyl group, a morpholinyl group, a phenyl group, a (C₁-C₁₀ alkyl)phenyl group, a di(C₁-C₁₀ alkyl)phenyl group, a biphenyl group, a di(phenyl)phenyl group, a terphenyl group, a naphthyl group, a pyridinyl group, a pyrimidinyl group, -O(Q₃₁), -S(Q₃₁), - Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁), -S(=O)₂(Q₃₁), and -P(=O)(Q₃₁)(Q₃₂), preferably wherein:
at least one selected from R₁₁ to R₁₄ is none of hydrogen, a phenyl group, a mesityl group, and a methyl group; and/or
each of at least one selected from R₁₁ to R₁₄ is independently -O(Q₁), -S(Q₁), - Si(Q₁)(Q₂)(Q₃), -N(Q₁)(Q₂), -B(Q₁)(Q₂), -P(Q₁)(Q₂), -C(=O)(Q₁), -S(=O)₂(Q₁), - P(=O)(Q₁)(Q₂), or a group represented by Formula A5: 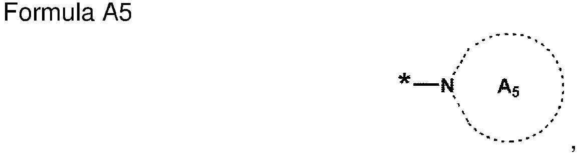
Q₁ to Q₃ are each independently the same as defined in claim 1, and
A₅ in Formula A5 is a substituted or unsubstituted C₁-C₁₀ heterocycloalkyl group, a substituted or unsubstituted C₁-C₁₀ heterocycloalkenyl group, a substituted or unsubstituted C₁-C₆₀ heteroaryl group, or a substituted or unsubstituted monovalent non-aromatic condensed heteropolycyclic group.

6. An organometallic compound according to any one of claims 1 to 5, wherein:
a) R₁₁ and R₁₂ are different from each other, R₁₃ and R₁₄ are different from each other, R₁₁ and R₁₃ are identical to each other, and R₁₂ and R₁₄ are identical to each other;
b) R₁₁ and R₁₃ are different from each other, R₁₂ and R₁₄ are different from each other, R₁₁ and R₁₄ are identical to each other, and R₁₂ and R₁₃ are identical to each other;
c) R₁₁ and R₁₂ are different from each other, and R₁₁, R₁₃, and R₁₄ are identical to each other; or
d) R₁₁ to R₁₄ are all different from each other.

7. An organometallic compound according to claim 1, wherein the organometallic compound is selected from Compounds 1 to 79, 82 to 116, 118 to 169 and 172 to 180: 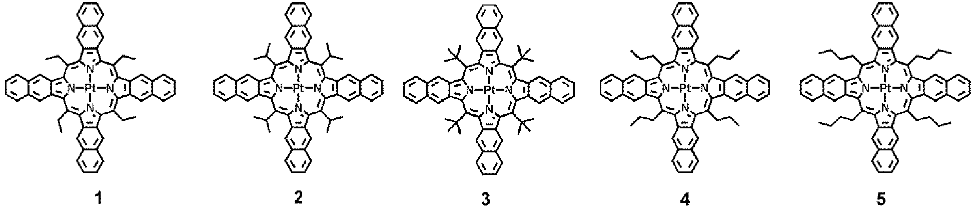 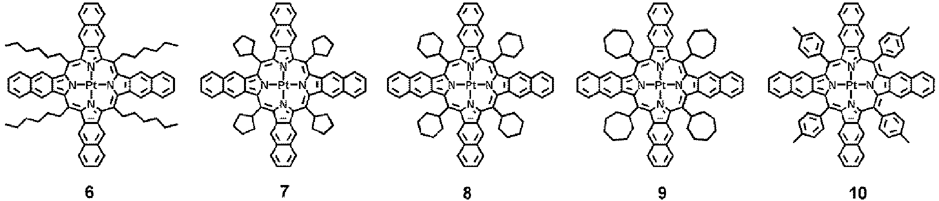 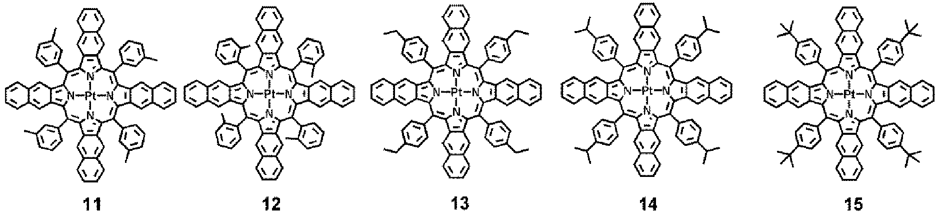 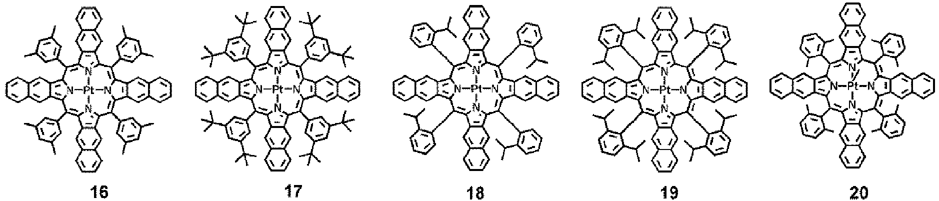 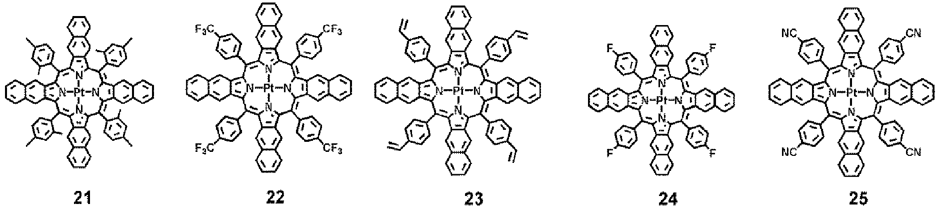 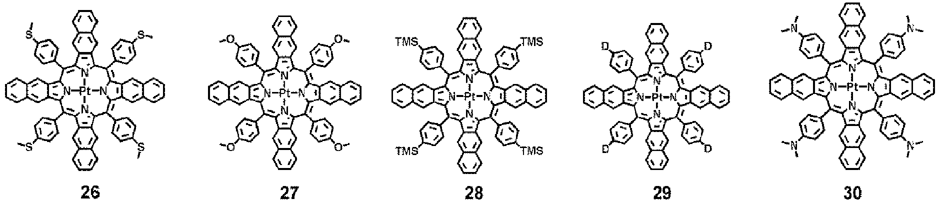 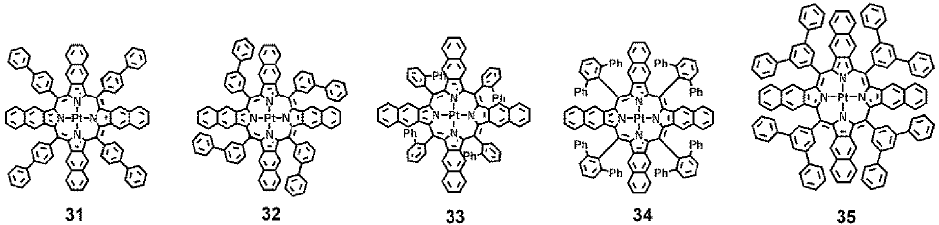 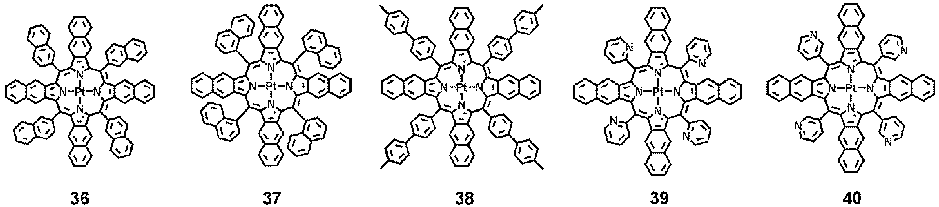 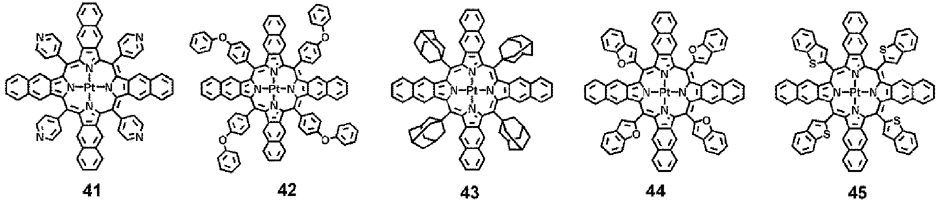 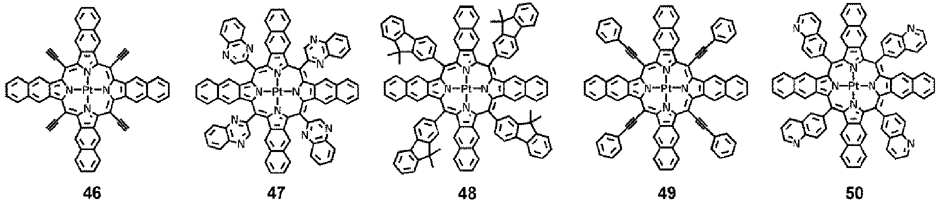 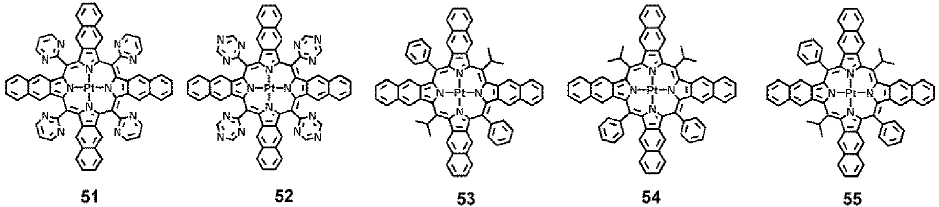 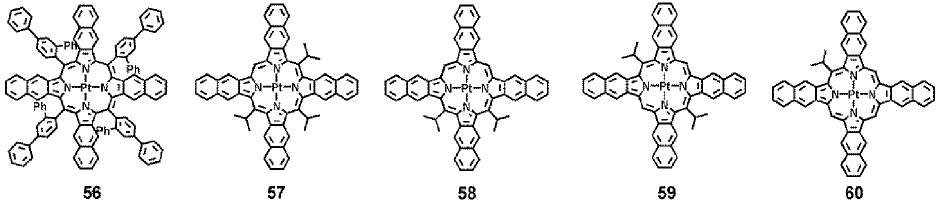 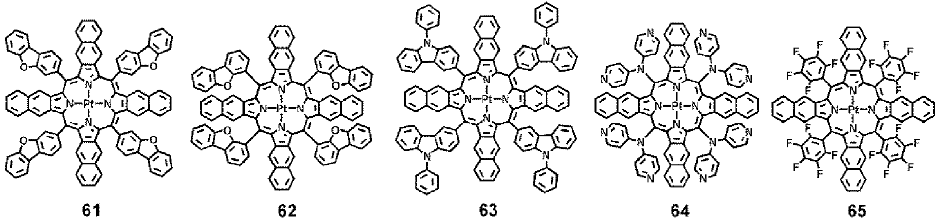 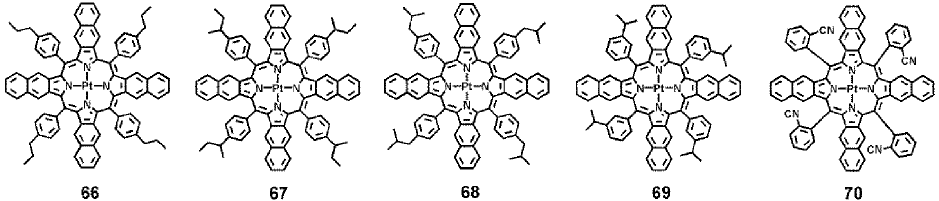 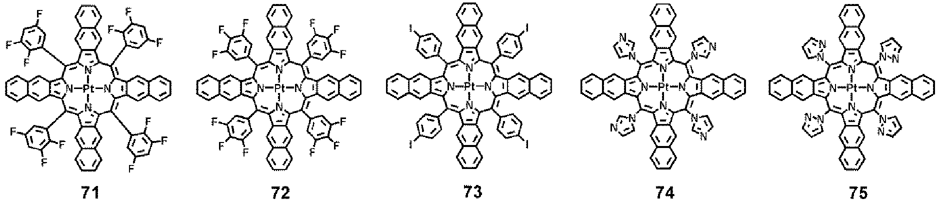 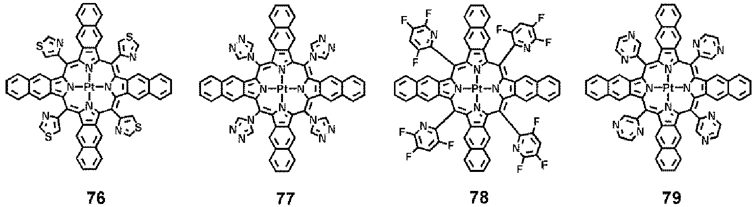 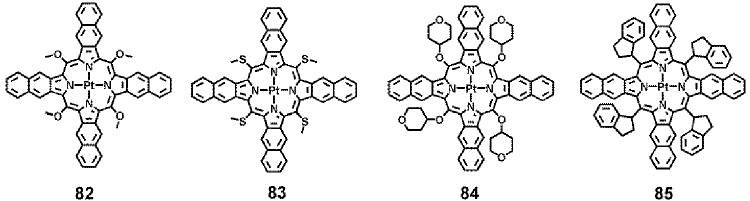 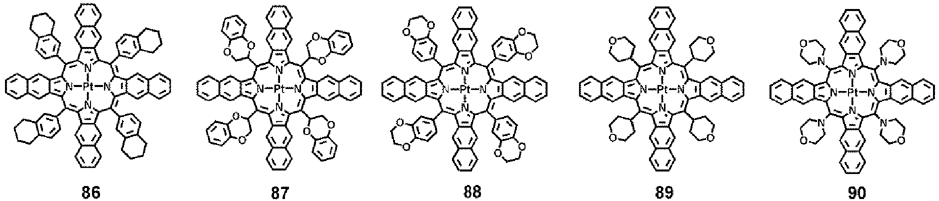 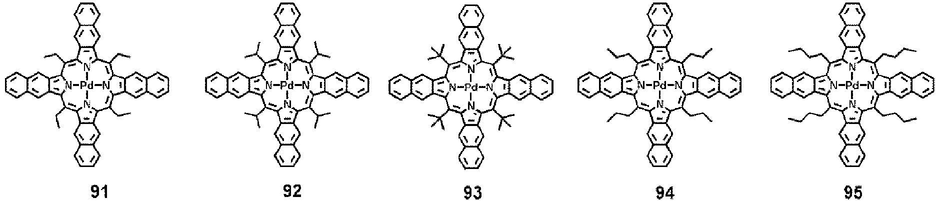 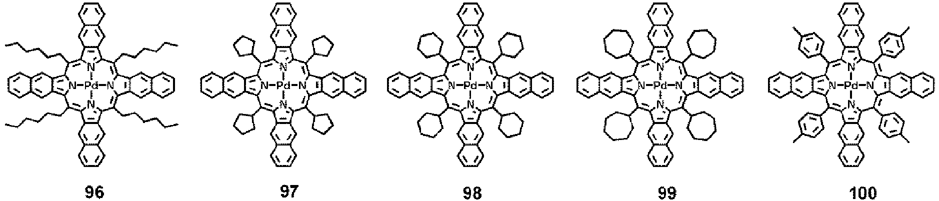 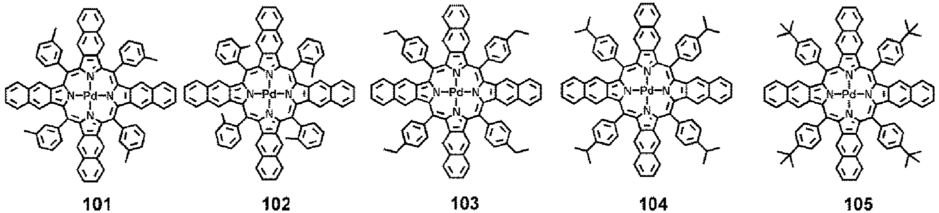 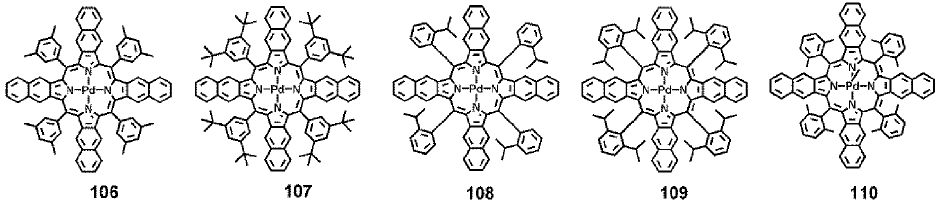 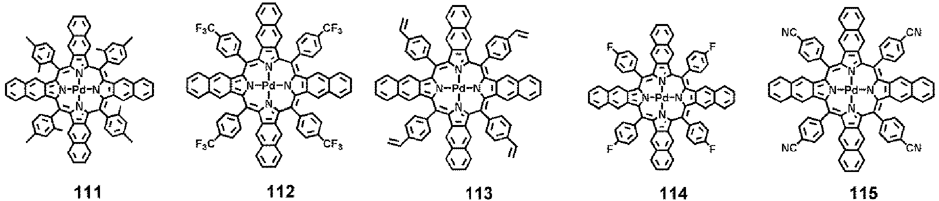 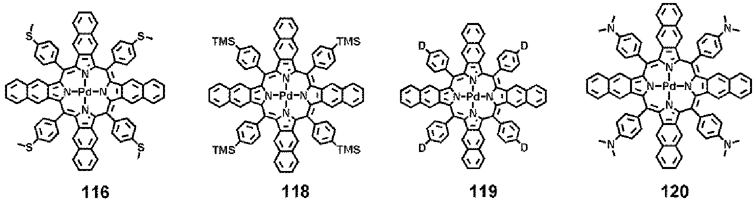 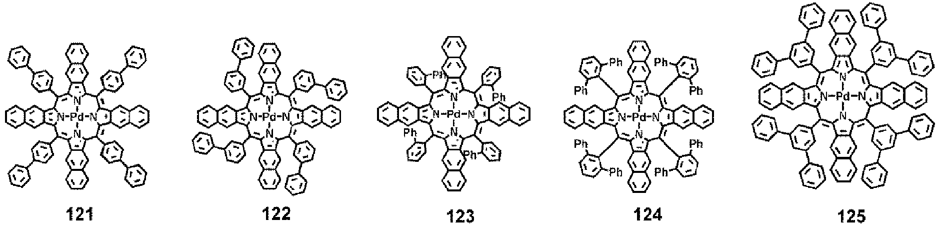 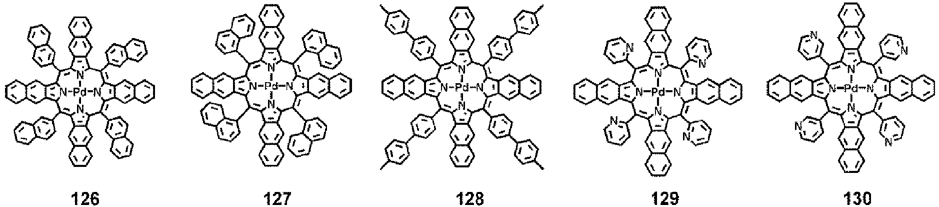 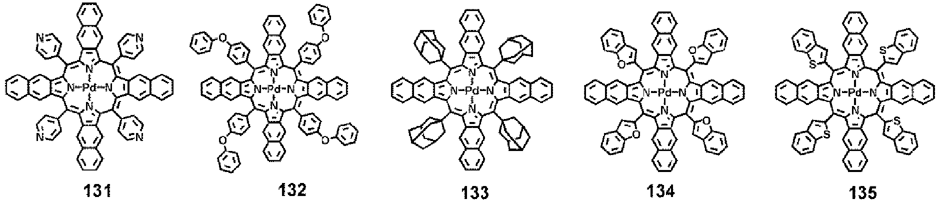 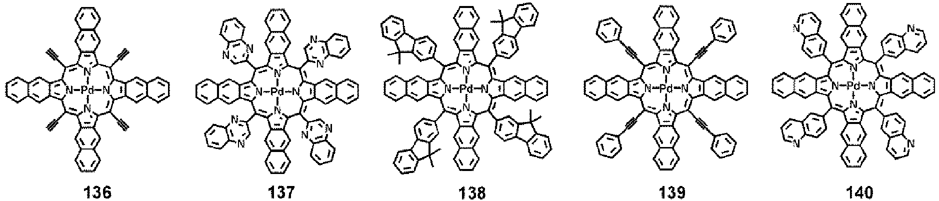 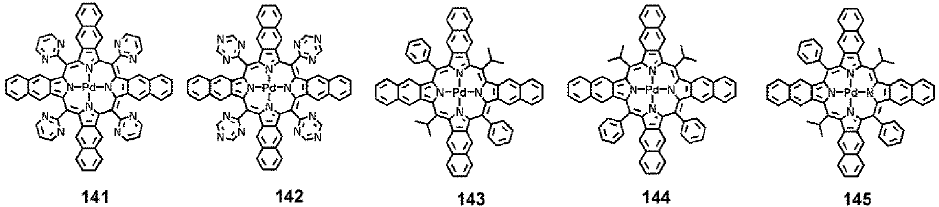 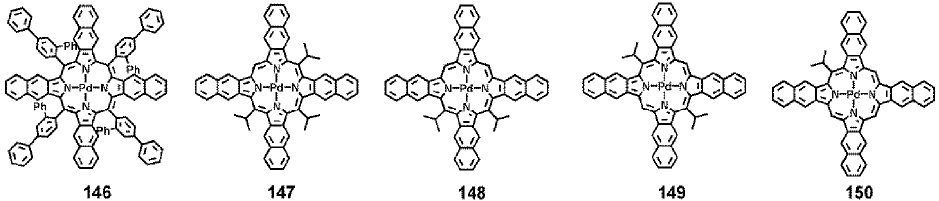 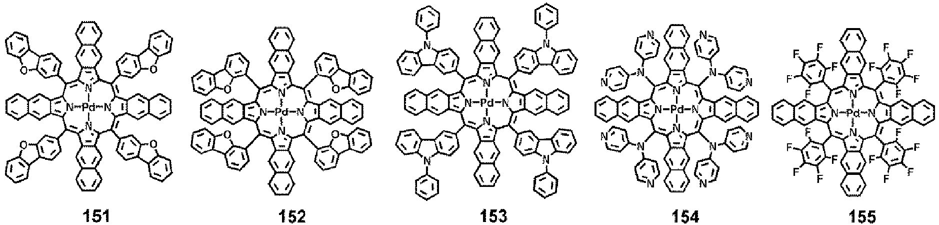 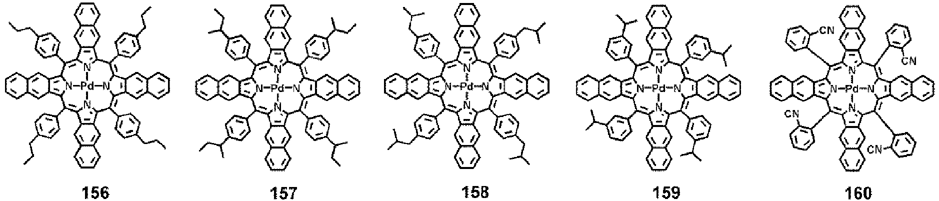 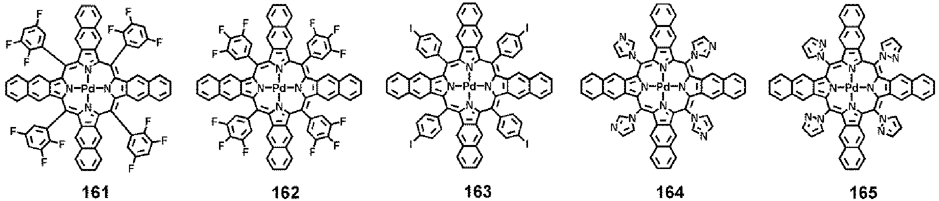 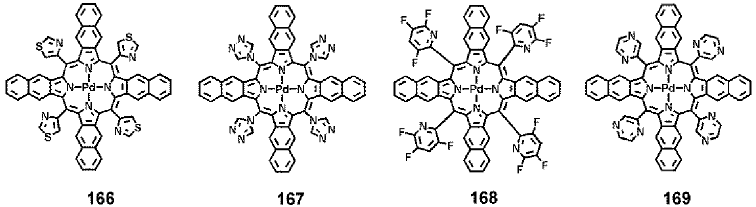 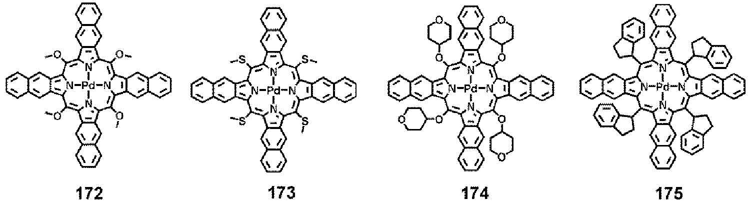 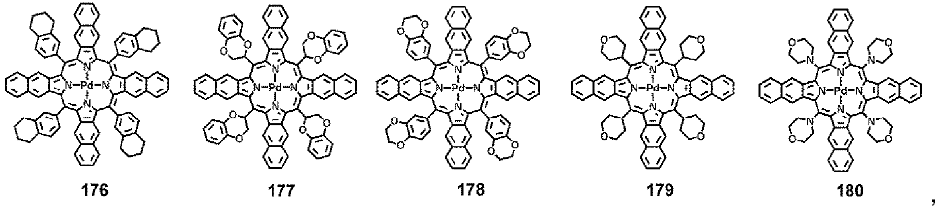 wherein, in Compounds 1 to 79, 82 to 116, 118 to 169 and 172 to 180, TMS indicates a trimethylsilyl group, and Ph indicates a phenyl group.

8. An organic light-emitting device comprising:
a first electrode;
a second electrode facing the first electrode; and
an organic layer between the first electrode and the second electrode,
wherein the organic layer comprises an emission layer, and
the emission layer comprises at least one of the organometallic compound according to any one of claims 1 to 7 and emits near-infrared phosphorescent light having a maximum emission wavelength in a range from 720 nm to 2,500 nm.

9. An organic light-emitting device according to claim 8, wherein:
the first electrode is an anode,
the second electrode is a cathode, and
the organic layer further comprises a hole transport region between the first electrode and the emission layer and an electron transport region between the emission layer and the second electrode,
the hole transport region comprises a hole injection layer, a hole transport layer, an emission auxiliary layer, an electron blocking layer, or any combination thereof, and
the electron transport region comprises a hole blocking layer, an electron transport layer, an electron injection layer, or any combination thereof.

10. An organic light-emitting device according to claim 9, wherein:
at least one selected from the hole transport region and the emission layer comprises an arylamine group-containing compound, an acridine ring-containing compound, and a carbazole ring-containing compound, or
at least one selected from the emission layer and the electron transport region comprises a silicon-containing compound, a phosphine oxide group-containing compound, a sulfur oxide group-containing compound, a triazine ring-containing compound, a pyrimidine ring-containing compound, a pyridine ring-containing compound, a dibenzofuran ring-containing compound, and a dibenzothiophene ring-containing compound.

11. An apparatus comprising the organic light-emitting device according to any one of claims 8 to 10.

12. An apparatus according to claim 11, wherein the apparatus is an electronic device.

13. An apparatus according to claim 11 or claim 12, wherein:
the apparatus further comprises a thin-film transistor,
the thin-film transistor comprises a source electrode and a drain electrode, and
the first electrode of the organic light-emitting device is electrically coupled with one of the source electrode and the drain electrode of the thin-film transistor.

## Patentansprüche

1. Metallorganische Verbindung der Formel 1, die phosphoreszierendes Licht im nahen Infrarotbereich mit einer maximalen Emissionswellenlänge im Bereich von 720 nm bis 2.500 nm emittiert: 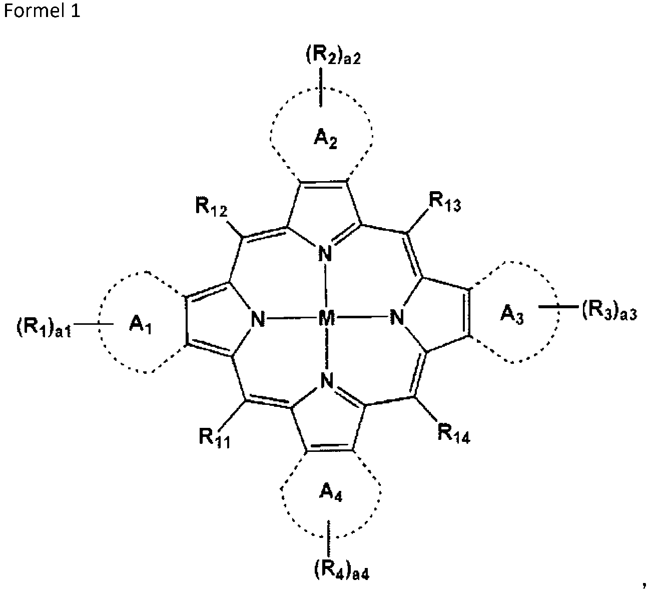 wobei in der Formel 1
M ein Übergangsmetall ist,
Ring A₁ bis Ring A₄ jeweils unabhängig voneinander eine Naphthalingruppe, eine Anthracengruppe, eine Phenanthrengruppe, eine Triphenylengruppe, eine Pyrengruppe, eine Chrysengruppe, eine Cyclopentadiengruppe, eine 1,2,3,4-Tetrahydronaphthalingruppe, eine Thiophengruppe, eine Furangruppe, eine Pyrrolgruppe, eine Indolgruppe, eine Indengruppe, eine Benzosilolgruppe, eine Benzothiophengruppe, eine Benzofurangruppe, eine Carbazolgruppe, eine Fluorengruppe, eine Dibenzosilolgruppe, eine Dibenzothiophengruppe, eine Dibenzofurangruppe, eine Pyridingruppe, eine Pyrimidingruppe, eine Pyrazingruppe, eine Pyridazingruppe, eine Triazingruppe, eine Chinolingruppe, eine Isochinolingruppe, eine Chinazolingruppe, eine Phenanthrolingruppe, eine Pyrazolgruppe, eine Imidazolgruppe, eine Triazolgruppe, eine Oxazolgruppe, eine Isooxazolgruppe, eine Thiazolgruppe, eine Isothiazolgruppe, eine Oxadiazolgruppe, eine Thiadiazolgruppe, eine Benzopyrazolgruppe, eine Benzimidazolgruppe, eine Benzoxazolgruppe, eine Benzothiazolgruppe, eine Benzoxadiazolgruppe, eine Benzothiadiazolgruppe, eine 5,6,7,8-Tetrahydroisochinolingruppe oder eine 5,6,7,8-Tetrahydrochinolingruppe ist,
R₁ bis R₄ und R₁₁ bis R₁₄ jeweils unabhängig voneinander ausgewählt sind aus Wasserstoff, Deuterium, -F, -Cl, -Br, -I, einer Hydroxylgruppe, einer Cyanogruppe, einer Nitrogruppe, einer Amidinogruppe, einer Hydrazinogruppe, einer Hydrazonogruppe, einer substituierten oder unsubstituierten C₁-C₆₀-Alkylgruppe, einer substituierten oder unsubstituierten C₂-C₆₀-Alkenylgruppe, einer substituierten oder unsubstituierten C₂-C₆₀-Alkinylgruppe, einer substituierten oder unsubstituierten C₁-C₆₀-Alkoxygruppe, einer substituierten oder unsubstituierten C₃-C₁₀-Cycloalkylgruppe, einer substituierten oder unsubstituierten C₁-C₁₀-Heterocycloalkylgruppe, einer substituierten oder unsubstituierten C₃-C₁₀-Cycloalkenylgruppe, einer substituierten oder unsubstituierten C₁-C₁₀-Heterocycloalkenylgruppe, einer substituierten oder unsubstituierten C₆-C₆₀-Arylgruppe, einer substituierten oder unsubstituierten C₁-C₆₀-Heteroarylgruppe, einer substituierten oder unsubstituierten monovalenten nicht-aromatischen kondensierten polycyclischen Gruppe, einer substituierten oder unsubstituierten monovalenten nicht-aromatischen kondensierten heteropolycyclischen Gruppe, -O(Q₁), -S(Q₁), -Si(Q₁)(Q₂)(Q₃), -N(Q₁)(Q₂), -B(Q₁)(Q₂), -P(Q₁)(Q₂), -C(=O)(Q₁), -S(=O)₂(Q₁), und -P(=O)(Q₁)(Q₂),
wobei a1 bis a4 jeweils unabhängig voneinander ganze Zahlen von 0 bis 10 sind,
a) R₁₁ bis R₁₄ nicht gleichzeitig Wasserstoff sind, b) R₁₁ bis R₁₄ nicht gleichzeitig eine Phenylgruppe sind, c) R₁₁ bis R₁₄ nicht gleichzeitig eine Mesitylgruppe sind, oder d) R₁₁ bis R₁₄ nicht gleichzeitig eine Methylgruppe sind,
zwei ausgewählt aus R₁₁ bis R₁₄ nicht miteinander verknüpft sind,
mindestens ein Substituent der substituierten C₁-C₆₀-Alkylgruppe, der substituierten C₂-C₆₀-Alkenylgruppe, der substituierten C₂-C₆₀-Alkinylgruppe, der substituierten C₁-C₆₀-Alkoxygruppe, der substituierten C₃-C₁₀-Cycloalkylgruppe, der substituierten C₁-C₁₀-Heterocycloalkylgruppe, der substituierten C₃-C₁₀-Cycloalkenylgruppe, der substituierten C₁-C₁₀-Heterocycloalkenylgruppe, der substituierten C₆-C₆₀-Arylgruppe, der substituierten C₁-C₆₀-Heteroarylgruppe, der substituierten monovalenten nicht-aromatischen kondensierten polycyclischen Gruppe und der substituierten monovalenten nicht-aromatischen kondensierten heteropolycyclischen Gruppe ausgewählt ist aus:
Deuterium, -F, -Cl, -Br, -I, einer Hydroxylgruppe, einer Cyanogruppe, einer Nitrogruppe, einer Amidinogruppe, einer Hydrazinogruppe, einer Hydrazonogruppe, einer C₁-C₆₀-Alkylgruppe, einer C₂-C₆₀-Alkenylgruppe und einer C₂-C₆₀-Alkinylgruppe;
einer C₁-C₆₀-Alkylgruppe, einer C₂-C₆₀-Alkenylgruppe und einer C₂-C₆₀-Alkinylgruppe, jeweils substituiert mit mindestens einem ausgewählt aus: Deuterium, -F, -Cl, -Br, -I, einer Hydroxylgruppe, einer Cyanogruppe, einer Nitrogruppe, einer Amidinogruppe, einer Hydrazinogruppe, einer Hydrazonogruppe, einer C₃-C₁₀-Cycloalkylgruppe, einer C₁-C₁₀Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₁-C₆₀-Heteroarylgruppe, einer monovalenten nicht-aromatischen kondensierten polycyclischen Gruppe, einer monovalenten nicht-aromatischen kondensierten heteropolycyclischen Gruppe, -O(Q₁₁), -S(Q₁₁), -Si(Q₁₁)(Q₁₂)(Q₁₃), -N(Q₁₁)(Q₁₂), -B(Q₁₁)(Q₁₂), -P(Q₁₁)(Q₁₂), - C(=O)(Q₁₁), -S(=O)₂(Q₁₁), und -P(=O)(Q₁₁)(Q₁₂);
einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₁-C₆₀-Heteroarylgruppe, einer monovalenten nicht-aromatischen kondensierten polycyclischen Gruppe und einer monovalenten nicht-aromatischen kondensierten heteropolycyclischen Gruppe;
einer C₃-C₁₀ Cycloalkylgruppe, einer C₁-C₁₀-Heterocycloalkylgruppe, einer C₃-C₁₀-Cycloalkenylgruppe, einer C₁-C₁₀Heterocycloalkenylgruppe, einer C₆-C₆₀-Arylgruppe, einer C₁-C₆₀-Heteroarylgruppe, einer monovalenten nicht-aromatischen kondensierten polycyclischen Gruppe und einer monovalenten nicht-aromatischen kondensierten heteropolycyclischen Gruppe, jeweils substituiert mit mindestens einem Substituenten ausgewählt aus: Deuterium, -F, -Cl, -Br, -I, einer Hydroxylgruppe, einer Cyanogruppe, einer Nitrogruppe, einer Amidinogruppe, einer Hydrazinogruppe, einer Hydrazonogruppe, einer C₁-C₆₀-Alkylgruppe, einer C₂-C₆₀-Alkenylgruppe, einer C₂-C₆₀-Alkinylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₁-C₆₀-Heteroarylgruppe, einer monovalenten nicht-aromatischen kondensierten polycyclischen Gruppe, einer monovalenten nicht-aromatischen kondensierten heteropolycyclischen Gruppe, -O(Q₂₁), -S(Q₂₁), -Si(Q₂₁)(Q₂₂)(Q₂₃), -N(Q₂₁)(Q₂₂), -B(Q₂₁)(Q₂₂), -P(Q₂₁)(Q₂₂), -C(=O)(Q₂₁), -S(=O)₂(Q₂₁), und -P(=O)(Q₂₁)(Q₂₂); und
-O(Q₃₁), -S(Q₃₁), -Si(Q₃₁)(Q₃₂)(Q₃₃), -N(Q₃₁)(Q₃₂), -B(Q₃₁)(Q₃₂), -P(Q₃₁)(Q₃₂), - C(=O)(Q₃₁), -S(=O)₂(Q₃₁), und-P(=O)(Q₃₁)(Q₃₂), und
Q₁ bis Q₃, Q₁₁ to Q₁₃, Q₂₁ bis Q₂₃, und Q₃₁ bis Q₃₃ jeweils unabhängig voneinander ausgewählt sind aus Wasserstoff, Deuterium, -F, -Cl, -Br, -I, einer Hydroxylgruppe, einer Cyanogruppe, einer Nitrogruppe, einer Amidinogruppe, einer Hydrazinogruppe, einer Hydrazonogruppe, einer C₁-C₆₀-Alkylgruppe, einer C₂-C₆₀-Alkenylgruppe, einer C₂-C₆₀-Alkenylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₁-C₆₀-Heteroarylgruppe, einer monovalenten nicht-aromatischen kondensierten polycyclischen Gruppe, einer monovalenten nicht-aromatischen kondensierten heteropolycyclischen Gruppe, einer Biphenylgruppe, einer Terphenylgruppe, einer C₁-C₆₀-Alkylgruppe, die mit mindestens einem substituiert ist, ausgewählt aus: Deuterium, -F, eine Cyanogruppe, einer C₁-C₆₀-Alkylgruppe, einer Phenylgruppe, und einer Biphenylgruppe, und einer C₆-C₆₀-Arylgruppe, die mit mindestens einem Substituenten substituiert ist, ausgewählt aus: Deuterium, -F, eine Cyanogruppe, einer C₁-C₁₀-Alkylgruppe, einer Phenylgruppe und einer Biphenylgruppe,
und die metallorganische Verbindung keine der folgenden Verbindungen ist: 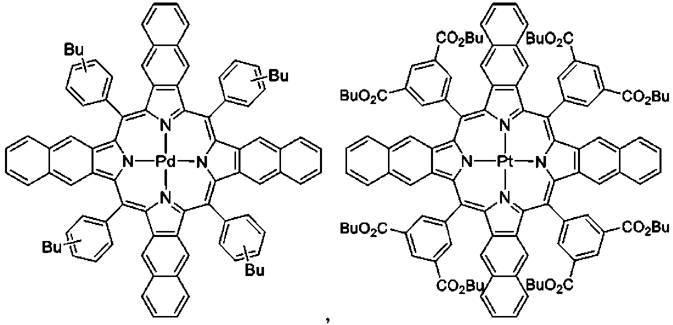
ode

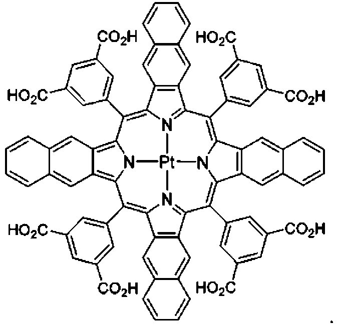

2. Metallorganische Verbindung nach Anspruch 1, wobei M Platin (Pt), Palladium (Pd) oder Gold (Au) ist.

3. Metallorganische Verbindung nach Anspruch 1 oder Anspruch 2,
wobei die Ringe A₁ bis A₄ jeweils unabhängig voneinander aus den durch die Formeln 2-2 bis 2-13 dargestellten Gruppen ausgewählt sind: 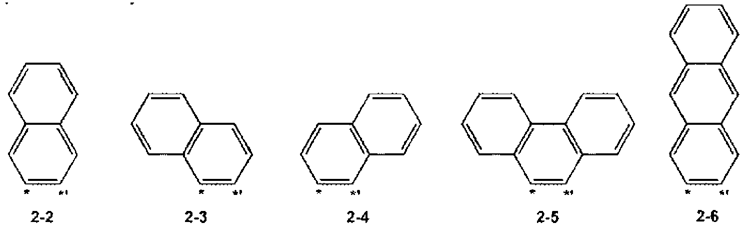 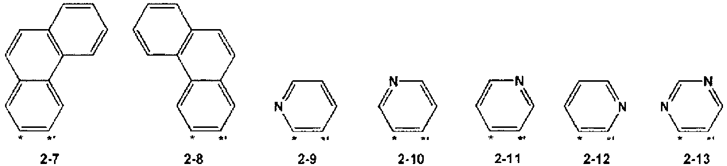
wobei in den Formeln 2-2 bis 2-13, * und *' jeweils eine Position eines Kohlenstoffatoms bezeichnet, das mit einem benachbarten 5-gliedrigen Ring geteilt wird, an den jeweils einer der Ringe A₁ bis A₄ kondensiert ist.

4. Metallorganische Verbindung nach einem der Ansprüche 1 bis 3, wobei:
R₁ bis R₄ und R₁₁ bis R₁₄ jeweils unabhängig voneinander ausgewählt sind aus:
Wasserstoff, Deuterium, -F, -Cl, -Br, -I, einer Hydroxylgruppe, einer Cyanogruppe, einer Nitrogruppe, einer Amidinogruppe, eine Hydrazinogrupper und einer Hydrazonogruppe;
einer C₁-C₁₀ Alkylgruppe, einer C₂-C₁₀-Alkenylgruppe, einer C₂-C₁₀-Alkinylgruppe, und einer C₁-C₁₀-Alkoxygruppe, jeweils unsubstituiert oder substituiert mit mindestens einem ausgewählt aus: Deuterium, -F, -Cl, -Br, -I, -CD₃, -CD₂H, -CDH₂, -CF₃, - CF₂H, -CFH₂, einer Hydroxylgruppe, einer Cyanogruppe, einer Nitrogruppe, einer Amidinogruppe, einer Hydrazinogruppe, einer Hydrazonogruppe, einer Cyclopentylgruppe, einer Cyclohexylgruppe, einer Cycloheptylgruppe, einer Cycloctylgruppe, einer Adamantanylgruppe, einer Norbornanylgruppe, einer Norbornenylgruppe, einer Cyclopentenylgruppe, einer Cyclohexenylgruppe, einer Cycloheptenylgruppe, einer Tetrahydro-2H-pyranylgruppe, einer Morpholinylgruppe, einer Phenylgruppe, einer (C₁-C₁₀-Alkyl)Phenylgruppe, einer Di(C₁-C₁₀-Alkyl)Phenylgruppe, einer Biphenylgruppe, einer Di(phenyl)phenylgruppe, einer Terphenylgruppe, einer Naphthylgruppe, einer Pyridinylgruppe, einer Pyrimidinylgruppe, -O(Q₃₁), -S(Q₃₁), - Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁), -S(=O)₂(Q₃₁), und - P(=O)(Q₃₁)(Q₃₂);
einer Cyclopentylgruppe, einer Cyclohexylgruppe, einer Cycloheptylgruppe, einer Cycloctylgruppe, einer Adamantanylgruppe, einer Norbornanylgruppe, einer Norbornenylgruppe, einer Cyclopentenylgruppe, einer Cyclohexenylgruppe, einer Cycloheptenylgruppe, einer Tetrahydro-2H-pyranylgruppe, einer Morpholinylgruppe, einer Dioxanylgruppe, einer Phenylgruppe, einer (C₁-C₁₀-Alkyl)phenylgruppe, einer Di(C₁-C₁₀-Alkyl)phenylgruppe, einer Biphenylgruppe, einer Di(phenyl)phenylgruppe, einer Terphenylgruppe, einer Naphthylgruppe, einer Fluorenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Chinolinylgruppe, einer Isochinolinylgruppe, einer Benzochinolinylgruppe, einer Chinoxalinylgruppe, einer Chinazolinylgruppe, einer Cinnolinylgruppe, einer Carbazolylgruppe, einer Phenanthrolinylgruppe, einer Benzimidazolylgruppe, einer Benzofuranylgruppe, einer Benzothiophenylgruppe, einer Benzoisothiazolylgruppe, einer Benzoxazolylgruppe, einer Benzoisoxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Benzocarbazolylgruppe, einer Dibenzocarbazolylgruppe, einer Imidazopyridinylgruppe, einer Imidazopyrimidinylgruppe, einer 2,3-Dihydro-1H-indenylgruppe, einer 1,2,3,4-Tetrahydronaphthalenylgruppe, einer 2,3-Dihydrobenzodioxinylgruppe und einer Benzodioxanylgruppe, jeweils unsubstituiert oder substituiert mit mindestens einem Substituenten ausgewählt aus: Deuterium, -F, -Cl, -Br, -I, -CD₃, -CD₂H, -CDH₂, -CF₃, -CF₂H, -CFH₂, einer Hydroxylgruppe, einer Cyanogruppe, einer Nitrogruppe, einer Amidinogruppe, einer Hydrazinogruppe, einer Hydrazonogruppe, einer C₁-C₁₀-Alkylgruppe, einer C₂-C₁₀-Alkenylgruppe, einer C₂-C₁₀-Alkinylgruppe, einer Cyclopentylgruppe, einer Cyclohexylgruppe, einer Cycloheptylgruppe, einer Cycloctylgruppe, einer Adamantanylgruppe, einer Norbornanylgruppe, einer Norbornenylgruppe, einer Cyclopentenylgruppe, einer Cyclohexenylgruppe, einer Cycloheptenylgruppe, einer Tetrahydro-2H-pyranylgruppe, einer Morpholinylgruppe, einer Dioxanylgruppe, einer Phenylgruppe, einer (C₁-C₁₀-Alkyl)phenylgruppe, einer Di(C₁-C₁₀-Alkyl)phenylgruppe, einer Biphenylgruppe, einer Di(phenyl)phenylgruppe, einer Terphenylgruppe, einer Naphthylgruppe, einer Fluorenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Chinolinylgruppe, einer Isochinolinylgruppe, einer Benzochinolinylgruppe, einer Chinoxalinylgruppe, einer Chinazolinylgruppe, einer Cinnolinylgruppe, einer Carbazolylgruppe, einer Phenanthrolinylgruppe, einer Benzimidazolylgruppe, einer Benzofuranylgruppe, einer Benzothiophenylgruppe, einer Benzoisothiazolylgruppe, einer Benzoxazolylgruppe, einer Benzoisoxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Benzocarbazolylgruppe, einer Dibenzocarbazolylgruppe, einer Imidazopyridinylgruppe, einer Imidazopyrimidinylgruppe, einer 2,3-Dihydro-1H-indenylgruppe, einer 1,2,3,4-Tetrahydronaphthalenylgruppe, einer 2,3-Dihydrobenzodioxinylgruppe, einer Benzodioxanylgruppe, -O(Q₃₁), S(Q₃₁), - Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁), -S(=O)₂(Q₃₁), und - P(=O)(Q₃₁)(Q₃₂); und
-O(Q₁), -S(Q₁), -Si(Q₁)(Q₂)(Q₃), -N(Q₁)(Q₂), -B(Q₁)(Q₂), -P(Q₁)(Q₂), -C(=O)(Q₁), - S(=O)₂(Q₁), und -P(=O)(Q₁)(Q₂), und
Q₁ bis Q₃ und Q₃₁ bis Q₃₃ jeweils unabhängig voneinander ausgewählt sind aus Wasserstoff, Deuterium, -F, -Cl, -Br, -I, -CD₃, -CD₂H, -CDH₂, -CF₃, -CF₂H, -CFH₂, einer Hydroxylgruppe, einer Cyanogruppe, einer Nitrogruppe, einer Amidinogruppe, einer Hydrazinogruppe, einer Hydrazonogruppe, einer C₁-C₁₀ Alkylgruppe, einer C₂-C₁₀ Alkenylgruppe, einer C₂-C₁₀ Alkinylgruppe, einer C₁-C₁₀ Alkoxygruppe, einer Cyclopentylgruppe, einer Cyclohexylgruppe, einer Cycloheptylgruppe, einer Cycloctylgruppe, einer Adamantanylgruppe, einer Norbornanylgruppe, einer Norbornenylgruppe, einer Cyclopentenylgruppe, einer Cyclohexenylgruppe, einer Cycloheptenylgruppe, einer Tetrahydro-2H-pyranylgruppe, einer Morpholinylgruppe, einer Dioxanylgruppe, einer Phenylgruppe, einer (C₁-C₁₀-Alkyl)phenylgruppe, einer Di(C₁-C₁₀-Alkyl)phenylgruppe, einer Biphenylgruppe, einer Di(phenyl)phenylgruppe, einer Terphenylgruppe, einer Naphthylgruppe, einer Fluorenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Chinolinylgruppe, einer Isochinolinylgruppe, einer Benzochinolinylgruppe, einer Chinoxalinylgruppe, einer Chinazolinylgruppe, einer Cinnolinylgruppe, einer Carbazolylgruppe, einer Phenanthrolinylgruppe, einer Benzimidazolylgruppe, einer Benzofuranylgruppe, einer Benzothiophenylgruppe, einer Benzoisothiazolylgruppe, einer Benzoxazolylgruppe, einer Benzoisoxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Benzocarbazolylgruppe, einer Dibenzocarbazolylgruppe, einer Imidazopyridinylgruppe, einer Imidazopyrimidinylgruppe, einer 2,3-Dihydro-1H-indenylgruppe Gruppe, einer 1,2,3,4-Tetrahydronaphthalenylgruppe, einer 2,3-Dihydrobenzodioxinylgruppe und vorzugsweise einer Benzodioxanylgruppe,
wobei:
R₁ bis R₄ jeweils unabhängig voneinander ausgewählt sind aus Wasserstoff, Deuterium, -F, -Cl, -Br, -I, -CD₃, -CD₂H, -CDH₂, -CF₃, -CF₂H, -CFH₂, C₁-C₁₀-Alkylgruppe, einer C₂-C₁₀-Alkenylgruppe, einer C₂-C₁₀-Alkinylgruppe, einer C₁-C₁₀-Alkoxygruppe, einer Cyclopentylgruppe, einer Cyclohexylgruppe, einer Cycloheptylgruppe, einer Phenylgruppe, einer (C₁-C₁₀-Alkyl)phenylgruppe, einer Di(C₁-C₁₀-Alkyl)phenylgruppe, einer Biphenylgruppe, einer Di(phenyl)phenylgruppe, einer Terphenylgruppe, -O(Q₁) und - S(Q₁), und
Q₁ ausgewählt ist aus einer C₁-C₁₀-Alkylgruppe, einer C₂-C₁₀-Alkenylgruppe, einer C₂-C₁₀-Alkinylgruppe, einer C₁-C₁₀-Alkoxygruppe, einer Cyclopentylgruppe, einer Cyclohexylgruppe, einer Cycloheptylgruppe, einer Phenylgruppe, einer (C₁-C₁₀-Alkyl)phenylgruppe, einer Di(C₁-C₁₀-Alkyl)phenylgruppe, einer Biphenylgruppe, einer Di(phenyl)phenylgruppe und einer Terphenylgruppe.

5. Metallorganische Verbindung nach einem der Ansprüche 1 bis 4, wobei:
jede der mindestens einen ausgewählt aus R₁₁ bis R₁₄ unabhängig voneinander ausgewählt ist aus einer C₂-C₁₀-Alkylgruppe, einer C₂-C₁₀-Alkenylgruppe, einer C₂-C₁₀-Alkinylgruppe und einer C₁-C₁₀-Alkoxygruppe, jeweils unsubstituiert oder substituiert mit mindestens einer ausgewählt aus: Deuterium, -F, -Cl, -Br, -I, -CD₃, -CD₂H, -CDH₂, -CF₃, - CF₂H, -CFH₂, einer Hydroxylgruppe, einer Cyanogruppe, einer Nitrogruppe, einer Amidinogruppe, einer Hydrazinogruppe, einer Hydrazonogruppe, einer Cyclopentylgruppe, einer Cyclohexylgruppe, einer Cycloheptylgruppe, einer Cycloctylgruppe, einer Adamantanylgruppe, einer Norbornanylgruppe, einer Norbornenylgruppe, einer Cyclopentenylgruppe, einer Cyclohexenylgruppe, einer Cycloheptenylgruppe, einer Tetrahydro-2H-pyranylgruppe, einer Morpholinylgruppe, einer Phenylgruppe, einer (C₁-C₁₀-Alkyl)phenylgruppe, einer Di(C₁-C₁₀-Alkyl)phenylgruppe, einer Biphenylgruppe, einer Di(phenyl)phenylgruppe, einer Terphenylgruppe, einer Naphthylgruppe, einer Pyridinylgruppe, einer Pyrimidinylgruppe, -O(Q₃₁), -S(Q₃₁), - Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁), -S(=O)₂(Q₃₁), und - P(=O)(Q₃₁)(Q₃₂), vorzugsweise wobei:
mindestens eine ausgewählt aus R₁₁ bis R₁₄ keine Wasserstoff, Phenylgruppe, Mesitylgruppe und Methylgruppe ist; und/oder
jede von mindestens einer ausgewählt aus R₁₁ bis R₁₄ unabhängig -O(Q₁), -S(Q₁), - Si(Q₁)(Q₂)(Q₃), -N(Q₁)(Q₂), -B(Q₁)(Q₂), -P(Q₁)(Q₂), -C(=O)(Q₁), -S(=O)₂(Q₁), -P(=O)(Q₁)(Q₂) ist, oder eine durch Formel A5 dargestellte Gruppe: 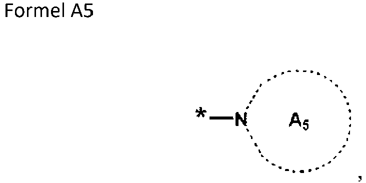
Q₁ bis Q₃ jeweils unabhängig voneinander gleich wie in Anspruch 1 definiert sind, und
A₅ in Formel A5 eine substituierte oder unsubstituierte C₁-C₁₀-Heterocycloalkylgruppe, eine substituierte oder unsubstituierte C₁-C₁₀-Heterocycloalkenylgruppe, eine substituierte oder unsubstituierte C₁-C₆₀-Heteroarylgruppe oder eine substituierte oder unsubstituierte monovalente nichtaromatische kondensierte heteropolycyclische Gruppe ist.

6. Metallorganische Verbindung nach einem der Ansprüche 1 bis 5, wobei:
a) R₁₁ und R₁₂ voneinander verschieden sind, R₁₃ und R₁₄ voneinander verschieden sind, R₁₁ und R₁₃ identisch miteinander sind, und R₁₂ und R₁₄ identisch miteinander sind;
b) R₁₁ und R₁₃ voneinander verschieden sind, R₁₂ und R₁₄ voneinander verschieden sind, R₁₁ und R₁₄ identisch miteinander sind, und R₁₂ und R₁₃ identisch miteinander sind;
c) R₁₁ und R₁₂ voneinander verschieden sind, und R₁₁, R₁₃und R₁₄ identisch sind; oder
d) R₁₁ bis R₁₄ alle voneinander verschieden sind.

7. Metallorganische Verbindung nach Anspruch 1, wobei die metallorganische Verbindung ausgewählt ist aus den Verbindungen 1 bis 79, 82 bis 116, 118 bis 169 und 172 bis 180: 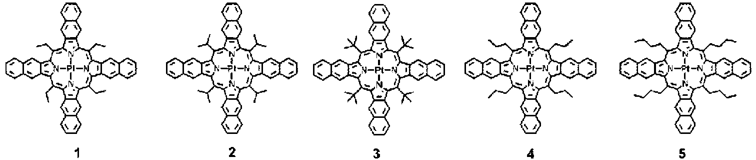 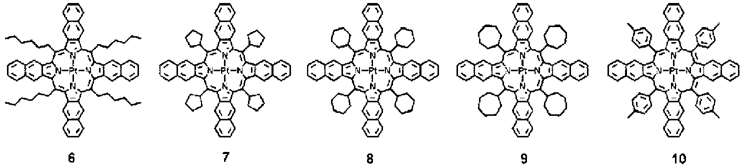 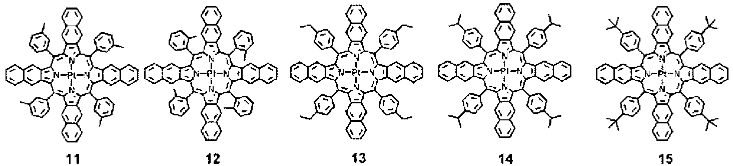 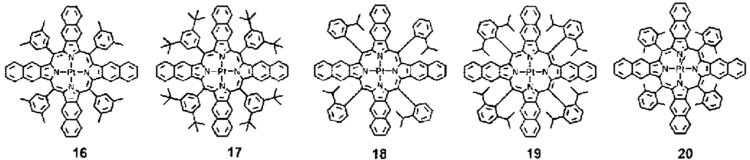 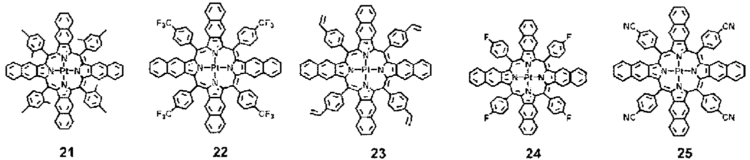 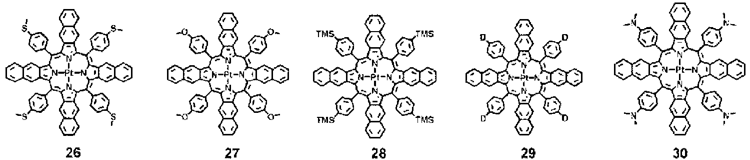 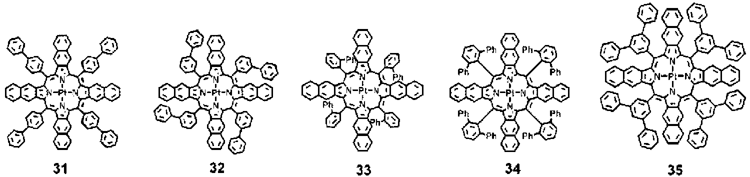 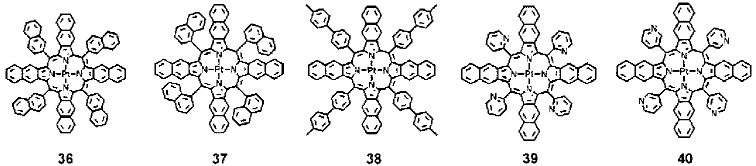 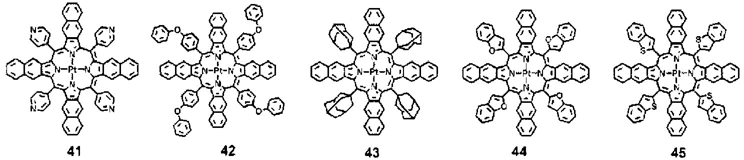 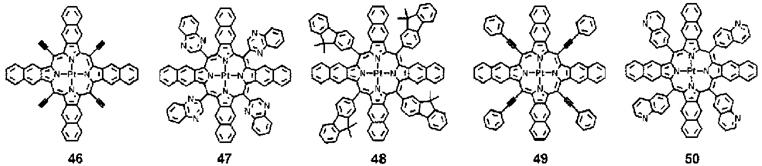 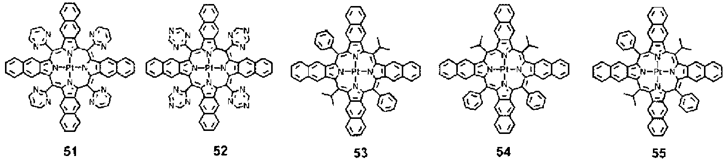 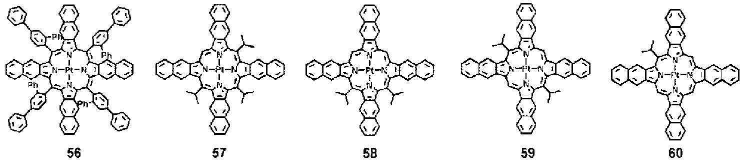 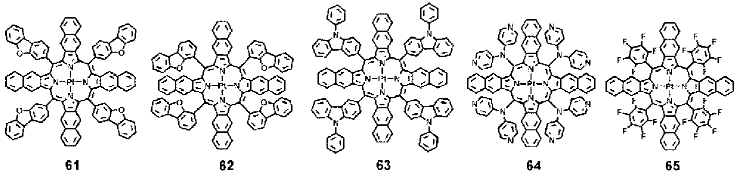 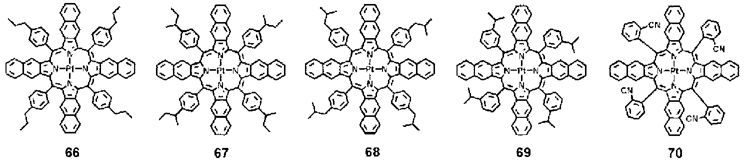 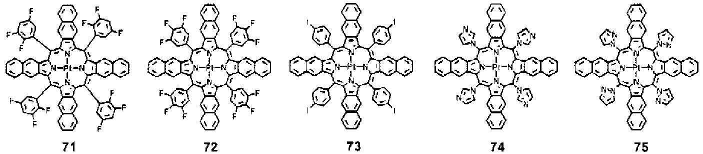 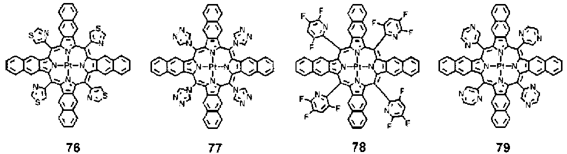 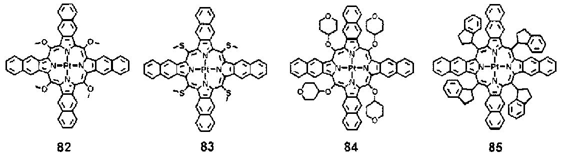 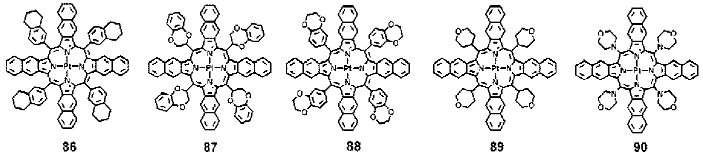 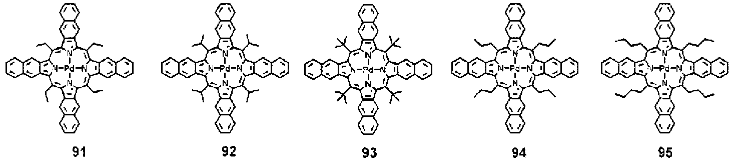 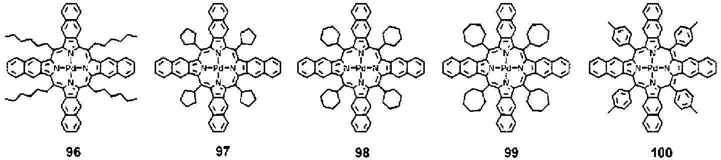 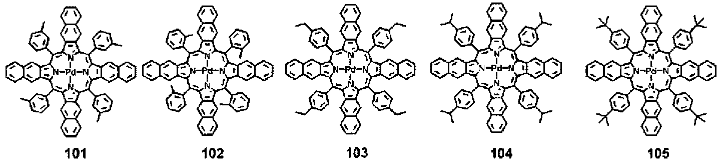 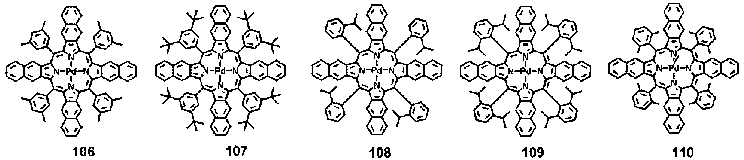 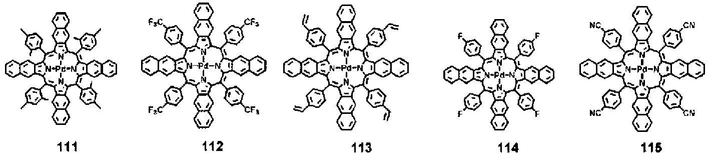 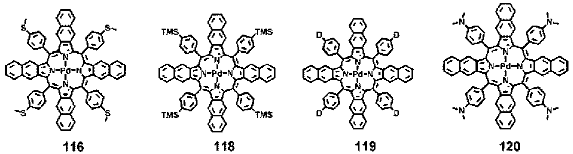 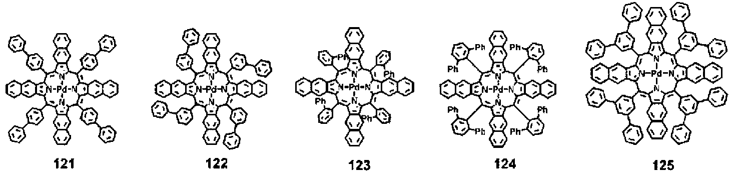 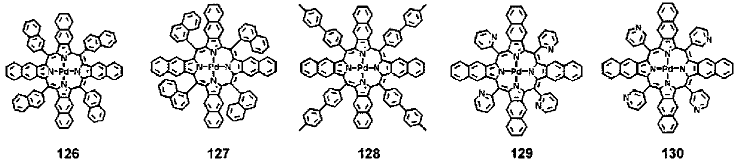 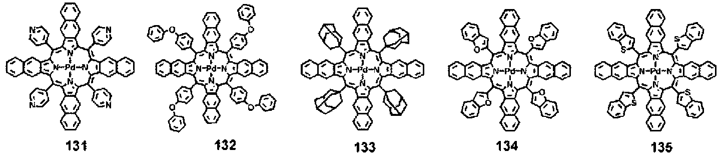 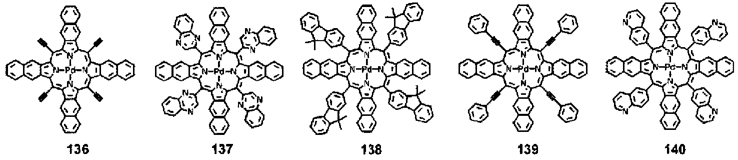 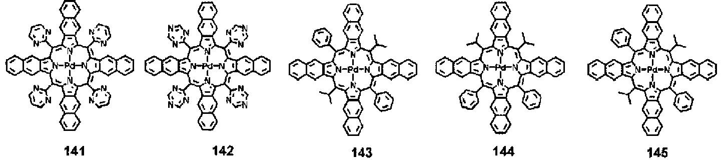 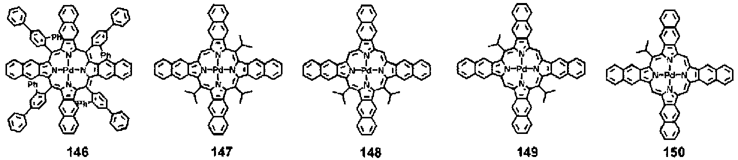 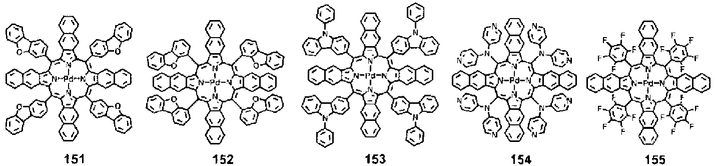 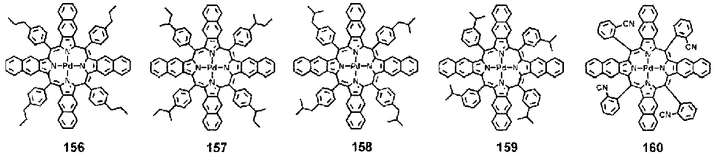 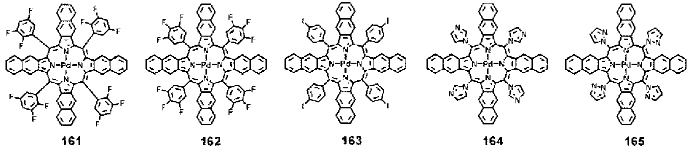 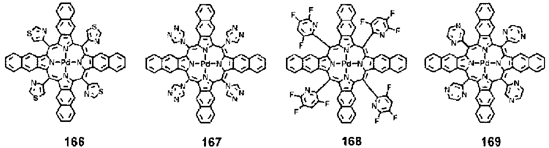 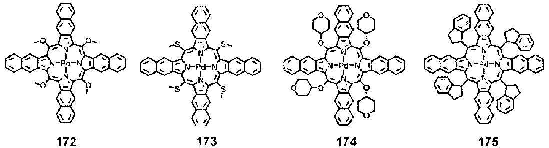 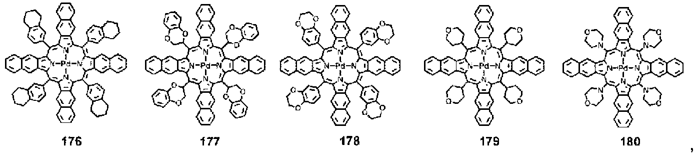 wobei in den Verbindungen 1 bis 79, 82 bis 116, 118 bis 169 und 172 bis 180 TMS eine Trimethylsilylgruppe und Ph eine Phenylgruppe bezeichnet.

8. Organische Licht-emittierende Vorrichtung, umfassend:
eine erste Elektrode;
eine zweite Elektrode, die der ersten Elektrode zugewandt ist; und
eine organische Schicht zwischen der ersten und der zweiten Elektrode,
wobei die organische Schicht eine Emissionsschicht umfasst,
die Emissionsschicht mindestens eine der metallorganischen Verbindungen nach einem der Ansprüche 1 bis 7 umfasst und phosphoreszierendes Nahinfrarotlicht mit einer maximalen Emissionswellenlänge im Bereich von 720 nm bis 2.500 nm emittiert.

9. Organische Licht-emittierende Vorrichtung nach Anspruch 8, wobei:
die erste Elektrode eine Anode ist,
die zweite Elektrode eine Kathode ist, und
die organische Schicht weiter eine Lochtransportregion zwischen der ersten Elektrode und der Emissionsschicht sowie eine Elektronentransportregion zwischen der Emissionsschicht und der zweiten Elektrode umfasst,
die Lochtransportregion eine Lochinjektionsschicht, eine Lochtransportschicht, eine Emissionshilfsschicht, eine Elektronenblockierschicht oder eine beliebige Kombination davon umfasst und
die Elektronentransportregion eine Lochblockierschicht, eine Elektronentransportschicht, eine Elektroneninjektionsschicht oder eine beliebige Kombination davon umfasst.

10. Organische Licht-emittierende Vorrichtung nach Anspruch 9, wobei:
mindestens eine ausgewählt aus der Lochtransportregion und der Emissionsschicht eine Arylamingruppe enthaltende Verbindung, eine Acridinring enthaltende Verbindung und eine Carbazolring enthaltende Verbindung umfasst, oder
mindestens eine ausgewählt aus der Emissionsschicht und der Elektronentransportregion eine siliziumhaltige Verbindung, eine phosphinoxidgruppenhaltige Verbindung, eine schwefeloxidgruppenhaltige Verbindung, eine triazinringhaltige Verbindung, eine pyrimidinringhaltige Verbindung, eine pyridinringhaltige Verbindung, eine dibenzofuranringhaltige Verbindung und eine dibenzothiophenringhaltige Verbindung umfasst.

11. Einrichtung, umfassend die organische Licht-emittierende Vorrichtung nach einem der Ansprüche 8 bis 10.

12. Einrichtung nach Anspruch 11, wobei es sich bei der Einrichtung um eine elektronische Vorrichtung handelt.

13. Einrichtung nach Anspruch 11 oder Anspruch 12, wobei:
die Einrichtung weiter einen Dünnschichttransistor umfasst,
der Dünnschichttransistor eine Source-Elektrode und eine Drain-Elektrode umfasst, und
die erste Elektrode der organischen Licht-emittierende Vorrichtung elektrisch mit einer der Source-Elektroden und der Drain-Elektrode des Dünnschichttransistors gekoppelt ist.

## Revendications

1. Composé organométallique représenté par la formule 1 et émettant une lumière phosphorescente proche infrarouge présentant une longueur d'onde d'émission maximale comprise entre 720 nm et 2500 nm : 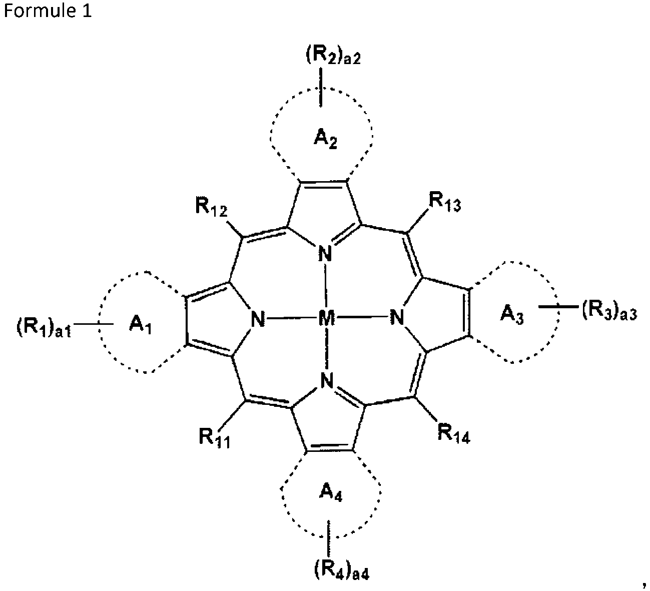 dans lequel, dans la Formule 1,
M est un métal de transition,
les cycles A₁ à A₄ sont chacun indépendamment un groupe naphtalène, un groupe anthracène, un groupe phénanthrène, un groupe triphénylène, un groupe pyrène, un groupe chrysène, un groupe cyclopentadiène, un groupe 1,2,3,4-tétrahydronaphtalène, un groupe thiophène, un groupe furane, un groupe pyrrole, un groupe indole, un groupe indène, un groupe benzosilole, un groupe benzothiophène, un groupe benzofurane, un groupe carbazole, un groupe fluorène, un groupe dibenzosilole, un groupe dibenzothiophène, un groupe dibenzofurane, un groupe pyridine, un groupe pyrimidine, un groupe pyrazine, un groupe pyridazine, un groupe triazine, un groupe quinoléine, un groupe isoquinoléine, un groupe quinazoline, un groupe phénanthroline, un groupe pyrazole, un groupe imidazole, un groupe triazole, un groupe oxazole, un groupe isooxazole, un groupe thiazole, un groupe isothiazole, un groupe oxadiazole, un groupe thiadiazole, un groupe benzopyrazole, un groupe benzimidazole, un groupe benzoxazole, un groupe benzothiazole, un groupe benzoxadiazole, un groupe benzothiadiazole, un groupe 5,6,7,8-tétrahydroisoquinoléine ou un groupe 5,6,7,8-tétrahydroquinoléine,
R₁ à R₄ et R₁₁ à R₁₄ sont chacun indépendamment choisis parmi l'hydrogène, le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₆₀ substitué ou non substitué, un groupe alcényle en C₂-C₆₀ substitué ou non substitué, un groupe alcynyle en C₂-C₆₀ substitué ou non substitué, un groupe alcoxy en C₁-C₆₀ substitué ou non substitué, un groupe cycloalkyle en C_{C3}-C₁₀ substitué ou non substitué, un groupe hétérocycloalkyle en C₁-C₁₀ substitué ou non substitué, un groupe cycloalcényle en C₃-C_{C10} substitué ou non substitué, un groupe hétérocycloalcényle en C₁-C₁₀ substitué ou non substitué, un groupe aryle en C₆-C₆₀ substitué ou non substitué, un groupe hétéroaryle en C₁-C₆₀ substitué ou non substitué, un groupe polycyclique condensé monovalent non aromatique substitué ou non substitué, un groupe hétéropolycyclique condensé monovalent non aromatique substitué ou non substitué, -O(Qi), -S(Q1), -Si(Q₁)(Q₂)(Q₃), - N(Q₁)(Q₂), -B(Q₁)(Q₂), -P(Q₁)(Q₂), -C(=O)(Q₁), -S(=O)₂(Q₁), et -P(=O)(Q₁)(Q₂),
a1 à a4 sont chacun indépendamment un entier compris entre 0 et 10,
a) R₁₁ à R₁₄ ne sont pas simultanément des atomes d'hydrogène, b) R₁₁ à R₁₄ ne sont pas simultanément un groupe phényle, c) R₁₁ à R₁₄ ne sont pas simultanément un groupe mésityle, ou d) R₁₁ à R₁₄ ne sont pas simultanément un groupe méthyle,
deux choisis parmi R₁₁ à R₁₄ ne sont pas liés entre eux,
au moins un substituant du groupe alkyle substitué en C₁-C₆₀, du groupe alcényle substitué en C₂-C₆₀, du groupe alcynyle substitué en C₂-C₆₀, du groupe alcoxy substitué en C₁-C₆₀, du groupe cycloalkyle substitué en C₃-C₁₀, du groupe hétérocycloalkyle substitué en C₁-C₁₀, du groupe cycloalcényle substitué en C₃-C₁₀, du groupe hétérocycloalcényle substitué en C₁-C₁₀, du groupe aryle substitué en C₆-C₆₀, du groupe hétéroaryle substitué en C₁-C₆₀, du groupe polycyclique condensé monovalent non aromatique substitué et du groupe hétéropolycyclique condensé monovalent non aromatique substitué est choisi parmi :
le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₆₀, un groupe alcényle en C₂-C₆₀ et un groupe alcynyle en C₂-C₆₀ ;
un groupe alkyle en C₁-C₆₀, un groupe alcényle en C₂-C₆₀ et un groupe alcynyle en C₂-C₆₀, chacun substitué par au moins un groupe choisi parmi le deutérium, -F, -Cl, -Br, -I, un groupe hydroxyle, un groupe cyano, un groupe nitro, un groupe amidino, un groupe hydrazino, un groupe hydrazono, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe hétéroaryle en C₁-C₆₀, un groupe polycyclique condensé non aromatique monovalent, un groupe hétéropolycyclique condensé non aromatique monovalent, -O(Q₁₁), -S(Q₁₁), - Si(Q₁₁)(Q₁₂)(Q₁₃), -N(Q₁₁)(Q₁₂), -B(Q₁₁)(Q₁₂), -P(Q₁₁)(Q₁₂), -C(=O)(Q₁₁), -S(=O)₂(Q₁₁), and - P(=O)(Q₁₁)(Q₁₂) ;
un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe hétéroaryle en C₁-C₆₀, un groupe polycyclique condensé monovalent non aromatique et un groupe hétéropolycyclique condensé monovalent non aromatique ;
un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe hétéroaryle en C₁-C₆₀, un groupe polycyclique condensé monovalent non aromatique et un groupe hétéropolycyclique condensé monovalent non aromatique, chacun substitué par au moins un groupe choisi parmi le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₆₀, un groupe alcényle en C₂-C₆₀, un groupe alcynyle en C₂-C₆₀, un groupe alcoxy en C₁-C₆₀, un groupe cycloalkyle en C₃-C_{C10}, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe hétéroaryle en C₁-C₆₀, un groupe polycyclique condensé monovalent non aromatique, un groupe hétéropolycyclique condensé monovalent non aromatique, -O(Q₂₁), -S(Q₂₁), - Si(Q₂₁)(Q₂₂)(Q₂₃), -N(Q₂₁)(Q₂₂), -B(Q₂₁)(Q₂₂), -P(Q₂₁)(Q₂₂), -C(=O)(Q₂₁), -S(=O)₂(Q₂₁), et - P(=O)(Q₂₁)(Q₂₂) ; et
-O(Q₃₁), -S(Q₃₁), -Si(Q₃₁)(Q₃₂)(Q₃₃), -N(Q₃₁)(Q₃₂), -B(Q₃₁)(Q₃₂), -P(Q₃₁)(Q₃₂), - C(=O)(Q₃₁), -S(=O)₂(Q₃₁), et -P(=O)(Q₃₁)(Q₃₂), et
Q₁ à Q₃, Q₁₁ à Q₁₃, Q₂₁ à Q₂₃, et Q₃₁ à Q₃₃ sont chacun choisis indépendamment parmi l'hydrogène, le deutérium, -F, -Cl, -Br, -I, un groupe hydroxyle, un groupe cyano, un groupe nitro, un groupe amidino, un groupe hydrazino, un groupe hydrazono, un groupe alkyle en C₁-C₆₀, un groupe alcényle en C₂-C₆₀, un groupe alcynyle en C₂-C₆₀, un groupe alcoxy en C₁-C₆₀, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe hétéroaryle en C₁-C₆₀, un groupe polycyclique condensé non aromatique monovalent, un groupe hétéropolycyclique condensé non aromatique monovalent, un groupe biphényle, un groupe terphényle, un groupe alkyle en C₁-C₆₀ substitué par au moins un élément choisi parmi le deutérium, le fluor, un groupe cyano, un groupe alkyle en C₁-C₆₀, un groupe phényle et un groupe biphényle, et un groupe aryle en C₆-C₆₀ substitué par au moins un élément choisi parmi le deutérium, le fluor, un groupe cyano, un groupe alkyle en C₁-C₁₀, un groupe phényle et un groupe biphényle,
et le composé organométallique n'est aucun des composés suivants : 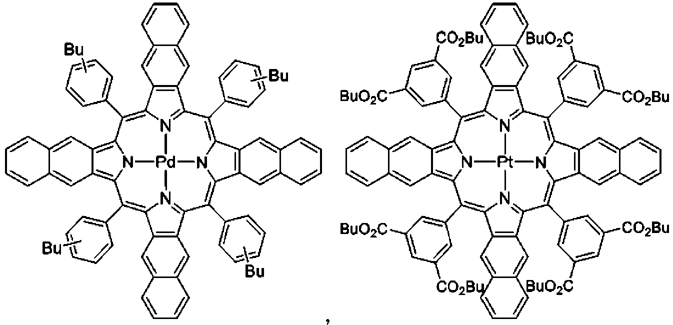
ou

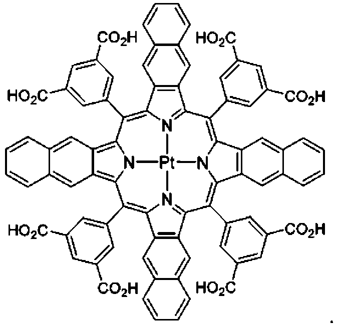

2. Composé organométallique selon la revendication 1, dans lequel M est du platine (Pt), du palladium (Pd) ou de l'or (Au).

3. Composé organométallique selon la revendication 1 ou la revendication 2,
dans lequel les cycles A₁ à A₄ sont chacun choisis indépendamment parmi les groupes représentés par les formules 2-2 à 2-13 : 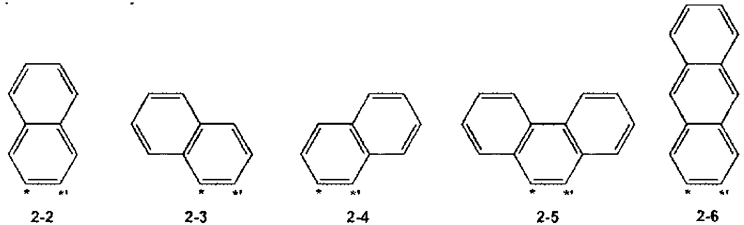 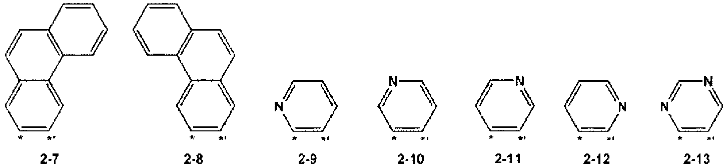
dans lequel, dans les formules 2-2 à 2-13, * et *' indiquent chacun une position d'un atome de carbone partagé avec un cycle voisin à 5 membres auquel chacun des cycles A₁ à A₄ est condensé, respectivement.

4. Composé organométallique selon l'une quelconque des revendications 1 à 3, dans lequel :
R₁ à R₄ et R₁₁ à R₁₄ sont chacun choisis indépendamment parmi :
l'hydrogène, le deutérium, -F, -Cl, -Br, -I, un groupe hydroxyle, un groupe cyano, un groupe nitro, un groupe amidino, un groupe hydrazino et un groupe hydrazono ;
un groupe alkyle en C₁-C₁₀, un groupe alcényle en C₂-C₁₀, un groupe alcynyle en C₂-C₁₀ et un groupe alcoxy en C₁-C₁₀, chacun non substitué ou substitué par au moins un groupe choisi parmi le deutérium, -F, -Cl, -Br, -I, -CD₃, -CD₂H, CDH₂, -CF₃, -CF₂H, -CFH₂, un groupe hydroxyle, un groupe cyano, un groupe nitro, un groupe amidino, un groupe hydrazino, un groupe hydrazono, un groupe cyclopentyle, un groupe cyclohexyle, un groupe cycloheptyle, un groupe cycloctyle, un groupe adamantyle, un groupe norbornanyle, un groupe norbornényle, un groupe cyclopentényle, un groupe cyclohexényle, un groupe cycloheptényle, un groupe tétrahydro-2H-pyranyle, un groupe morpholinyle, un groupe phényle, un groupe (alkyle en C₁-C₁₀)phényle, un groupe di(alkyle en C₁-C₁₀)phényle, un groupe biphényle, un groupe di(phényl)phényle, un groupe terphényle, un groupe naphtyle, un groupe pyridinyle, un groupe pyrimidinyle, -O(Q₃₁), - S(Q₃₁), -Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁), -S(=O)₂(Q₃₁), et -P(=O)(Q₃₁)(Q₃₂) ;
un groupe cyclopentyle, un groupe cyclohexyle, un groupe cycloheptyle, un groupe cycloctyle, un groupe adamantanyle, un groupe norbornanyle, un groupe norbornényle, un groupe cyclopentényle, un groupe cyclohexényle, un groupe cycloheptényle, un groupe tétrahydro-2H-pyranyle, un groupe morpholinyle, un groupe dioxanyle, un groupe phényle, un groupe (alkyle en C₁-C₁₀ )phényle, un groupe di(alkyle en C₁-C₁₀ )phényle, un groupe biphényle, un groupe di(phényl)phényle, un groupe terphényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe benzoisothiazolyle, un groupe benzoxazolyle, un groupe benzoisoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, un groupe imidazopyrimidinyle, un groupe 2,3-dihydro-1H-indényle, un groupe 1,2,3,4-tétrahydronaphtalényle, un groupe 2,3-dihydrobenzodioxinyle et un groupe benzodioxanyle, chacun non substitué ou substitué par au moins un groupe choisi parmi le deutérium, -F, -Cl, -Br, -I, -CD₃, -CD₂H, - CDH₂, -CF₃, -CF₂H, -CFH₂, un groupe hydroxyle, un groupe cyano, un groupe nitro, un groupe amidino, un groupe hydrazino, un groupe hydrazono, un groupe alkyle en C₁-C₁₀, un groupe alcényle en C₂-C₁₀, un groupe alcynyle en C₂-C₁₀, un groupe cyclopentyle, un groupe cyclohexyle, un groupe cycloheptyle, un groupe cycloctyle, un groupe adamantanyle, un groupe norbornanyle, un groupe norbornényle, un groupe cyclopentényle, un groupe cyclohexényle, un groupe cycloheptényle, un groupe tétrahydro-2H-pyranyle, un groupe morpholinyle, un groupe dioxanyle, un groupe phényle, un groupe (alkyle en C₁-C₁₀)phényle, un groupe di(alkyle en C₁-C₁₀)phényle, un groupe biphényle, un groupe di(phényl)phényle, un groupe terphényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe benzoisothiazolyle, un groupe benzoxazolyle, un groupe benzoisoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, un groupe imidazopyrimidinyle, un groupe 2,3-dihydro-1H-indényle, un groupe 1,2,3,4-tétrahydronaphtalényle, un groupe 2,3-dihydrobenzodioxinyle, un groupe benzodioxanyle, -O(Q₃₁), -S(Q₃₁), - Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁), -S(=O)₂(Q₃₁), and - P(=O)(Q₃₁)(Q₃₂) ; et
-O(Q₁), -S(Q₁), -Si(Q₁)(Q₂)(Q₃), -N(Q₁)(Q₂), -B(Q₁)(Q₂), -P(Q₁)(Q₂), -C(=O)(Q₁), - S(=O)₂(Q₁), et -P(=O)(Q₁)(Q₂), et
Q₁ à Q₃ et Q₃₁ à Q₃₃ sont choisis indépendamment parmi l'hydrogène, le deutérium, -F, -Cl, -Br, -I, -CD₃, -CD₂H, -CDH₂, -CF₃, -CF₂H, -CFH₂, un groupe hydroxyle, un groupe cyano, un groupe nitro, un groupe amidino, un groupe hydrazino, un groupe hydrazono, un groupe alkyle en C₁-C₁₀, un groupe alcényle en C₂-C₁₀, un groupe alcynyle en C₂-C₁₀, un groupe alcoxy en C₁-C₁₀, un groupe cyclopentyle, un groupe cyclohexyle, un groupe cycloheptyle, un groupe cycloctyle, un groupe adamantyle, un groupe norbornanyle, un groupe norbornényle, un groupe cyclopentényle, un groupe cyclohexényle. groupe cycloheptényle, un groupe tétrahydro-2H-pyranyle, un groupe morpholinyle, un groupe dioxanyle, un groupe phényle, un groupe (alkyle en C₁-C₁₀)phényle, un groupe di(alkyle en C₁-C₁₀)phényle, un groupe biphényle, un groupe di(phényl)phényle, un groupe terphényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinoléinyle, un groupe benzoquinoléinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe benzoisothiazolyle, un groupe benzoxazolyle, un groupe benzoisoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, un groupe imidazopyrimidinyle, un groupe 2,3-dihydro-1H-indényle, un groupe 1,2,3,4-tétrahydronaphtalényle, un groupe 2,3-dihydrobenzodioxinyle et un groupe benzodioxanyle, de préférence,
dans lequel :
R₁ à R₄ sont chacun choisis indépendamment parmi l'hydrogène, le deutérium, -F, -Cl, -Br, -I, -CD₃, -CD₂H, -CDH₂, -CF₃, -CF₂H, -CFH₂, un groupe alkyle en C₁-C₁₀, un groupe alcényle en C₂-C₁₀, un groupe alcynyle en C₂-C₁₀, un groupe alcoxy en C₁-C₁₀, un groupe cyclopentyle, un groupe cyclohexyle, un groupe cycloheptyle, un groupe phényle, un groupe (alkyle en C₁-C₁₀)phényle, un groupe di(alkyle en C₁-C₁₀)phényle, un groupe biphényle, un groupe di(phényl)phényle, un groupe terphényle, -O(Q₁), et -S(Q₁), et
Q₁ est choisi parmi un groupe alkyle en C₁-C₁₀, un groupe alcényle en C₂-C₁₀, un groupe alcynyle en C₂-C₁₀, un groupe alcoxy en C₁-C₁₀, un groupe cyclopentyle, un groupe cyclohexyle, un groupe cycloheptyle, un groupe phényle, un groupe (alkyle C₁-C₁₀)phényle, un groupe di(alkyle C₁-C₁₀)phényle, un groupe biphényle, un groupe di(phényl)phényle et un groupe terphényle.

5. Composé organométallique selon l'une quelconque des revendications 1 à 4, dans lequel :
chacun d'au moins un choisi parmi R₁₁ à R₁₄ est choisi indépendamment parmi un groupe alkyle en C₂-C₁₀, un groupe alcényle en C₂-C₁₀, un groupe alcynyle en C₂-C₁₀ et un groupe alcoxy en C₁-C₁₀, chacun non substitué ou substitué par au moins un groupe choisi parmi le deutérium, -F, -Cl, -Br, -I, -CD₃, -CD₂H, -CDH₂, -CF₃, -CF₂H, -CFH₂, un groupe hydroxyle, un groupe cyano, un groupe nitro, un groupe amidino, un groupe hydrazino, un groupe hydrazono, un groupe cyclopentyle, un groupe cyclohexyle, un groupe cycloheptyle, un groupe cycloctyle, un groupe adamantyle, un groupe norbornanyle, un groupe norbornényle, un groupe cyclopentényle. un groupe cyclohexényle, un groupe cycloheptényle, un groupe tétrahydro-2H-pyranyle, un groupe morpholinyle, un groupe phényle, un groupe (alkyle en C₁-C₁₀)phényle, un groupe di(alkyle en C₁-C₁₀)phényle, un groupe biphényle, un groupe di(phényl)phényle, un groupe terphényle, un groupe naphtyle, un groupe pyridinyle, un groupe pyrimidinyle, -O(Q₃₁), -S(Q₃₁), -Si(Q₃₁)(Q₃₂)(Q₃₃), -N(Q₃₁)(Q₃₂), -B(Q₃₁)(Q₃₂), -P(Q₃₁)(Q₃₂), -C(=O)(Q₃₁), -S(=O)₂(Q₃₁), et -P(=O)(Q₃₁)(Q₃₂), de préférence dans lequel :
au moins un choisi parmi R₁₁ à R₁₄ n'est ni un hydrogène, ni un groupe phényle, ni un groupe mésityle, ni un groupe méthyle ; et/ou
chacun d'au moins un choisi parmi R₁₁ à R₁₄ est indépendamment -O(Q₁), -S(Q₁), - Si(Q₁)(Q₂)(Q₃), -N(Q₁)(Q₂), -B(Q₁)(Q₂), -P(Q₁)(Q₂), -C(=O)(Q₁), -S(=O)₂(Q₁), -P(=O)(Q₁)(Q₂), ou un groupe représenté par la formule A5 : 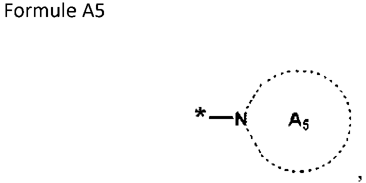
Q₁ à Q₃ sont chacun indépendamment identiques à ceux définis dans la revendication 1, et
A₅ dans la formule A5 est un groupe hétérocycloalkyle en C₁-C₁₀ substitué ou non substitué, un groupe hétérocycloalcényle en C₁-C₁₀ substitué ou non substitué, un groupe hétéroaryle en C₁-C₆₀ substitué ou non substitué, ou un groupe hétéropolycyclique condensé monovalent non aromatique substitué ou non substitué.

6. Composé organométallique selon l'une quelconque des revendications 1 à 5, dans lequel :
a) R₁₁ et R₁₂ sont différents l'un de l'autre, R₁₃ et R₁₄ sont différents l'un de l'autre, R₁₁ et R₁₃ sont identiques l'un à l'autre, et R₁₂ et R₁₄ sont identiques l'un à l'autre ;
b) R₁₁ et R₁₃ sont différents l'un de l'autre, R₁₂ et R₁₄ sont différents l'un de l'autre, R₁₁ et R₁₄ sont identiques l'un à l'autre, et R₁₂ et R₁₃ sont identiques l'un à l'autre ;
c) R₁₁ et R₁₂ sont différents l'un de l'autre, et R₁₁, R₁₃ et R₁₄ sont identiques l'un à l'autre ; ou
d) R₁₁ à R₁₄ sont tous différents les uns des autres.

7. Composé organométallique selon la revendication 1, dans lequel le composé organométallique est choisi parmi les composés 1 à 79, 82 à 116, 118 à 169 et 172 à 180 : 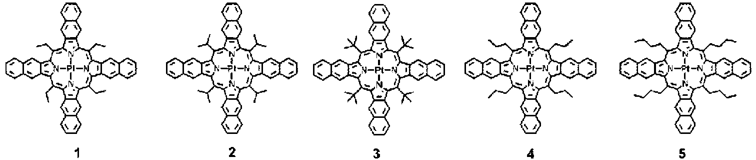 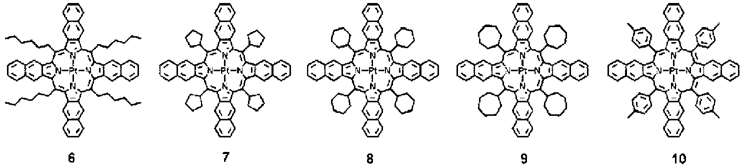 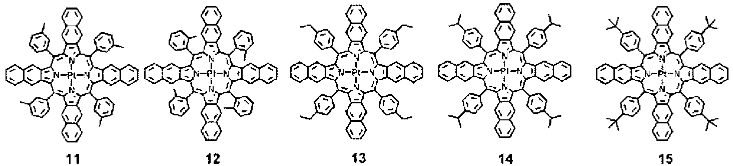 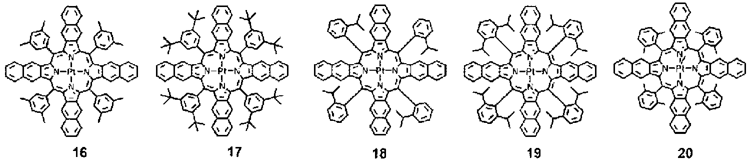 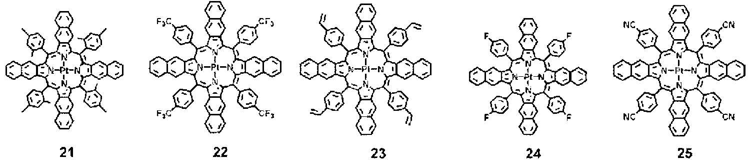 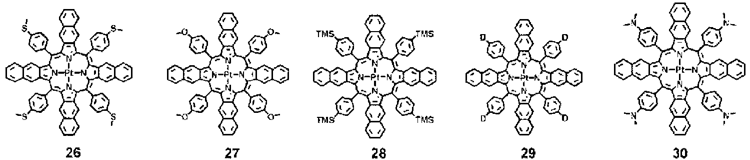 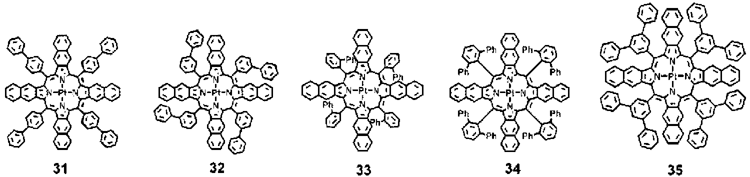 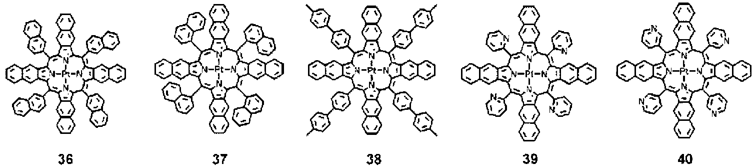 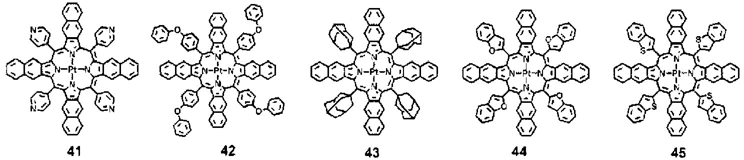 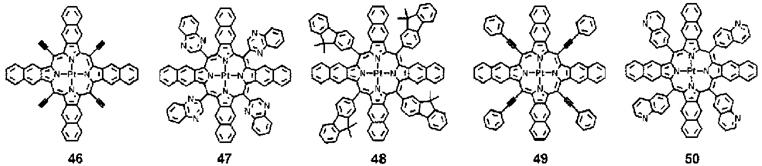 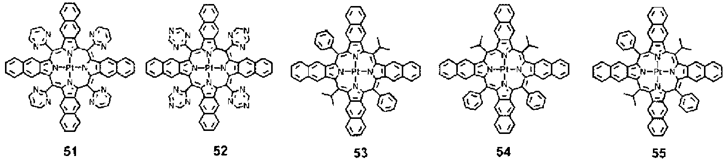 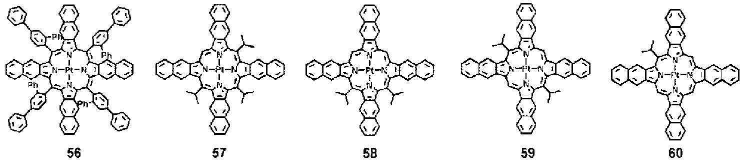 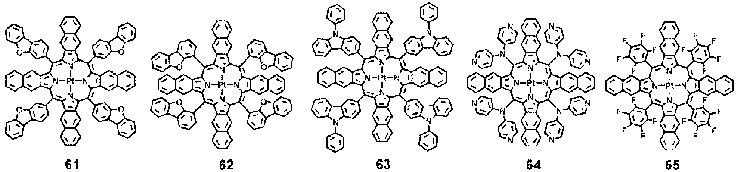 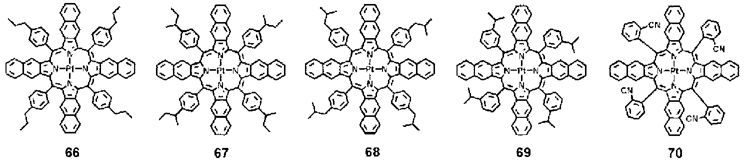 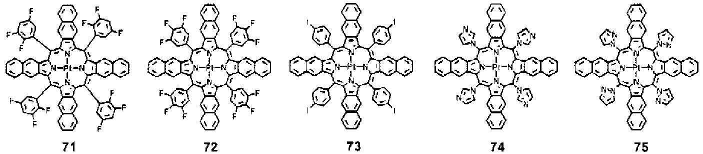 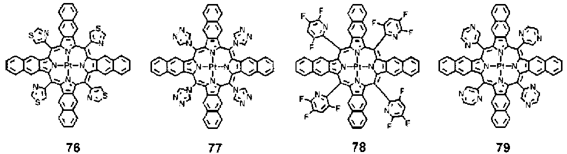 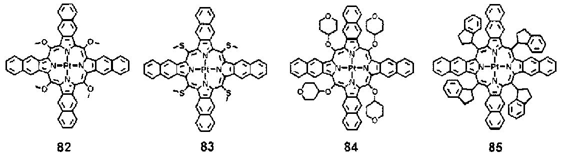 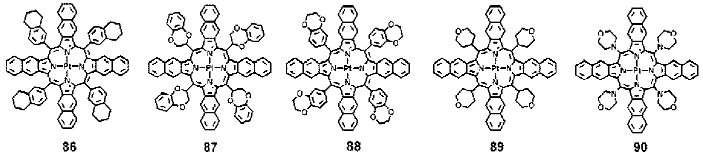 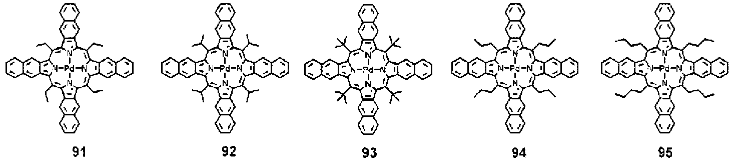 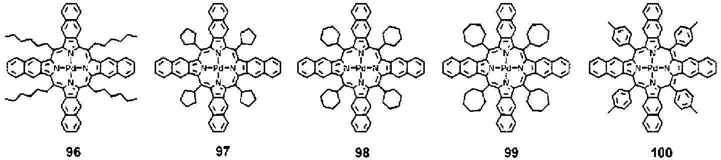 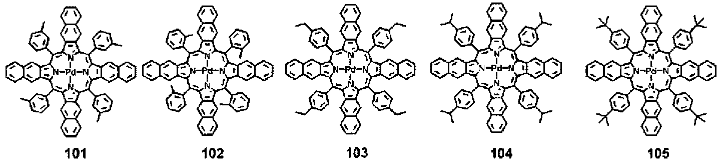 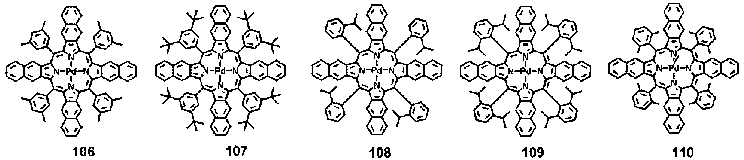 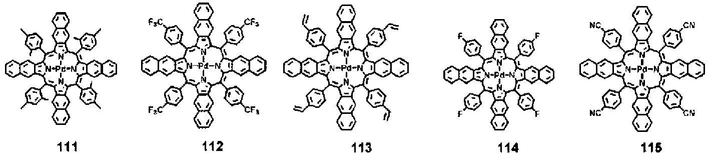 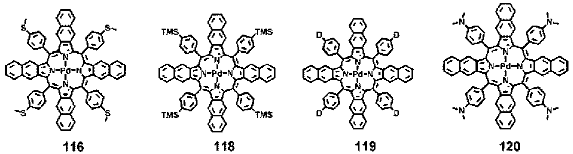 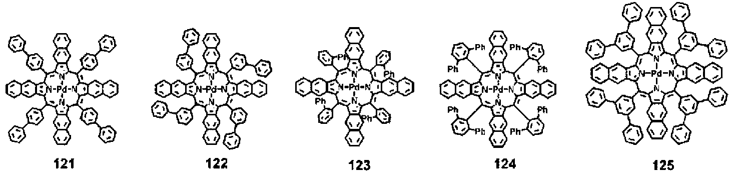 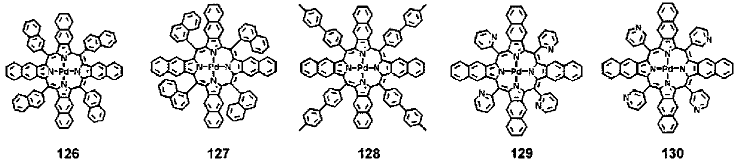 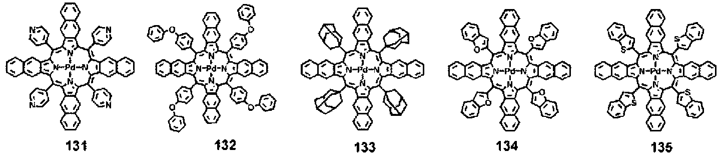 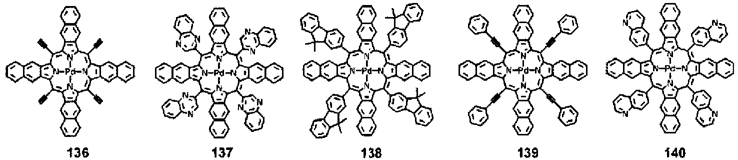 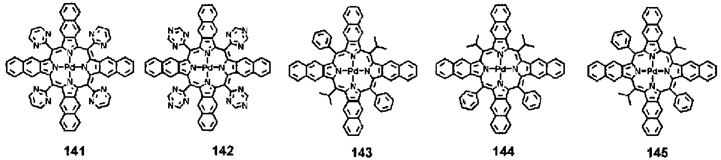 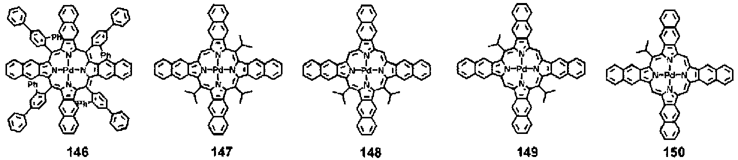 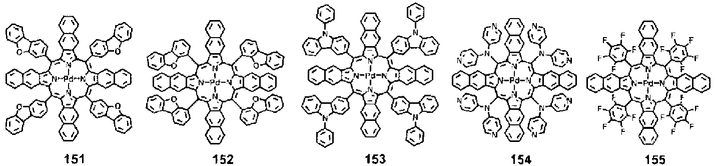 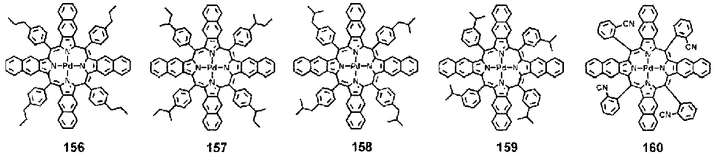 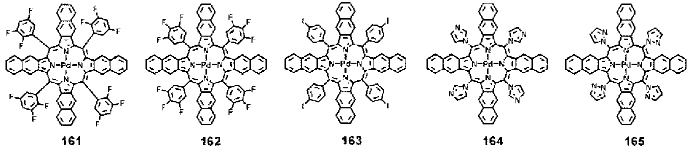 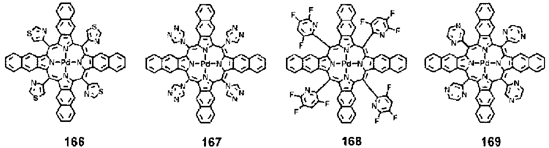 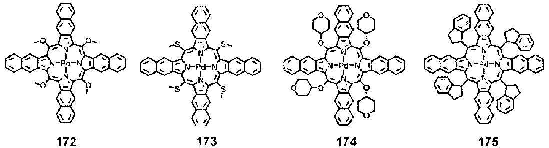 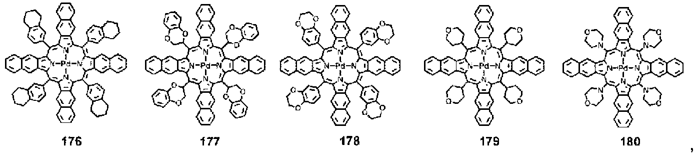 dans lequel, dans les composés 1 à 79, 82 à 116, 118 à 169 et 172 à 180, TMS indique un groupe triméthylsilyle et Ph indique un groupe phényle.

8. Dispositif électroluminescent organique comprenant :
une première électrode ;
une seconde électrode faisant face à la première électrode ; et
une couche organique entre la première électrode et la seconde électrode,
dans lequel la couche organique comprend une couche d'émission, et
la couche d'émission comprend au moins l'un du composé organométallique selon l'une quelconque des revendications 1 à 7 et émet une lumière phosphorescente proche infrarouge présentant une longueur d'onde d'émission maximale dans une plage de 720 nm à 2500 nm.

9. Dispositif électroluminescent organique selon la revendication 8, dans lequel :
la première électrode est une anode,
la seconde électrode est une cathode, et
la couche organique comprend en outre une région de transport de trous entre la première électrode et la couche d'émission et une région de transport d'électrons entre la couche d'émission et la seconde électrode,
la région de transport de trous comprend une couche d'injection de trous, une couche de transport de trous, une couche auxiliaire d'émission, une couche de blocage d'électrons, ou toute combinaison de celles-ci, et
la région de transport d'électrons comprend une couche de blocage de trous, une couche de transport d'électrons, une couche d'injection d'électrons, ou toute combinaison de celles-ci.

10. Dispositif électroluminescent organique selon la revendication 9, dans lequel :
au moins une choisie parmi la région de transport de trous et la couche d'émission comprend un composé contenant un groupe arylamine, un composé contenant un cycle acridine et un composé contenant un cycle carbazole, ou
au moins un composé choisi parmi la couche d'émission et la région de transport d'électrons comprend un composé contenant du silicium, un composé contenant un groupe oxyde de phosphine, un composé contenant un groupe oxyde de soufre, un composé contenant un cycle triazine, un composé contenant un cycle pyrimidine, un composé contenant un cycle pyridine, un composé contenant un cycle dibenzofurane et un composé contenant un cycle dibenzothiophène.

11. Appareil comprenant le dispositif électroluminescent organique selon l'une quelconque des revendications 8 à 10.

12. Appareil selon la revendication 11, dans lequel l'appareil est un dispositif électronique.

13. Appareil selon la revendication 11 ou la revendication 12, dans lequel :
l'appareil comprend en outre un transistor à couche mince,
le transistor à couche mince comprend une électrode source et une électrode drain, et
la première électrode du dispositif électroluminescent organique est couplée électriquement à l'une parmi l'électrode source et l'électrode drain du transistor à couche mince.
